(19) Europäisches Patentamt
European Patent Office
Office européen des brevets

(11) EP 3 984 532 A2

(12) EUROPEAN PATENT APPLICATION

(43) Date of publication:
**20.04.2022 Bulletin 2022/16**

(21) Application number: **21202004.4**

(22) Date of filing: **30.03.2017**

(51) International Patent Classification (IPC):
***A61K 31/14*** $^{(2006.01)}$ ***A61P 25/00*** $^{(2006.01)}$

(52) Cooperative Patent Classification (CPC):
**A61K 31/20; A61K 31/201; A61K 31/202; A61P 25/00**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:
**30.03.2016 US 201662315142 P**
**30.03.2016 US 201662315152 P**
**30.03.2016 US 201662315158 P**
**30.03.2016 US 201662315163 P**
**30.03.2016 US 201662315134 P**
**27.04.2016 US 201662328094 P**
**13.12.2016 PCT/EP2016/080786**

(62) Document number(s) of the earlier application(s) in accordance with Art. 76 EPC:
**17714731.1 / 3 435 997**

(27) Previously filed application:
**30.03.2017 PCT/EP2017/714731**

(71) Applicant: **Société des Produits Nestlé S.A.**
**1800 Vevey (CH)**

(72) Inventors:
- **SCHNEIDER, Nora**
  **1066 Epalinges (CH)**
- **HAUSER, Jonas**
  **1003 Lausanne (CH)**
- **SILVA ZOLEZZI, Irma**
  **276750 Singapore (SG)**
- **SAMUEL, Tinu Mary**
  **1814 La Tour-de-Peilz (CH)**
- **DEONI, Sean**
  **PROVIDENCE, 2912 (US)**
- **BARTFAI, Tamas**
  **11328 Stockholm (SE)**

(74) Representative: **Gagliardi, Tatiana**
**Société des Produits Nestlé S.A.**
**Avenue Nestlé 55**
**1800 Vevey (CH)**

Remarks:
This application was filed on 11-10-2021 as a divisional application to the application mentioned under INID code 62.

# (54) COMPOSITIONS COMPRISING CHOLINE AND THEIR USE

(57) *The present invention relates to a composition comprising one or more fatty acid derivatives for use in promoting, supporting or optimizing one or more of the following:*
*(i) de novo myelination;*
*(ii) brain structure;*
*(iii) brain connectivity;*
*(iv) intellectual potential;*
*(v) cognitive potential; and*
*(vi) learning potential;*
*(vii) cognitive functioning*
*in an offspring of a female subject, wherein said composition is for administration to the female subject.*

EP 3 984 532 A2

## Description

### Field of the Invention

The present invention relates to a composition for promoting, supporting or optimizing de novo myelination, and/or brain structure, and/or brain connectivity, and/or one or more of cognitive potential, learning potential, and intellectual potential and/or cognitive functioning in an offspring of a female subject.

### Background to the Invention

In-utero and during the first weeks or months of life, a number of external factors may influence an infant's growth and development. In particular, on-going research is making it more and more apparent that a mother's diet may affect an infant's neurodevelopment, in particular brain development, and that said affect may have irreversible short and long term consequences.

Neurodevelopment, in particular brain development, *in-utero* and over the first 2 or 3 years of life is rapid and places exceptionally high demands on the supply of key nutrients to the infant. Failure to meet these nutrient demands during this crucial period may result in sub-optimal neurodevelopment, in particular brain development.

A process that plays a key role in neurodevelopment, in particular brain development, and that may be particularly sensitive to nutritional factors, is de novo myelination, the process by which naked axons are ensheathed in myelin. This process, and in particular the trajectory it follows, plays a central role in determining brain structure, in particular the amount and/or the spatial distribution of myelinated matter throughout the brain.

The relevance of brain structure, in particular the amount and/or spatial distribution of myelinated matter throughout the brain, for cognitive functioning and intelligence is well documented. In itself myelin in the brain provides an insulating sheet along neurons permitting much faster conduction of nerve impulses. However, it is brain structure, in particular the amount and/or spatial distribution of myelin throughout the brain, that affects brain connectivity e.g. via what pathway and how quickly and efficiently, messages in the form of neural impulses are communicated within the brain and in particular between different brain regions. This interbrain communication can play a role in cognitive functioning and learning, and may affect or even serve to physiologically limit intellectual, cognitive and/or learning potential.

Accordingly, there is a need to identify nutrients necessary to promote support or optimise de novo myelination, in particular the de novo myelination trajectory, and thereby brain structure, in particular the amount and/or the spatial distribution of myelinated matter throughout the brain. Further there is a need to provide compositions that may be consumed pre pregnancy, whilst pregnant, and whilst breastfeeding to ensure a mother has a sufficient supply of any said nutrients to meet the demands of her offspring and to thereby to promote support or optimise de novo myelination, in particular the de novo myelination trajectory, and thereby brain structure, more particularly the amount or spatial distribution of myelinated matter throughout the brain, in said offspring. Further still, there is a need to find ways to optimise intellectual potential and/or cognitive potential and/or learning potential and/or cognitive functioning in said offspring.

In addition, it is important to ensure that a mother is adequately supplied with said nutrients so as to ensure her wellbeing and to avoid any depletion of said nutrients in her body, and any associated health effects, that may occur due to the demand for said nutrients from her offspring.

Surprisingly the inventors have now found that fatty acid derivatives may promote, support or optimise de novo myelination, in particular the de novo myelination trajectory, and/or brain structure, in particular the amount and/or spatial distribution of myelinated matter throughout the brain, in an infant.

### Summary of Invention

The present invention is based on a maternal supplement comprising a fatty acid derivative, preferably docosahexaenoic acid and/or arachidonic acid, or a mixture thereof, which is for administration to a female subject prior to or during pregnancy, and/or while an offspring is receiving breast milk from the female subject. The maternal supplements of the invention are understood to have a beneficial effect on the de novo myelination in the offspring. Promoting, supporting and/or optimizing de novo myelination by way of maternal supplements enables the mother to provide her offspring with health benefits, including long-term benefits to the infant later in life when the infant is no longer breast-fed.

A first aspect of the invention therefore relates to a composition comprising one or more fatty acid derivatives for use in promoting, supporting or optimizing one or more of the following:

(i) de novo myelination;
(ii) brain structure;
(iii) brain connectivity;

(iv) intellectual potential;
(v) cognitive potential;
(vi) learning potential; and
(vii) cognitive functioning

in an offspring of a female subject, wherein said composition is for administration to the female subject.

Surprisingly the inventors have found that compositions comprising a fatty acid such as docosahexaenoic acid, when administered to a female subject can promote, support or optimize de novo myelination, in particular the de novo myelination trajectory, and/or brain structure, in particular the amount and/or temporal-spatial distribution of myelinated matter throughout the brain, in a subject.

The current finding stems from the results of the nutritional analysis of the results of a longitudinal cognitive and brain imaging study wherein de novo myelination, in particular the de novo myelination trajectory, and/or brain structures, including the amount and spatial distribution of myelinated matter throughout the brain, were examined and compared. Further details of this study and the results are given in the accompanying examples.

A second aspect of the invention relates to a fatty acid derivative, preferably docosahexaenoic acid and/or arachidonic acid, or a mixture thereof, for use in promoting, supporting or optimizing one or more of the following:

(i) de novo myelination;
(ii) brain structure;
(iii) brain connectivity;
(iv) intellectual potential;
(v) cognitive potential;
(vi) learning potential; and
(vii) cognitive functioning

in an offspring of a female subject, wherein said fatty acid derivative is for administration to the female subject.

A third aspect of the invention relates to a method of promoting, supporting or optimizing one or more of the following:

(i) de novo myelination;
(ii) brain structure;
(iii) brain connectivity;
(iv) intellectual potential;
(v) cognitive potential;
(vi) learning potential; and
(vii) cognitive functioning

in an offspring of a female subject, said method comprising administering to the female subject a therapeutically effective amount of a fatty acid derivative, preferably docosahexaenoic acid and/or arachidonic acid, or a mixture thereof.

A fourth aspect of the invention relates to the use of a fatty acid derivative, preferably docosahexaenoic acid and/or arachidonic acid, or a mixture thereof, in the manufacture of a composition to promote, support or optimize one or more of the following:

(i) de novo myelination;
(ii) brain structure;
(iii) brain connectivity;
(iv) intellectual potential;
(v) cognitive potential;
(vi) learning potential; and
(vii) cognitive functioning

in an offspring of a female subject, wherein said composition is for administration to the female subject.

A fifth aspect of the invention relates to a composition comprising a fatty acid derivative, preferably docosahexaenoic acid and/or arachidonic acid, or a mixture thereof, wherein said composition is for use as a pre-pregnancy supplement for promoting, supporting or optimizing one or more of the following:

(i) de novo myelination;
(ii) brain structure;

(iii) brain connectivity;
(iv) intellectual potential;
(v) cognitive potential;
(vi) learning potential; and
(vii) cognitive functioning

in an offspring.

**Brief Description of the Figures**

Fig. 1 - Shows the mean whole brain (all white matter) myelination trajectories in infants and young children breastfed vs fed with two commercial formulas comprising different levels of docosahexaenoic acid.
Fig. 1a - Shows the mean regional myelination trajectories in infants and young children breastfed vs fed with two commercial formulas comprising different levels of docosahexaenoic acid.
Fig. 1b - Is a brain image showing the myelinated brain regions associated with docosahexaenoic acid.
Fig. 1c - Is a brain image showing the myelinated brain regions associated with arachidonic acid.
Fig. 1d - Is a brain image showing the myelinated brain regions associated with folic acid.
Fig. 1e - Is a brain image showing the myelinated brain regions associated with vitamin B12.
Fig. 1f - Is a brain image showing the myelinated brain regions associated with Iron.
Fig. 1g - Is a brain image showing the myelinated brain regions associated with Zinc.
Fig. 1h - Is a brain image showing the myelinated brain regions associated with Calcium.
Fig. 1I - Is a brain image showing the myelinated brain regions associated with Phosphorus.
Fig. 1J - Is a brain image showing the myelinated brain regions associated with Magnesium.
Fig. 1k - Is a brain image showing the myelinated brain regions associated with Sphingomyelin.
Fig. 1L - Is a brain image showing the myelinated brain regions associated with Phosphatidylinositol.
Fig. 1M - Is a brain image showing the myelinated brain regions associated with phosphatidylcholine.
Fig. 1n - Is a brain image showing the myelinated brain regions associated with phosphatidylcholine.
Figure 2: Shows the effect of nervonic acid on neuronal cell density and astrocyte cell density.
Figure 3: Shows the effect of stearic acid on neuronal cell density and astrocyte cell density.
Figure 4: Shows the effect of octanoic acid on neuronal cell density and astrocyte cell density.
Figure 5: Shows the effect of sphingomyelin on number of neurospheres and neuronal proliferation.
Figure 6: Shows the relative abundance of main SM species in ingredient, infant formula, cow milk and human milk. (Error bars represented the standard deviation with n=3).
Figure 7: Shows the relative FA abundance in SM fraction from ingredient, infant formula, cow's milk, and human milk. (Error bars represented the standard deviation with n=3).
Figure 8: Shows the impact of DHA on MBP, NF, and/or MBP/NF at day 18 and/or day 30.
Figure 9: Shows the impact of stearic acid on A2B5, MBP, MAG, NF, MBP/NF, and/or MAG/NF at day 12, day 18 and/or day 30.
Figure 10: Shows the impact of vitamin B12 on A2B5, NF, MBP/NF, and/or MAG at day 12, day 18 and/or day 30.
Figure 11: Shows the impact of folic acid on A2B5, NF, MAG, MAG/NF, and/or MBP/NF at day 12, day 18 and/or day 30.
Figure 12: Shows the impact of choline on A2B5, MAG and/or MBP at day 12, day 18 or day 30.
Figure 13: Shows the impact of Iron on A2B5, MBP, MAG, NF, and/or MAG/NF at day 12, day 18 and/or day 30.
Figure 14: Shows the impact of Zinc on MBP, NF and/or MBP/NF at day 12, day 18 and/or day 30.
Figure 15: Shows the impact of phosphorus on MAG, NF, and/or MAG/NF at day 12, day 18 and/or day 30.
Figure 16: Shows the impact of magnesium on A2B5, MBP, NF, MAG, MBP/NF and/or MAG/NF at day 12, day 18 and/or day 30.
Figure 17: Shows the impact of copper on A2BF, MAG, and/or MAG/NF at day 12 and/or day 18.
Figure 18: shows the impact of phosphatidylcholine on A2B5 at day 12 and on MAG at day 18.
Figure 19: Shows the impact of phosphatidylinositol on A2B5, MBP, MAG, NF, MAG/NF at day 12, day 18 and/or day 30.
Figure 20: Shows the impact of phosphatidylserine on A2B5, NF, and/or MAG/NF at day 12 and/or D18.
Figure 21: Shows the impact of sphingomyelin on A2B5, MAG, and/or MBP at day 12, day 18 and/or day 30.
Figure 22: Shows the impact of ceramide on A2B5 at day 12, and on MAG at day 18.
Figure 23: Shows the impact of galactoceramide on A2B5, MBP, NF, and/or MBP/NF at day 12 and/or day 30.
Figure 24: Shows the impact of glucoceramide on A2B5 at day 12 and NF at day 12 and day 18.

Figure 25: Shows the impact of D-erythroceramide on A2B5 at day 12 and on MAG at day 18.
Figure 26: Shows the impact of Ceramide-1-phosphate on A2B5 at day 12, and on NF and MAG at day 18.
Figure 27: Shows the impact of monosialoganglioside-3 (GM3) on A2B5, MBP, MAG, and/or MBP/NF at day 12, day 18 and/or day 30.
Figure 28: Shows the impact of disialogangliosides 3 (GD3) on A2B5, MBP, NF and/or MAG at day 12, day 18 and/or day 30.
Figure 29: Shows the fatty acid profile of Phosphatidylinositol (PI), Phosphatidylcholine, Phosphatidyl (PC), Phosphatidylserine (PS), and Sphingomyelin used in example 6.
Figure 30: Shows the impact of vitamin B12 on MAG and MBP mRNA expression and on MBP and BetaIII Co-expression.
Figure 31: Shows the impact of ARA on MAG and MBP mRNA expression and on MBP and Betalll Co-expression.
Figure 32: Shows the impact of stearic acid on MAG and MBP mRNA expression and on MBP and BetaIII Co-expression.
Figure 33: Shows the impact of zinc on MAG and MBP mRNA expression and on MBP and Betalll Co-expression.
Figure 34: Shows the impact of phosphatidylinositol on MAG and MBP mRNA expression.
Figure 35: Shows the impact of GD3 on MAG and MBP mRNA expression and on MBP and Betalll Co-expression.
Figure 36: Shows the impact of DHA on MAG and MBP mRNA expression and on MBP and Betalll Co-expression.
Figure 37: Shows the impact of nervonic acid on MAG and MBP mRNA expression and on MBP and BetaIII Co-expression.
Figure 38: Shows the impact of Iron on MAG and MBP mRNA expression and on MBP and Betalll Co-expression.
Figure 39: Shows the impact of phosphatidylcholine on MAG and MBP mRNA expression and on MBP and Betalll Co-expression.
Figure 40: Shows the impact of phosphatidylserine on MAG and MBP mRNA expression and on MBP and Betalll Co-expression.
Figure 41: Shows the impact of folic acid on MAG and MBP mRNA expression and on MBP and BetaIII Co-expression.
Figure 42: Shows the impact of choline on MAG and MBP mRNA expression and on MBP and BetaIII Co-expression.
Figure 43: Shows the impact of ceramide on MAG and MBP mRNA expression and on MBP and BetaIII Co-expression.
Figure 44: Shows the impact of galactoceramide on MAG and MBP mRNA expression and on MBP and Betalll Co-expression.
Figure 45: Shows the impact of glucoceramide on MAG and MBP mRNA expression and on MBP and BetaIII Co-expression.
Figure 46: Shows the impact of Ceramide-1-phosphate on MAG and MBP mRNA expression and on MBP and Betalll Co-expression.
Figure 47: Shows the impact of D-erythroceramide on MAG and MBP mRNA expression and on MBP and Betalll Co-expression.
Figure 48: Shows the impact of sphingomyelin on MBP and Betalll Co-expression.
Figure 49: Shows the impact of GM3 on MBP and Betalll Co-expression.
Fig. 50a - Is a brain image showing the myelinated brain regions associated with maternal DHA intake.
Fig. 50b - Is a brain image showing the myelinated brain regions associated with maternal choline intake.
Fig. 50c - Is a brain image showing the myelinated brain regions associated with maternal Iron intake.
Fig. 50d - Is a brain image showing the myelinated brain regions associated with maternal folic acid intake.
Fig. 50e - Is a brain image showing the myelinated brain regions associated with maternal vitamin B12 intake.

## Detailed description

The invention will now be described in further detail. It is noted that the various aspects, features, examples and embodiments described in the present application may be compatible and/or combined together.

For the sake of clarity, all the embodiments and aspects reported hereafter will be applicable to different embodiments and aspects described for the present invention *mutatis mutandis.*

In one aspect of the present invention there is provided a synthetic composition comprising a fatty acid, preferably docosahexaenoic acid and/or arachidonic acid, or a mixture thereof, for use in promoting supporting or optimizing de novo myelination, in particular the de novo myelination trajectory, and/or brain structure, in particular the amount and spatial distribution of myelinated matter throughout the brain, in particular as determined by de novo myelination and the de novo myelination trajectory, and/or brain connectivity, and/or intellectual potential and/or cognitive potential and/or learning potential and/or cognitive functioning in an offspring of a female subject, wherein said choline is for administration to the female subject.

By promoting, supporting and/or optimizing de novo myelination, in particular the de novo myelination trajectory,

and/or brain structure, in particular the amount and spatial distribution of myelinated matter throughout the brain, in particular as determined by de novo myelination and the de novo myelination trajectory, and/or the intellectual potential and/or cognitive potential and/or learning potential and/or cognitive functioning, the composition of the present invention may prevent, reduce the risk and/or mitigate a sub-optimal de novo myelination, in particular de novo myelination trajectory, and/or brain structure, in particular the amount and spatial distribution of myelinated matter throughout the brain, in particular as determined by de novo myelination and the de novo myelination trajectory, and/or the intellectual potential and/or cognitive potential and/or learning potential, and/or cognitive functioning. This may be non-therapeutic or therapeutic.

In the context of the present invention, the terms "comprising" or "comprises" do not exclude other possible elements. The composition of the present invention, including the many embodiments described herein, can comprise, consist of, or consist essentially of the essential elements and limitations of the invention described herein, as well as any additional or optional ingredients, components, or limitations described herein or otherwise depending on the needs.

The term "promote" or "promoting" as used herein refers to a factor or a number of factors causing a certain process to occur.

The term "support" or "supporting" used herein refers to a factor or a number of factors sustaining a certain process once it has started to occur.

The term "optimize" or "optimizing" as used herein refers to an improvement or enhancement.

Unless otherwise indicated, all amounts indicated for nutrients are expressed as amounts per weight of dry nutritional composition.

All percentages expressed herein are by weight unless otherwise stated.

The term "cfu" should be understood as colony-forming unit.

As used herein the term "therapeutically effective amount" refers to an amount of the "active" (here, a fatty acid, e.g. docosahexaenoic acid and/or arachidonic acid, or a mixture thereof) that gives rise to a therapeutic effect, for example, in terms of one or more of the following effects in an offspring: (i) de novo myelination; (ii) brain structure; (iii) brain connectivity; (iv) intellectual potential; (v) cognitive potential; and (vi) learning potential, and (vii) cognitive functioning.

As used herein a "metabolite" refers to a substance produced during metabolism.

As used herein a "metabolic precursor" refers to a substance from which another is formed by a metabolic reaction.

Where a nutrient may be comprised in a composition under different forms (as such or in the form of salts, complexes or more complex structures comprising the nutrient) the amounts reported hereafter are intended to make reference to the amount of the nutrient as such.

In one preferred embodiment, the composition promotes, supports or optimizes the amount and/or spatial distribution of myelinated matter throughout the brain, in particularly the amount and/or spatial temporal distribution of myelinated matter throughout the brain.

In one preferred embodiment, the composition promotes, supports or optimizes the de novo myelination trajectory.

Since human breastmilk is the gold standard when it comes to infant nutrition, the de novo myelination trajectory measured or observed in breastfed, more particularly exclusively breastfed, subjects of a well-nourished or nutritionally replete mothers, may be considered optimal. A composition of the invention may therefore be considered to optimise a subject’s myelination trajectory if it brings a subject’s de novo myelination trajectory in line or closer to that measured or observed in a breastfed, more particularly exclusively breastfed, subject of a well-nourished or nutritionally replete mother.

An offspring’s de novo myelination trajectory may be considered to be in line or closer to that measured or observed in a breastfed, more particularly exclusively breastfed subject, in particular of a well-nourished or nutritionally replete mother, if the distance between any equivalent/same measurement points on the offspring’s trajectory and said breastfed subject’s trajectory is up to 50%, in particular up to 25%, more particularly up to 20%. Non limiting examples within the range of up to 50% include, 50%, 40%, 30%, 25%, 20%, 10%, 5%, 1%, 0.5%, and 0.01%. In particular the trajectories will be considered bioequivalent.

The myelination trajectory can be measured at any combination of time points. In particular the time points are within the first 5 years of a human subject’s life, more particularly the first 2 or 3 years of a human’s life, even more particularly in the first year of a human’s life.

The de novo myelination trajectory may be determined by measuring the myelin associated water fraction and/or the myelin associated water pool in a subject at different times points, in particular at different time points across the first 5 years of a human subject’s life, more particularly the first 2 or 3 years of a human’s life, even more particularly the 1$^{st}$ year of a human’s life. The myelin associated water fraction and/or the myelin associated water pool in a subject may be measured using a multicomponent relaxation (MCR) magnetic resonance imaging (MRI) technique and in particular using the mcDESPOT technique (Deoni et al 2008). In particular the de novo myelination trajectory may be

determined by measuring the myelin associated water pool using the mcDESPOT technique (Magn.Reson.Med.2008 60:1372-1387 the subject matter of which is hereby incorporated by reference).

A composition of the invention may be considered to optimise an offspring's cognitive functioning if it brings one or more offspring's scores in a standardized neurodevelopmental test, for example on the Mullen Scales of Early Learning, in line or closer to that measured or observed in a breastfed, more particularly exclusively breastfed subject, in particular of a well-nourished or nutritionally replete mother. An offspring's cognitive and neurodevelopmental functioning may be considered to be in line or closer to that measured in said breastfed subject, if the difference between one or more of said offspring's standardized neurodevelopmental test scores, for example Mullen's T scores, and that of said breastfed subject is less than one standard deviation, more particularly less than half a standard deviation of a standardized test score, for example less than 10 points, more particularly less than 5 points for the Mullen's T score, in particular less than 2 points. Said standardized neurodevelopmental test scores, for example Mullen's T scores, being measured at the same time point in said subject and said breastfed subject.

Said Mullen's score can be measured at any appropriate time point and in particular within the first 5 years of a human subject's life, more particularly the first 2 or 3 years of a human's life, even more particularly in the first year of a human's life.

As used herein, the term "female subject" refers to a female, especially prior to or during pregnancy or shortly after childbirth. Preferably, the female subject is a mammalian subject, more preferably, a cat, dog or human. Even more preferably, the female subject is a human. In all cases the female subject may be deficient or borderline deficient (sub-clinically deficient) in choline, or the female subject does not get a sufficient daily supply of choline through their diet.

As used herein, unless otherwise indicated, the term "offspring" encompasses the offspring of the female subject at any stage of development including fetus, neonate, infant, child and adult, or the fetus, infant or adult in the case of other mammals (for example cats and dogs). Preferably, in any embodiment of the present invention, the term offspring in relation to a human, refers to the neonate, infant and child stages, and more preferably the neonate and infant stages. Preferably, in any embodiment of the present invention, the term offspring in relation to other mammals (e.g. cats and dogs), refers to a neonate or infant stage.

As used herein, the term "neonate" refers to a newborn subject. In humans, the term neonate typically refers to an infant less than 4 weeks old.

The term "infant" as used herein refers to a human infant of up to 12 months of age and includes preterm and very preterm born infants, infants having a low birth weight i.e. a new born having a body weight below 2500g (5.5 pounds) either because of preterm birth or restricted fetal growth, and infants born small for gestational age (SGA) i.e. babies with birth weights below the 10th percentile for babies of the same gestational age.

The term "child" as used herein refers to a human of 1 to 18 years of age, more preferably a human of 1 to 10 years of age, even more preferably a human of 1 to 5 years of age, and even more preferably a human of 1 to 2 years of age.

The expression "young child" means a child aged between one and five years, (including toddlers).

A "preterm" or "premature" means an infant or young child that was not born at term. Generally it refers to an infant born prior to the completion of 37 weeks of gestation.

The expression "term born infant" indicates an infant born after 37 weeks gestation.

The expression "postnatal period" or ""postpartum period" is the period beginning immediately after the birth of a child and extending for about six weeks.

In one preferred embodiment, the offspring is a formula fed infant or child. The term "formula fed infant or child" as used herein refers to an infant or child fed either infant formula and/or growing up milk.

Exclusive breast feeding / infants or young children exclusively breast fed refers to infants for which the great majority of nutrients and/or energy originates from human breast milk (the "great majority" is preferably at least 90% or at least 95%, or at least 99%).

Infants/young children predominantly fed infant formula refers to infants or young children for which nutritional sources of nutrients and/or energy predominantly originates from synthetic infant formula, follow-on milk or growing-up milks. Predominantly refers to at least 50% of those nutrients and/or energy, or at least 75%.

The term "de novo myelination" as used herein refers to the process by which naked axons in the brain of a subject are myelinated during growth and development. The process starts *in utero* and is most prolific in the first 5 years of a human subject's life, in particular the first 2 or 3 years of a human's life. More preferably, the term refers to the de novo myelination trajectory.

The term "de novo myelination trajectory" as used herein refers to the extent of myelination (as linked to Myelin Water Fraction) as a function of time, and in particular across infancy and childhood, in particular early childhood, and more particularly in the first 5 years of a human subject's life, more particularly the first 2 or 3 years of a human's life, or first year of life.

The term "brain structure" as used herein refers to the structure of grey and white matter within the brain and specific brain regions, and in particular to myelinated matter within the brain and specific brain regions as determined by de novo myelination and in particular the de novo myelination trajectory i.e. by the de novo structural deposition of

myelin.More particularly the term refers to the amount and/or spatial distribution of myelinated matter throughout the brain, and/or in specific brain regions, and even more particularly the amount and/or temporal spatial distribution of myelinated matter throughout the brain and/or in specific brain regions.

The term "intellectual potential" as used herein refers to the possible intellectual ability or capacity attainable by a subject as determined by physiological factors. In particular, intellectual potential may refer to fluid intelligence.

The term "fluid intelligence" as used herein refers to a subject's neural potential and/or a subject's novel or abstract problem solving capability as determined by physiological factors. This is distinct from crystallized intelligence which is determined, at least in part by learned or acculturated knowledge.

The term "cognitive potential" as used herein refers to the possible cognitive and/or mental ability or capacity attainable by a subject as determined by physiological factors. In particular the term may refer to one or more of: information processing potential, perception potential, attention potential, thinking potential, reasoning potential, understanding and remembering potential, psychomotor potential, including gross motor and fine motor potential, visual potential, including visual reception potential, language potential, including expressive and receptive language potential, memory and recall potential, concentration potential, executive function potential, including problem-solving, decision-making and inhibition potential.

The term "learning potential" as used herein refers to the possible ability or capacity a subject has to learn e.g. how easily and/or quickly a subject may be able to acquire knowledge or skills through experience, study or being taught, as determined by physiological factors. As well as the possible ability a subject has to adapt in response to environmental factors, as determined by physiological factors.

Fatty acids such as docosahexaenoic acid and/or arachidonic acid may be particularly effective at supporting, promoting or optimizing de novo myelination, in particular the de novo myelination trajectory, and/or brain structure, in particular the amount and/or spatial distribution of myelinated matter throughout the brain, in the following brain areas: cerebellum, internal capsule, temporal lobe, frontal cortex, parietal lobe, motor and sensory cortices (including coordination and execution of movement), visual cortex, frontal cortices. Accordingly, a composition comprising a fatty acid may be particularly effective at promoting, supporting or optimising vision potential, motor function and psychomotor potential (including coordination and execution of movement potential), and/or executive functioning potential including problem solving potential, social processing, behaviour interaction potential, and social-emotional functioning potential.

In a preferred embodiment of the invention, cognitive potential is selected from the group consisting of vision potential, motor function and psychomotor potential (including coordination and execution of movement potential), and/or executive functioning potential including problem solving potential, social processing, behaviour interaction potential, and social-emotional functioning potential.

Through optimising these potentials the composition of the invention may optimise vision, motor function and psychomotor function (including coordination and execution of movement), and/or executive functioning including problem solving, social processing, behaviour interaction, and social-emotional functioning.

### Fatty Acids Derivatives

The compositions of the invention comprises one or more fatty acid derivatives.

Preferably, a fatty acid derivative may be comprised in the composition of the invention in an amount constituting up to 99.999% of the composition.

The term "fatty acid derivative" as used herein includes fatty acids and, in particular, free fatty acids, and/or a monoacylglycerol (hereinafter MAG), and/or a diacylglycerol (hereinafter DAG), and/or a triacylgylcerol (hereinafter TAG) and/or a cholesterol ester. In one preferred embodiment, the fatty acid derivative is a free fatty acid.

In another preferred embodiment, the fatty acid derivative is a MAG, DAG, TAG and/or a cholesterol ester. Even more preferably, the fatty acid derivative is a TAG.

The term "MAG" as used herein refers to a glycerol molecule in which one of the OH groups is modified to form an ester bond with a fatty acid.

Preferably, the MAG is a compound of formula (X)

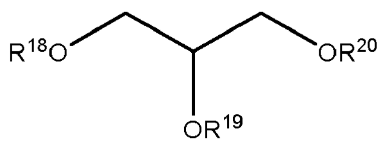

(X)

wherein two of $R^{18}$, $R^{19}$ and $R^{20}$ are H and one of $R^{18}$, $R^{19}$ and $R^{20}$ is a C4 to C44 saturated or unsaturated acyl group.

More preferably, two of $R^{18}$, $R^{19}$ and $R^{20}$ are H, and one of $R^{18}$, $R^{19}$ and $R^{20}$ is a C10 to C24 saturated or unsaturated acyl group, even more preferably a C14 to C24 saturated or unsaturated acyl group.

The term "DAG" as used herein refers to glycerol molecule in which two of the OH groups are modified to form ester bonds with fatty acids.

Preferably, the DAG is a compound of formula (X) wherein one of $R^{18}$, $R^{19}$ and $R^{20}$ are H, and two of $R^{18}$, $R^{19}$ and $R^{20}$ are each independently a C4 to C44 saturated or unsaturated acyl group.

More preferably, two of $R^{18}$, $R^{19}$ and $R^{20}$ are each independently a C10 to C24 saturated or unsaturated acyl group, even more preferably a C14 to C24 saturated or unsaturated acyl group.. The two C4 to C44 saturated or unsaturated acyl groups of $R^{18}$, $R^{19}$ and $R^{20}$ may be the same or different.

The term "TAG" as used herein refers to a glycerol molecule that has formed an ester bond with three fatty acids.

Preferably, the TAG as used herein is a compound of formula (X) wherein $R^{18}$, $R^{19}$ and $R^{20}$ are each independently a C4 to C44 saturated or unsaturated acyl group, more preferably, a C10 to C24 saturated or unsaturated acyl group, even more preferably a C14 to C24 saturated or unsaturated acyl group. The three C4 to C44 saturated or unsaturated acyl groups of $R^{18}$, $R^{19}$ and $R^{20}$ may all be the same, all different, or two may be the same and one different.

The term "cholesterol ester" as used herein refers to a compound of formula (XI):

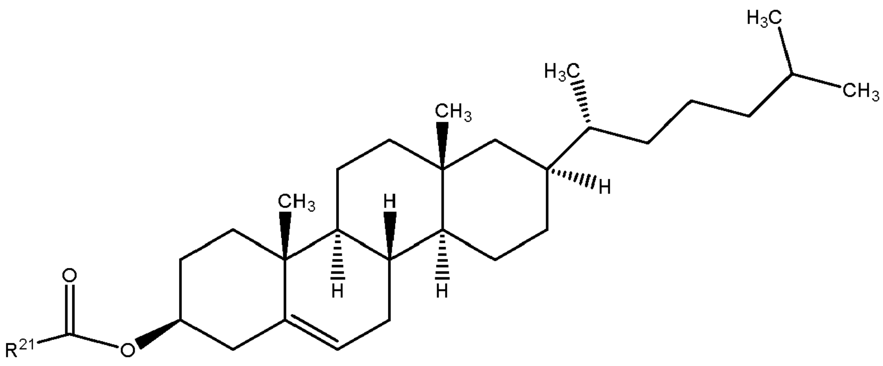

(XI)

wherein $R^{21}$ is a C2 to C43 branched or unbranched acyclic alkyl or acyclic alkenyl group.

More preferably, $R^{21}$ is a C9 to C43 branched or unbranched acyclic alkyl or acyclic alkenyl group which together with the adjacent carbonyl group corresponds to a C10 to C44 saturated or unsaturated fatty acid residue, even more preferably a C14 to C24 saturated or unsaturated fatty acid residue.

Preferably, the compositions of the invention comprise a fatty acid. A fatty acid is a carboxylic acid with a long aliphatic chain, which is either saturated or unsaturated. Most naturally occurring fatty acids have an unbranched chain of an even number of carbon atoms, from 4 to 28. Fatty acids are usually derived from triglycerides or phospholipids. Fatty acids are important sources of fuel because, when metabolized, they yield large quantities of ATP.

The term "fatty acid" as used herein refers to a compound of formula (XII)

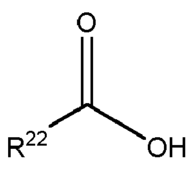

(XII)

wherein $R^{22}$ is a C2 to C43 branched or unbranched acyclic alkyl or acyclic alkenyl group.

More preferably, $R^{22}$ is a C9 to C43 branched or unbranched acyclic alkyl or acyclic alkenyl group, even more preferably a C9 to C23 branched or unbranched acyclic alkyl or acyclic alkenyl group.

In one preferred embodiment, the fatty acid is a saturated fatty acid.

In another preferred embodiment, the fatty acid is an unsaturated fatty acid.

Examples of saturated fatty acids include, but are not limited to, myristoleic acid, palmitoleic acid, sapienic acid, oleic acid, elaidic acid, vaccenic acid, linoleic acid, linoelaidic acid, $\alpha$-linolenic acid, arachidonic acid, eicosapentaenoic acid, erucic acid and docosahexaenoic acid.

Examples of unsaturated fatty acids include, but are not limited to, caprylic acid, capric acid, lauric acid, myristic acid, palmitic acid, stearic acid, arachidic acid, behenic acid, lignoceric acid and cerotic acid.

Non limiting examples of C10 to C44 saturated or unsaturated fatty acids that may be comprised in the fatty acid derivative i.e. that may be the free fatty acid or fatty acid from which the fatty acid residue(s) of the MAG, DAG, TAG and/or cholesterol ester may stem include; C10:0, C12:0, C14:0, C15:0, C16:0, C16:1n-7, C18:0, C18:1n-7, C18:1n-9, C18:2n-6, 18:3n-3, C20:0, C20:1n-9, C20:2n-6, C20:3n-6, C20:4n-6, 20:5n-3, C21:0, C22:0, C22:1n-9, C22:6n-3 C23:0, C24:1, in particular 24:1n-9, C25:0, C28:1, C30:2, C30:1, C30:0, C32:3, C32:2, C32:1, C32:0, C33:1, C34:3, C34:2, C34:1, C34:0, C35:2, C35:0, C36:4, C36:3, C36:2, C36:1, C36:0, C37:1, C37:0, C38:4, C38:3, C38:1, C38:0, C39:1, C39:0, C40:2, C40:1, C40:0, C41:2, C41:1, C41:0, C42:47, C42:3, C42:2, C42:1, C42:0, C44:3, C44:1. In particular, said fatty acids will be selected from the group consisting of: C10:0, C12:0, C14:0, C16:0, C16:1n-7, C18:0, C18:1n-7, C18:1n-9, C18:2n-6, 18:3n-3, C20:0, C20:1n-9, C20:2n-6, C20:3n-6, C20:4n-6, 20:5n-3, C22:0, C22:1n-9, C22:6n-3, C24:1, 24:1n-9.

Any fatty acid derivative suitable for ingestion by a subject for which the composition is intended to be consumed may be used in the invention.

In particular, the fatty acid derivative will come from natural sources, non limiting examples of which include, eggs, algae, fish oil, mould, yeast, seeds, plants e.g. soy, and animal sources e.g. bovine brains, and/or mammalian milk or extracts thereof. Non limiting examples of soy sources include soy lecithin-food additive, non limiting examples of mammalian milk include bovine, camel, sheep, goat milk including skilled milks. Non limiting extracts of milk include protein extracts, milk fat globule membranes (MFGM) and extracts comprising them. Fatty acid derivatives may also come from palm oil, tallow, lard, cotton seed oil, peanut oil.

It may be particularly beneficial if the fatty acid derivative comprises a saturated or unsaturated fatty acid selected from the group consisting of: C20:4n-6, C22:6n-3, C24:1n-9, C16:0, C18:1n-9, and C18.0, in particular C20:4n-6 and/or C22:6n-3 and/or C18:0. More particularly 22:6n-3 and/or C18:0.

A composition comprising a phospholipid, in particular sphingomyelin, phosphatidylcholine, phosphatidylserine, phosphatidylinositol, more particularly sphingomyelin, may be particularly effective if used in combination with one or more of these fatty acids.

C20:4n-6 is arachidonic acid hereinafter ARA. C22:6n-3 is docosahexaenoic acid hereinafter DHA. 24:1n-9 is nervonic acid. C18.0 is stearic acid. C16:0 is palmitic acid. C18:1n-9 is Oleic acid. Preferably, the fatty acid derivative is DAA and/or ARA and/or stearic acid. Most particularly a fatty acid derivative comprising DHA and/ or Stearic acid.

In one preferred embodiment, the composition according to the present invention comprises docosahexaenoic acid (hereinafter "DHA").

DHA is an omega-3 fatty acid that is a primary structural component of the human brain, cerebral cortex, skin, sperm, testicles and retina. It can be synthesized from alpha-linolenic acid or obtained directly from maternal milk or fish oil. DHA is a carboxylic acid (*-oic acid*) with a 22-carbon chain (*docosa-* is Greek for 22) and six (*hexa-*) *cis* double bonds (*-en-*); with the first double bond located at the third carbon from the omega end. The trivial name for DHA is cervonic acid, its systematic name is *all-cis*-docosa-4,7,10,13,16,19-hexa-enoic acid, and its shorthand name is 22:6(n-3) in the nomenclature of fatty acids.

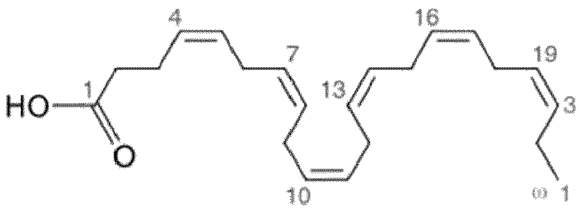

**DHA**

DHA may be incorporated in the compositions of the invention as a single species (as a fatty acid, in the form of a physiologically acceptable salt thereof or in the form of a triglyceride comprising it), as an ingredient consisting of a mixture of different DHA species, or by addition of a natural or synthetic ingredient comprising one or more DHA species.

Within the context of the present invention, the term "DHA" includes all the DHA present in the compositions on the invention, either in free form (as a fatty acid or physiologically acceptable salt thereof) or comprised in a triglyceride structure.

Preferably, DHA is comprised in the composition of the invention in an amount of about 0.001% to about 99.999% of the composition.

In another preferred embodiment, the composition according to the present invention comprises arachidonic acid (hereinafter "ARA").

ARA is a polyunsaturated fatty acid present in the phospholipids (especially phosphatidylethanolamine, phosphatidylcholine, and phosphatidylinositides) of membranes of the body's cells, and is abundant in the brain, muscles, and liver. Arachidonic acid is a carboxylic acid with a 20-carbon chain and four *cis*-double bonds; the first double bond is located at the sixth carbon from the omega end.

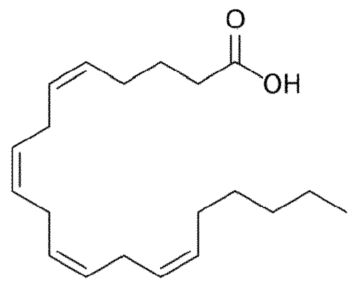

**ARA**

Within the context of the present invention, the term "ARA" includes all the ARA present in the compositions on the invention, either in free form (as a fatty acid or a physiologically acceptable salt thereof) or comprised in a triglyceride structure.

Preferably, ARA is comprised in the composition of the invention in an amount constituting from about 0.001% to about 99.999% of the composition.

In a preferred embodiment the composition according to the invention comprises a fatty acid derivative comprising DHA and/or ARA and/or nervonic acid and/or stearic acid, in particular a fatty acid derivative comprising DHA and/or ARA.

Preferably, a fatty acid derivative comprising DHA and/or ARA and/or nervonic acid and/or stearic acid may be comprised in the composition of the invention in an amount constituting up to 99.999% of the composition.

In one preferred embodiment, the composition comprises DHA in an amount such that the total daily intake derived from the composition of the invention is from about 100 to about 1500 mg, more preferably from about 250 to about 1200 mg, even more preferably from about 800 to about 1200 mg.

In one preferred embodiment, the composition comprises ARA in an amount such that the total daily intake derived from the composition of the invention is from about 100 to about 500 mg, more preferably from about 150 to about 450 mg, even more preferably from about 200 to about 400 mg, more preferably still, from about 250 to about 350 mg, or about 300 mg.

In one preferred embodiment, the composition comprises nervonic acid in an amount such that the total daily intake derived from the composition of the invention is from about 5 to about 80 mg, more preferably from about 5 to about 50 mg, even more preferably from about 8 to about 32 mg.

In one preferred embodiment, the composition comprises stearic acid in an amount such that the total daily intake derived from the composition of the invention is from about 5 to about 80 mg, more preferably from about 5 to about 50 mg, even more preferably from about 4 to about 20 mg.

Fatty acid derivatives comprising stearic acid are present in natural sources, for example, palm oil, tallow, lard, cotton seed oil, peanut oil.

Fatty acid derivatives comprising nervonic acid are present in natural sources, for example, the seed oils of Cardamine gracea, Heliphila longifola, Thlaspi perfoliatum, Tropaeolum speciosum, Lunaria biennis, Lunaria annua and Malania oleifera; the moulds Neocallismastix frontalis, Erysiphe graminis and Sphaerotheca humuli; the bacterium Pseudomonas atlantica; the yeast Saccharomyces cerevisiae and the marine diatom Nitzschia cylindrus.

Fatty acid derivatives comprising DHA and/or ARA are present in natural sources such as, for example egg, algae, fungus or fish oil.

Oils comprising fatty acid derivatives comprising DHA and/or ARA and generally other polyunsaturated fatty acids (PUFAs), in particular EPA (eicosapentaenoic acid), may be of various origin. Preferably, fatty acid derivatives comprising DHA are provided in the form of a fish oil comprising fatty acid derivatives comprising DHA and/or ARA. Fish oils generally comprise 5wt.% or more, preferably 10wt.% or more of fatty acid derivatives comprising DHA and/or ARA. Oils comprising substantial amounts of fatty acid derivatives comprising DHA and/or ARA, obtained from algae or microorganisms in general are also available. For example, oils harvested from algae comprising 10wt.% or more, for example 20wt.% or more of fatty acid derivatives, may be used.

In one particularly preferred embodiment, the composition according to the invention comprises ARA and DHA.

If the nutritional composition according to the present invention comprises fatty acid derivatives comprising ARA and DHA, said ingredients may for example be comprised in the composition of the invention in amounts resulting in a weight ratio of DHA:ARA in the range of 4:1 to 1:4, for example 3:1 to 1:3, for example 2:1 to 1:2, for example 1.5:1 to 1:1.5, in particular 1.1:1 to 1:1.1.

It may also be beneficial if the composition of the invention comprises a mixture of fatty acid derivatives wherein, the mixture is such that the weight ratio of unsaturated to saturated fatty acids and/or fatty acid residues in the composition of the invention is within the range 1:1 to 1:2; 1:1.2 to 1:1.9, 1:1.25 to 1:1.5; 1:3 to 1:4.

Further, when high amounts of fatty acid derivatives comprising DHA and/ or ARA are comprised in the composition of the invention, it may be particularly beneficial if the total amount of fatty acid derivatives comprising saturated long chain fatty acids, in particular C20/24 is increased. These saturated long chain fatty acids may be an important component of myelin enabling it to wrap around and enrobe axons. Preferably, the weight ratio of DHA and/or AA to these unsaturated long fatty acids in the composition of the invention is, for example, within the range 1:11:10; 1:2 to 1:9, 1: 3 to 1:4.5, 1:3.5 to 1:4.5.

## Additional Components

In one highly preferred embodiment, the compositions of the invention further comprise one or more of the following ingredients: a vitamin, a mineral, choline, and a phospholipid or a metabolite thereof, or a metabolic precursor thereof.

The amount of each of these additional ingredients is selected depending on whether the composition is intended to be administered/consumed once a day or more frequently.

When the composition of the invention of the invention comprises a fatty acid such as ARA and/or DHA and one or more of these additional ingredients it may have an improved effect in terms of promoting supporting and/or optimizing de novo myelination, in particular the de novo myelination trajectory, and/or brain structure, in particular the amount and spatial distribution of myelinated matter throughout the brain and/or brain connectivity, and/or cognitive potential and/or intellectual potential and/or learning potential and/or cognitive function. This may for example be because said ingredients effect de novo myelination in the same and/or separate complementary brain areas. The improved effect may be synergistic.

In one preferred embodiment, the composition of the invention comprises a phospholipid, or a metabolite thereof, or a metabolic precursor thereof, or a mixture thereof. The term "phospholipid" as used herein refers to any phospholipid. As used herein, the term phospholipid refers to a molecule that is made up of two fatty acids attached to a glycerol 'head.' The glycerol molecule is also attached to a phosphate group.

Preferably the phospholipid is a compound of formula (I)

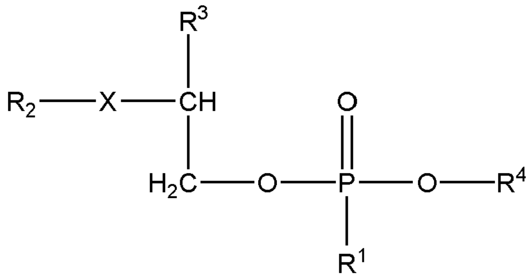

(I)

wherein:

- $R^1$ is O;
- X is NH or O;
- $R^2$ is a C2-C44 saturated or unsaturated, linear or branched acyl group;
- $R^3$ is a substituent of formula (II) or formula (III):

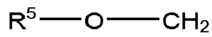

(II)

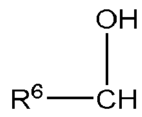

(III)

$R^4$ is selected from a C5 or C6 substituted or unsubstituted cyclic alkyl or cyclic alkenyl group, and $-(CH_2)_n-R^7$;
$R^5$ is a C2-C44 saturated or unsaturated, linear or branched acyl group;
$R^6$ is a C2-C44 saturated alkyl or alkenyl group;
$R^7$ is $-N(CH_3)_3^+$, $NH_3^+$, or a substituent of formula (IV):

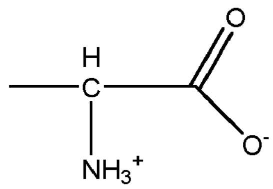

(IV)

and n is an integer from 1 to 4, preferably 1 or 2.

As used herein, the term "alkyl" includes both saturated straight chain and branched alkyl groups, which may be substituted (mono- or poly-) or unsubstituted. The term cyclic alkyl is to be construed accordingly. Preferably, the cyclic alkyl group is a C3-8, more preferably, a C3-6 cyclic alkyl group.
As used herein, the term "alkenyl" refers to a carbon chain containing one or more double bonds, which may be branched or unbranched, and substituted (mono- or poly-) or unsubstituted.
As used herein the term "acyl" refers to a group R'(C=O)-, where R' is a saturated (alkyl) or unsaturated (alkenyl) carbon chain.
As used herein the term "acyclic" refers to a group that is not cyclic.
In a preferred embodiment, $R^4$ is a C6 cyclic alkyl or cyclic alkenyl group substituted with one or more hydroxy groups. In one preferred embodiment, $R^4$ is derived from inositol ($C_6H_{12}O_6$), even more preferably myo-inositol, i.e. $R^4$ is:

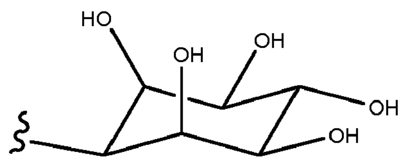

Non limiting examples of phospholipids include phosphatidylinositole, phosphatidylserine, phosphatidylethanolamine, sphingomyelin and phosphatidylcholine.
In one preferred embodiment, the phospholipid is selected from the group consisting of phosphatidyl choline, phosphatidyl inositole, phosphatidyl serine, phosphatidyl ethanolamine, sphingomyelin and mixtures thereof, and metabolic precursors and metabolites of any of the foregoing, and mixtures thereof.
Phosphatidylinositole is a compound of formula (V):

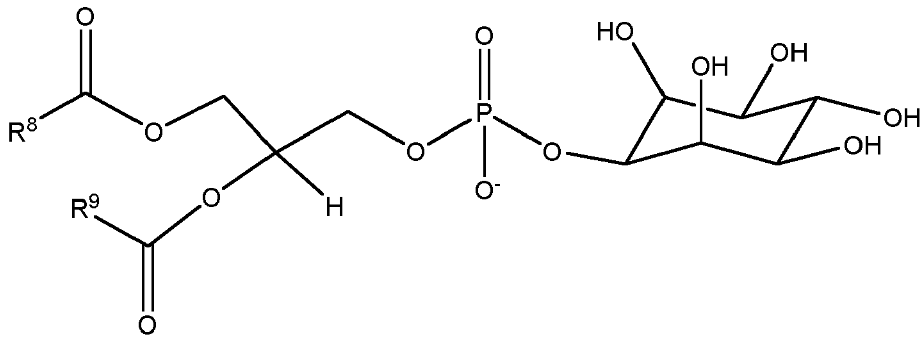

(V)

wherein $R^8$ and $R^9$ are each independently a C2 to C43 branched or unbranched acyclic alkyl or acyclic alkenyl group.

More preferably, $R^8$ and $R^9$ are each independently a C13 to C43 branched or unbranched acyclic alkyl or acyclic alkenyl group, which together with the adjacent carbonyl group corresponds to a C14 to C44 saturated or unsaturated fatty acid residue, even more preferably a C14 to C24 saturated or unsaturated fatty acid residue.

More particularly, $R^8$ and $R^9$ are C13 to C23 branched or unbranched acyclic alkyl, or acyclic alkenyl groups which together with their adjacent carbonyl group are C14 to C24 saturated or unsaturated fatty acid residues, wherein the fatty acids from which the fatty acid residues stem are selected from the group consisting of; C14:0, C15:0, C16:0, C18:0, C20:0, C20:3, C20:4, C21:0, C22:0, C23:0, C24:0, C18:1n-9, C18:2n-6, and C24:1n-9. Even more particularly C18:0, C18:1n-9, C18:2, C20:3, and C20:4.

As the skilled person would appreciate. The term Phosphatidylserine as used herein refers to Phosphatidyl-L-serine.

Phosphatidylserine is a compound of formula (VI):

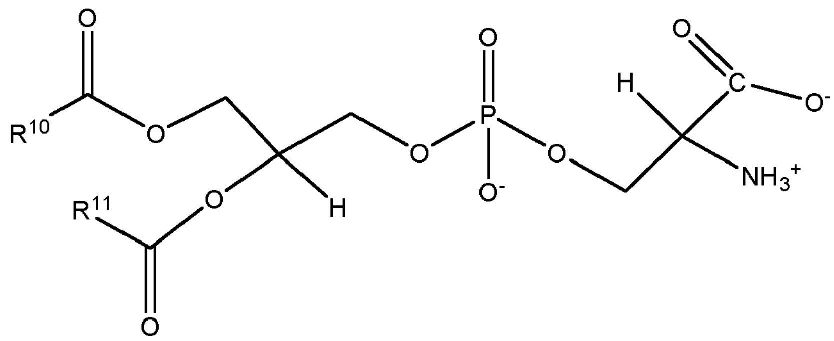

(VI)

wherein $R^{10}$ and $R^{11}$ are each independently a C2 to C43 branched or unbranched acyclic alkyl or acyclic alkenyl group.

More preferably, $R^{10}$ and $R^{11}$ are each independently a C13 to C43 branched or unbranched acyclic alkyl or acyclic alkenyl group which together with the adjacent carbonyl group corresponds to a C14 to C44 saturated or unsaturated fatty acid residue, even more preferably a C14 to C24 saturated or unsaturated fatty acid residue.

More particularly, $R^{10}$ and $R^{11}$ are C13 to C23 branched or unbranched acyclic alkyl, or acyclic alkenyl groups which together with their adjacent carbonyl group are C14 to C24 saturated or unsaturated fatty acid residues, wherein the fatty acids from which the fatty acid residues stem are selected from the group consisting of; C14:0, C15:0, C16:0, C18:0, C20:0, C20:3, C20:4, C21:0, C22:0, C23:0, C24:0, C18:1n-9, C18:2n-6, and C24:1n-9. Even more particularly C18:0, C18:1n-9, C20:4, and C22:6.

Phosphatidylethanolamine is a compound of formula (VII):

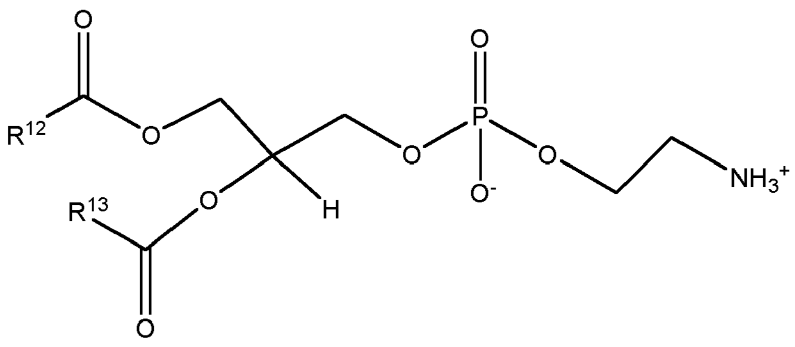

(VII)

wherein $R^{12}$ and $R^{13}$ are each independently a C2 to C43 branched or unbranched acyclic alkyl or acyclic alkenyl group.

More preferably, $R^{12}$ and $R^{13}$ are each independently a C13 to C43 branched or unbranched acyclic alkyl or acyclic alkenyl group which together with the adjacent carbonyl group corresponds to a C14 to C44 saturated or unsaturated fatty acid residue, even more preferably a C14 to C24 saturated or unsaturated fatty acid residue.

The term "sphingomyelin" as used herein refers to a lipid molecule, or mixture of lipid molecules, wherein a sphingosine backbone is acylated with a fatty acid residue at the amino group ($-NH_2$) and wherein the hydroxyl group at position 1 of the sphingosine backbone is linked to a phospho-choline or phospho-ethanolamine group.

Preferably, the sphingomyelin is a compound of formula (VIII) or a mixture of compounds of formula (VIII):

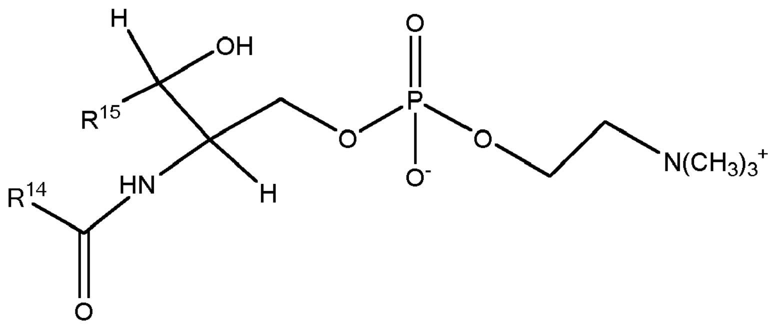

(VIII)

wherein $R^{14}$ and $R^{15}$ are each independently a C2 to C43 branched or unbranched acyclic alkyl or acyclic alkenyl group.

More preferably, $R^{14}$ is a C13 to C43 branched or unbranched acyclic alkyl or acyclic alkenyl group which together with the adjacent carbonyl group corresponds to a C14 to C44 saturated or unsaturated fatty acid residue.

Non limiting examples of C14 to C44 saturated or unsaturated fatty acids from which the fatty acid residue may stem include; C14:0, C15:0, C16:0, C18:0, C20:0, C21:0, C22:0, C23:0, C24:1, C25:0, C28:1, C30:2, C30:1, C30:0, C32:3, C32:2, C32:1, C32:0, C33:1, C34:3, C34:2, C34:1, C34:0, C35:2, C35:0, C36:4, C36:3, C36:2, C36:1, C36:0, C37:1, C37:0, C38:4, C38:3, C38:1, C38:0, C39:1, C39:0, C40:2, C40:1, C40:0, C41:2, C41:1, C41:0, C42:47, C42:3, C42:2, C42:1, C42:0, C44:3, C44:1.

Even more preferably, $R^{14}$ is a C13 to C23 branched or unbranched acyclic alkyl or acyclic alkenyl group which together with the adjacent carbonyl group corresponds to a C14 to C24 saturated or unsaturated fatty acid residue, wherein the fatty acid from which the fatty acid residue stemmed is selected from the group consisting of; C14:0, C15:0, C16:0, C18:0, C20:0, C21:0, C22:0, C23:0, C24:0, C18:1n-9, C18:2n-6, and C24:1n-9.

Even more preferably still, sphingomyelin is a mixture of compounds of formula (VIII) wherein the mixture is such that the total number of fatty acid residues ($R^{14}$ together with the adjacent carbonyl group) comprised in the mixture are predominately saturated fatty acids, and the least predominant are unsaturated fatty acids. More preferably, the mixture will be such that that 80% to 96% of said fatty acid residues in the mixture are saturated fatty acids, in particular C14, C15, C16, C18, C20, C22, C23, C24 saturated fatty acids, more particularly C16, C18, C20, C22 and C24.

Phosphatidylcholine is a compound of formula (IX):

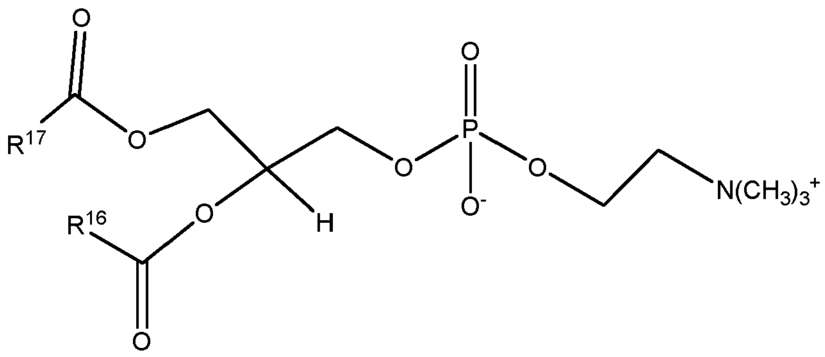

(IX)

wherein $R^{16}$ and $R^{17}$ are each independently a C2 to C43 branched or unbranched acyclic alkyl or acyclic alkenyl group.

More preferably, $R^{16}$ and $R^{17}$ are each independently a C13 to C43 branched or unbranched acyclic alkyl or acyclic alkenyl group which together with the adjacent carbonyl group corresponds to a C14 to C44 saturated or unsaturated fatty acid residue, even more preferably a C14 to C24 saturated or unsaturated fatty acid residue.

More particularly, $R^{16}$ and $R^{17}$ are C13 to C23 branched or unbranched acyclic alkyl, or acyclic alkenyl groups which together with their adjacent carbonyl group are C14 to C24 saturated or unsaturated fatty acid residues, wherein the fatty acids from which the fatty acid residues stem are selected from the group consisting of; C14:0, C15:0, C16:0, C16:1, C18:0, C20:0, C20:1, C20:3, C20:4, C21:0, C22:0, C22:6, C23:0, C24:0, C18:1n-9, C18:2n-6, and C24:1n-9. Even more particularly C14:0, C16:0, C18:0, C18:1n-9, C18:2n-6, C20:1, C20:3, C20:4, and C22:6. Particularly preferred phospholipids include phosphatidylcholine, phosphatidylinositole, phosphatidylserine, and sphingomyelin, more particularly phosphatidylcholine and/or sphingomyelin.

In a preferred embodiment of the present invention, the phospholipid is phosphatidylcholine, phosphatidylinositole, phosphatidylserine, or sphingomyelin, or a metabolic precursor or metabolite of any of the foregoing. More preferably, the phospholipid is phosphatidylcholine or sphingomyelin, or a metabolic precursor or metabolite of either of the foregoing.

Preferably, a phospholipid, a metabolic precursor and/or metabolite thereof is comprised in the composition in an amount up to 99.999% of the composition.

Preferably, sphingomyelin, a metabolic precursor and/or metabolite thereof is comprised in the composition in an amount up to 99.999% of the composition.

More preferably, the composition comprises sphingomyelin in an amount 200 to 1000 mg, 400 to 700mg, 650mg.

Preferably, phosphatidylcholine, a metabolic precursor and/or metabolite thereof is comprised in the composition in an amount up to 99.999% of the composition.

More preferably, the composition comprises phosphatidylcholine in an amount 300 to 5000mg, 1000 to 5000mg, 3000 to 5000mg, 4000 to 4500mg.

Preferably, phosphatidylinositole, a metabolic precursor and/or metabolite thereof is comprised in the composition in an amount up to 99.999% of the composition.

More preferably, the composition comprises phosphatidylinositole in an amount 50 to 400, 100 to 250, 200 to 210mg.

Preferably, phosphatidylserine, a metabolic precursor and/or metabolite thereof is comprised in the composition in an amount up to 99.999% of the composition.

More preferably, the composition comprises phosphatidylserine in an amount 50 to 500mg, 200 to 500mg, 400mg.

Preferably, phosphatidylethanolamine, a metabolic precursor and/or metabolite thereof is comprised in the composition in an amount up to 99.999% of the composition.

More preferably, the composition comprises phosphatidylethanolamine in an amount 50 to 500mg, 200 to 500mg, 400mg.

If a metabolic precursor and/or metabolite of one or more phospholipids is used in a composition in place of or in combination with a phospholipid, said compounds may be used in amounts such that the level of phospholipids physiologically delivered by said composition is in line with those set out hereinabove. It is well within the purview of the skilled person to determine appropriate amounts.

The term metabolic precursor and/or metabolite of one or more phospholipid as used herein does not include choline.

Non limiting examples of metabolic precursors and/or metabolites of phospholipids, in particular sphingomyelin, phosphatidylcholine, phosphatidylinositole, phosphatidylserine and/or phosphatidylethanolamine are: galactoceramides, glucoceramides, sphingosine, sphingosine-1-phosphate, ceramide, D-erythro-dihydroceramide and ceramide-1-phosphate , and gangliosides. Particularly effective phospholipids include phosphatidylcholine, phosphatidylserine, phosphatidylinositol and/or sphingomyelin, in particular sphingomyelin.

In a preferred embodiment of the present invention the phospholipid is phosphatidylcholine, phosphatidylserine, phosphatidylinositol, sphingomyelin and/or a metabolic precursor and/or metabolite of any of the foregoing and/or combinations of any of the foregoing. Preferably, the phospholipid is sphingomyelin, a metabolic precursor and/or metabolite thereof.

Particularly effective metabolic precursors and/or metabolites of phospholipids, in particular sphingomyelin, include ceramide and gangliosides and ceramide -1-phosphate and d-erythro-dihydroceramide.

The term "ceramide" indicates a lipid molecule wherein a sphingosine backbone is acylated with a fatty acid residue. When the term ceramide is used in the present specification, it may identify a single ceramide species as well as a mixture of single ceramide species.

Preferably, the ceramide is a compound of formula (IXa), or a mixture of compounds of formula (IXa):

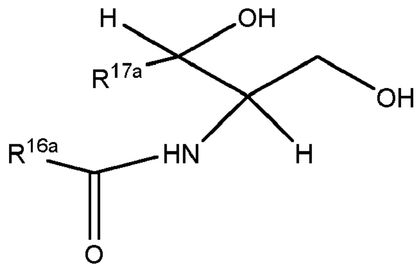

(IXa)

wherein $R^{16a}$ and $R^{17a}$ are each independently a C2 to C43 branched or unbranched acyclic alkyl or acyclic alkenyl group.

More preferably, $R^{16a}$ is a C13 to C43 branched or unbranched acyclic alkyl or acyclic alkenyl group which together with the adjacent carbonyl group corresponds to a C14 to C44 saturated or unsaturated fatty acid residue.

Non limiting examples of C14 to C44 saturated or unsaturated fatty acids from which the fatty acid residue may stem include; C14:0, C15:0, C16:0, C18:0, C20:0, C21:0, C22:0, C23:0, C24:1, C25:0, C28:1, C30:2, C30:1, C30:0, C32:3, C32:2, C32:1, C32:0, C33:1, C34:3, C34:2, C34:1, C34:0, C35:2, C35:0, C36:4, C36:3, C36:2, C36:1, C36:0, C37:1, C37:0, C38:4, C38:3, C38:1, C38:0, C39:1, C39:0, C40:2, C40:1, C40:0, C41:2, C41:1, C41:0, C42:47, C42:3, C42:2, C42:1, C42:0, C44:3, C44:1.

Even more preferably, $R^{16a}$ is a C13 to C23 branched or unbranched acyclic alkyl or acyclic alkenyl group which together with the adjacent carbonyl group corresponds to a C14 to C24 saturated or unsaturated fatty acid residue, wherein the fatty acid from which the fatty acid residue stemmed is selected from the group consisting of; C14:0, C15:0, C16:0, C18:0, C20:0, C21:0, C22:0, C23:0, C24:0, C18:1n-9, C18:2n-6, and C24:1n-9, and more particularly the group consisting of C16:0, C18:0, C20:0, C22:0 and C24:0.

Even more preferably still, the ceramide is a mixture of compounds of formula (IXa) wherein the mixture is such that the total number of fatty acid residues ($R^{16a}$ together with the adjacent carbonyl group) comprised in the mixture are predominately saturated fatty acids, and the least predominant are unsaturated fatty acids. More preferably, the mixture will be such that that 80% to 96% of said fatty acid residues in the mixture are saturated fatty acids, in particular C14, C15, C16, C18, C20, C22, C23, C24 saturated fatty acids, more particularly C16, C18, C20, C22 and C24.

The term "ganglioside" as used herein indicates an oligoglycosylceramide lipid molecule comprising the residue of a ceramide of formula IXa as defined herein. When the term ganglioside is used in the present specifications, it may identify a single ganglioside species as well as a mixture of single ganglioside species comprising the residue of a ceramide of formula IXa as defined herein.

Particularly effective gangliosides may be monosialoganglioside-3 (GM3) gangliosides and/or disialogangliosides 3 (GD3) gangliosides.

Ceramide -1-phosphate and d-erythro-dihydroceramide with comprise a residue of a ceramide of formula IXa as defined herein.

Spingomyelin may be synthesised from ceramide and phosphatidylcholine. Accordingly, it may be particularly beneficial if ceramide and/or one or more ganglioside is used in combination with phosphatidylcholine, a metabolic precursor or metabolite thereof.

The phospholipid, metabolic precursor and/or metabolite thereof, comprised in the composition of the invention may be natural, synthetic or a mixture thereof. Said metabolic precursor and/or a metabolite, may be used in the composition of the invention in their pure form, or substantially pure form. Alternatively, they may be added in the form of a

source comprising them.

Any source of a phospholipid metabolic precursors and/or metabolite thereof, suitable for ingestion by a subject for which the composition is intended to be consumed may be used in the invention.

In particular the phospholipid, metabolic precursor or metabolite thereof, will come from natural sources, non limiting examples of which include, eggs, soy, bovine brains, and/or mammalian milk or extracts thereof. Non limiting examples of soy sources include soy lecithin-food additive, non limiting examples of mammalian milk include bovine, camel, sheep, goat milk including skilled milks. Non limiting extracts of milk include protein extracts e.g. whey protein and casein, milk fat globule membranes (MFGM) and extracts comprising them.

A particularly useful source of a phospholipid, metabolic precursor or metabolite thereof, in particular sphingomyelin, that may be used in the present invention is a bovine milk whey protein concentrate enriched in alpha-lactalbumin, and/or none pure alpha-lactalbumin which has been extracted from milk whey protein, in particular bovine milk whey protein.

Alpha-Lactalbumin is a high-quality, easy-to-digest whey protein and is the primary protein found in HM. Alpha-lactalbumin and/or an alpha-lactalbumin enriched milk fraction is ideal for use in lower protein infant formulas due to its high content of essential amino acids, particularly tryptophan. Although alpha-Lactalbumin is in itself a protein non pure sources may comprise sphingomyelin.

In a preferred embodiment a phospholipid a metabolic precursor or metabolite thereof, in particular sphingomyelin, is used in the form of a whey protein concentrate enriched in alpha-lactalbumin or as alpha-lactalbumin.

In a more preferred embodiment, a whey protein concentrate enriched in alpha-lactalbumin or alpha-lactalbumin having a phospholipid content, in particular sphingomyelin content higherthan 500 mg/100g dry weight of the composition is used.

Another particularly useful source of phospholipid, metabolic precursor, or metabolite thereof, may be MFGM or extracts comprising them, in particular MFGM, or extracts comprising them from bovine milk. It may be particularly beneficial if the MFGM or extracts comprising them comprises at least 1%, 2%, 5%, 10%, 20%, 30%, 40% phospholipids and/or at least 0.1%, 0.2%, 0.5% to 5%, 0.8% to 3%, 1% to 2%, 1.6%, 1.9%, 1.8% of phosphatidylcholine, phosphatidylinositole, phosphatidylserine, phosphatidylethanolamine, and/or sphingomyelin. The MFGM may also further comprise magnesium, phosphorus and or calcium, preferably in concentrations ranging from 0.05% to 2%, 0.1% to 0.4%.

A composition of the invention comprising a phospholipid and/or a metabolic precursor and/or a metabolite thereof, in particular sphingomyelin, phosphatidylcholine and/or phosphatidylinositol, may be particularly effective at supporting, promoting or optimising de novo myelination, in particular the de novo myelination trajectory, and/or brain structure, in one or more of the following brain areas; cerebellum, visual cortex, corpus callosum, internal capsule, frontal lobe, parietal lobe, temporal lobe, motor cortex, frontal cortex.. These brain areas are associated with one or more of the following: vision, motor function (including coordination and execution of movement) , hemispherical interaction, language function, auditory function (including listening and attention), working memory, executive functioning including problem solving, social processing, and behaviour interaction, spatial reasoning, and language.

In one preferred embodiment, the composition comprises one or more vitamins. The term vitamin as used herein refers to any vitamin. Non limiting examples of vitamins include: vitamin A, vitamin B1, vitamin B2, vitamin B6, vitamin K, vitamin C, vitamin D, niacin, biotin, pantothenic acid, folic acid, vitamin B12, and combinations thereof.

In one preferred embodiment, the composition comprises folic acid. Folic acid may be incorporated in the nutritional compositions of the invention as such or in the form of a physiologically acceptable salt thereof (folate) or mixtures thereof. Within the context of the present invention, the term "folic acid" includes all the folic acid present in the compositions of the invention either as such or in the form of a physiologically acceptable salt thereof or mixtures thereof.

Preferably, folic acid is comprised in the composition in an amount constituting about 0.001% to about 99.999% of the composition.

In a preferred embodiment, the composition according to the present invention comprises levels of folic acid such that the total daily intake derived from the composition of the invention is from about 50 to about 1000 μg, more preferably from about 60 to about 1000 μg, even more preferably, from about 70 to about 700 μg, even more preferably, from about 100 to about 500 μg, more preferably still from about 200 to about 400 μg.

In one preferred embodiment, the composition comprises an amount of folic acid such that the total daily intake derived from the composition of the invention will not exceed about 1000 μg.

In one preferred embodiment, the composition of the invention comprises vitamin B12. Vitamin B12 may be comprised in the composition in an amount constituting from about 0.001% to about 99.999% of the composition.

In one preferred embodiment, the composition comprises an amount of vitamin B12 such that the total daily intake derived from the composition of the invention is from about 0.2 to about 250 μg, more preferably from about 0.26 to about 50 μg, more preferably from about 0.5 to about 30 μg, even more preferably from about 1 to about 10 μg, more preferably still, from about 2 to about 6 μg.

In one highly preferred embodiment, the composition comprises an amount of vitamin B12 such that the total daily intake derived from the composition of the invention is about 250 μg.

In one preferred embodiment, the composition comprises an amount of vitamin B12 such that the total daily intake derived from the composition of the invention does not exceed about 50 μg.

Vitamin B12 may be incorporated in the compositions of the invention as such or in the form of one physiologically acceptable salt thereof or mixtures thereof.

A composition of the invention comprising a vitamin, in particularly folic acid and/or vitamin B12, may be particularly effective at supporting, promoting or optimising de novo myelination, in particular the de novo myelination trajectory, and/or brain structure in one or more of the following brain areas; cerebellum, brain stem, temporal lobe, frontal lobe, visual cortex, motor and somatosensory cortices. These brain areas are associated with-Motor function (including coordination and execution of movement), visual function and psychomotor function.

In one preferred embodiment, the composition comprises a mineral nutrient. The term mineral as used herein may refer to any mineral. Non limiting examples of minerals include: iron, zinc, calcium, phosphorus, copper, magnesium iodine, manganese, chloride, potassium, sodium, selenium, chromium, and combinations thereof. Minerals are usually added in salt form.

In one preferred embodiment, the composition according to the present invention comprises iron. Particularly effective minerals include iron and/or copper and/or zinc and/or calcium and/or phosphorus and/or magnesium, in particular iron.

In a preferred embodiment, the composition of the invention comprises iron.

Iron is preferably comprised in the composition of the invention in an amount constituting up to 99.999% of the composition.

Iron may be incorporated in the compositions of the invention in the form of one physiologically acceptable salt such as, for example: ferric citrate, ferric phosphate, ferric pyrophosphate, ferrous ascorbate, ferrous carbonate, ferrous citrate, ferrous fumarate, ferrous gluconate, ferrous lactate, ferrous sulfate or mixtures thereof.

Iron may also be incorporated in the composition of the invention in the form of a physiologically acceptable iron complex (such as for example EDTA ferric sodium salt) or mixtures thereof.

$Fe^{2+}$ is more bioavailable and it may therefore be more beneficial if iron is added into the composition in the form of a ferrous salt or complex e.g. a ferrous salts listed hereinabove.

In a preferred embodiment, the composition according to the present invention comprises levels of iron such that the total daily intake derived from the composition of the invention is from about 2 to about 50 mg, more preferably, from about 2.5 to about 45 mg, even more preferably, from about 2.7 to about 45 mg, more preferably still from about 3 to about 30 mg, or about 5 to about 20 mg.

In a preferred embodiment, the composition according to the present invention comprises levels of iron such that the total daily intake derived from the nutritional composition of the invention will not exceed about 45 mg.

In another preferred embodiment, the composition according to the present invention comprises levels of iron such that the total daily intake derived from the nutritional composition of the invention will not exceed 40 mg.

In a preferred embodiment the composition of the invention comprises zinc.

Preferably, zinc is comprised in the composition of the invention in an amount constituting up to 99.999% of the composition.

Preferably, zinc is comprised in the composition in an amount such that the total daily intake derived from the composition of the invention is from about 1 to about 50 mg, more preferably from about 1 to about 40 mg, more preferably, from about 1.1 to about 40 mg, even more preferably from about 2 to about 20 mg, even more preferably, from about 5 to about 15 mg, even more preferably from about 8 to about 12 mg.

In one highly preferred embodiment, the zinc is comprised in the composition in an amount such that the total daily intake derived from the composition is about 10 mg.

In a preferred embodiment, the composition according to the present invention comprises levels of zinc such that the total daily intake derived from the nutritional composition of the invention will not exceed about 40 mg.

Zinc may be incorporated in the compositions of the invention in the form of a physiologically acceptable salt and /or via any source comprising Zinc, more specifically $Zn^{2+}$, such as, for example: zinc nitrate, zinc sulfate, zinc gluconate, zinc acetate or mixtures thereof, or in the form of a physiologically acceptable zinc complex (such as for example zinc piccolinate) or mixtures thereof.

In a preferred embodiment the composition of the invention comprises copper.

Preferably, copper is comprised in the composition of the invention in an amount up to 99.999% of the composition.

Preferably, copper is comprised in the composition in an amount such that the total daily intake derived from the composition of the invention is from about 0.1 to about 10 mg, more preferably from about 1 to about 8 mg, more preferably, from about 2 to about 6 mg, even more preferably from about 2 to about 5 mg.

In a preferred embodiment, the composition according to the present invention comprises levels of copper such that the total daily intake derived from the nutritional composition of the invention will not exceed about 10 mg.

Copper may be incorporated in the composition of the invention as such or in the form of a physiologically

acceptable salt and/or via any source comprising copper, more specifically Cu2+. For example copper may be incorporated into the composition as: copper sulfate and/or copper gluconate and/or copper carbonate, and/or copper citrate, and/or copper-lysine complex.

In a preferred embodiment the composition of the invention comprises magnesium.

Preferably, magnesium is comprised in the composition of the invention in an amount up to 99.999% of the composition.

Preferably, magnesium is comprised in the composition in an amount such that the total daily intake derived from the composition of the invention is from about 35 to about 350 mg, more preferably from about 50 to about 250 mg, more preferably, from about 100 to about 200 mg.

In a preferred embodiment, the composition according to the present invention comprises levels of magnesium such that the total daily intake derived from the nutritional composition of the invention will not exceed 350 mg.

Magnesium may be incorporated in the composition of the invention as such or in the form of a physiologically acceptable salt and/or via any source comprising magnesium, more specifically Mg2+. For example, magnesium carbonate, magnesium chloride, magnesium oxide, magnesium sulphate, magnesium gluconate, magnesium hydroxide, magnesium salts of citric acid, magnesium salts of orthophosphoric acid.

In a preferred embodiment the composition of the invention comprises calcium.

Preferably, calcium is comprised in the composition of the invention in an amount up to 99.999% of the composition.

Preferably, calcium is comprised in the composition in an amount such that the total daily intake derived from the composition of the invention is from about 100 to about 2500 mg, more preferably from about 200 to about 2000 mg, more preferably, from about 250 to about 1500 mg, even more preferably from about 500 to about 1000 mg.

In a preferred embodiment, the composition according to the present invention comprises levels of calcium such that the total daily intake derived from the nutritional composition of the invention will not exceed 2500 mg.

Calcium may be incorporated in the composition of the invention as such or in the form of a physiologically acceptable salt and/or via any source comprising calcium, more specifically Ca2+. For example calcium carbonate, calcium chloride, calcium salts of citric acid, calcium gluconate, calcium glycerophosphate, calcium lactate, calcium hydroxide, calcium salts of orthophosphoric acid.

In a preferred embodiment the composition of the invention comprises phosphorus.

Preferably, phosphorus is comprised in the composition of the invention in an amount up to 99.999% of the composition.

Preferably, phosphorus is comprised in the composition in an amount such that the total daily intake derived from the composition of the invention is from about 70 to about 3500 mg, more preferably from about 100 to about 2500 mg, more preferably, from about 200 to about 2000 mg, even more preferably from about 300 to about 1500 mg, even more preferably, from about 500 to about 1000 mg.

In a preferred embodiment, the composition according to the present invention comprises levels of phosphorus such that the total daily intake derived from the nutritional composition of the invention will not exceed 3500 mg .

Phosphorus may be incorporated in the composition of the invention as such or in the form of a physiologically acceptable salt and/or via any source comprising phosphorus for example: calcium phosphate, calcium hydrogen phosphate

In a preferred embodiment the composition of the invention comprises choline.

A composition of the invention comprising a mineral, in particular one or more of iron, zinc, copper, calcium, magnesium and phosphorus, may be particularly effective at supporting, promoting or optimising de novo myelination, in particular the de novo myelination trajectory, and/or brain structure, in one or more of the following brain areas; Cerebellum, brainstem, visual cortex, motor and somatosensory cortices, corpus callosum, frontal cortex, temporal white matter, internal capsule, prefrontal cortex, motor cortex. These brain areas are associated with Motor function (including coordination and execution of movement), visual function, hemispherical interaction, executive functioning, working memory, problem solving, social-emotional functioning, language, auditory function, problem solving, and/or working memory.

Choline is preferably comprised in the composition of the invention in an amount up to 99.999% of the composition.

The term "choline" identifies quaternary ammonium salts containing the N,N,N-trimethylethanolammonium cation and having the structure shown below:

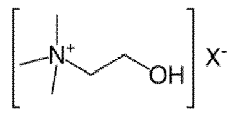

**Choline**

Unless stated otherwise, within the context of the present invention, the term "choline" should be intended to identify all the choline present in the nutritional compositions on the invention, in free form (or as a salt thereof)such as for example: choline hydroxide.

Preferably, choline is comprised in the composition in an amount such that the total daily intake derived from the composition of the invention is from about 100 to about 1000 mg, more preferably from about 200 to about 600 mg, more preferably, from about 250 to about 550 mg, even more preferably from about 300 to about 500 mg.

Preferably, choline is comprised in the composition in an amount such that the total daily intake of cholinederived from the composition of the invention is about 450 mg.

Choline may be incorporated in the composition of the invention as such or in the form of one physiologically acceptable salt such as, for example: choline chloride, choline citrate, choline bitartrate or mixtures thereof.

A composition of the invention comprising choline may be particularly effective at supporting, promoting or optimising de novo myelination, in particular the de novo myelination trajectory, and/or brain structure, in the following brain areas; cerebellum, internal capsule, motor cortex, visual cortex, thalamus, parietal cortex, and frontal lobe. These brain areas are associated with motor function (including coordination and execution of movement), vision, working memory and/or executive functioning and/or social-emotional reasoning and/or spatial reasoning.

The person skilled in the art can identify appropriate amounts of the above mentioned nutrients, metabolic precursors or metabolites thereof based on the nature and purpose of the composition.

In one preferred embodiment, the composition according to the invention comprises a fatty acid, preferably DHA and/or ARA, folic acid, iron and choline.

In one preferred embodiment, the composition according to the invention comprises a phospholipid, preferably phosphatidyl choline and/or sphingomyelin, folic acid, iron, choline, DHA, ARA, and zinc.

In one preferred embodiment, the composition according to the invention comprises a fatty acid, preferably DHA and/or ARA, iron and choline.

In another preferred embodiment, the composition according to the invention comprises sphingomyelin, iron and a fatty acid, preferably DHA and/or ARA.

In another preferred embodiment, the composition according to the invention comprises a fatty acid, preferably DHA and/or ARA, iron and folic acid.

In another preferred embodiment, the composition according to the invention comprises sphingomyelin, a fatty acid, preferably DHA and/or ARA, and choline.

In another preferred embodiment, the composition according to the invention comprises sphingomyelin, a fatty acid, preferably DHA and/or ARA, and iron.

In another preferred embodiment, the composition according to the invention comprises sphingomyelin, a fatty acid, preferably DHA and/or ARA, and folic acid.

In another preferred embodiment, the composition according to the invention comprises a fatty acid, preferably DHA and/or ARA, choline and folic acid.

In another preferred embodiment, the composition according to the invention comprises sphingomyelin, folic acid, iron and a fatty acid, preferably DHA and/or ARA.

In another preferred embodiment, the composition according to the invention comprises a fatty acid, preferably DHA and/or ARA, folic acid, choline and iron.

In another preferred embodiment, the composition according to the invention comprises sphingomyelin, folic acid, a fatty acid, preferably DHA and/or ARA, and choline.

In another preferred embodiment, the composition according to the invention comprises sphingomyelin, iron, a fatty acid, preferably DHA and/or ARA, and choline.

In another preferred embodiment, the composition according to the invention comprises sphingomyelin, folic acid, iron, choline and a fatty acid, preferably DHA and/or ARA.

In an embodiment, the composition according to the invention comprises a fatty acid derivative comprising DHA and/or ARA, vitamin B12 and/or folic acid, sphingomyelin and iron.

The person skilled in the art may identify appropriate amounts of the above mentioned nutrients, metabolic precursors or metabolites thereof based on the nature, purpose, the target subject and the dosage of the composition e.g. how many times per day the composition is to be ingested by the subject. Typically an effective dose will depend on age, size and health status of the subject, on the subject's lifestyle, the amounts of nutrients in the composition, and

maybe on the gender of the subject.

The person skilled in the art would be able to identify appropriate amounts of the above mentioned nutrients, metabolic precursors or metabolites in the composition in order to achieve their highest permitted levels after administration.

**Preferred composition types**

The composition of the invention may be any type of composition suitable for direct administration to a female subject.

Compositions according to the present invention are preferably in a solid form. The composition may, for example, be in the form of a chewable tablet, dispersible tablet, capsule, lozenge, pastille, chewing gum, powder (e.g. in a sachet), stickpack sachets, or bottle with powder in the cap. Preferably the composition is in the form of a tablet, capsule or powder. The tablet or capsule may be provided as a unit dosage form for, e.g. once or twice daily, preferably once daily, administration. A powder composition may be contained in a sachet. A powder composition according to the present invention may be used to sprinkle onto a food or beverage. A particularly preferred embodiment provides a composition according to the invention in the form of a sachet containing a powder, wherein the powder can be dispersed into a beverage (e.g. water, fruit juice, milk, etc.) to provide a palatable nutrient liquid for oral administration.

In one preferred embodiment, the composition is a nutritional composition. The nutritional composition may be a nutritionally complete formula, a nutritional supplement, a food product such as a dairy product, a chilled or shelf stable beverage or a soup, a dietary supplement, a meal replacement, or a nutritional bar for example.

In one preferred embodiment, the composition is a foodstuff intended for consumption by an adult, in particular a pregnant female.

The term "nutritional composition" as used herein refers to a composition that nourishes a subject. The composition may be a nutritionally complete formula, for example including a source of protein, carbohydrate and fat. This nutritional composition may be taken enterally, parenterally or intravenously. In one preferred embodiment, the composition is taken enterally and more preferably, orally.

In one preferred embodiment of the present invention, the composition comprises one or more of a protein source, a lipid source and a carbohydrate source.

For example such a composition may comprise protein in the range of about 2 to 6 g/100 kcal, lipids in the range of about 1.5 to 3 g/100kcal and/or carbohydrates in the range of about 1.7 to 12 g/100 kcal.

If the composition is liquid, its energy density may be between 60 and 75 kcal/100ml.

If the composition is solid, its energy density may be between 60 and 75 kcal/100g.

In one preferred embodiment, the composition is a synthetic nutritional composition.

The expression "synthetic composition" means a mixture obtained by chemical and/or biological means.

Preferably, the composition is a hypoallergenic nutritional composition. As used herein, the term "hypoallergenic nutritional composition" means a nutritional composition which is unlikely to cause allergic reactions.

In one preferred embodiment of the invention, the composition is selected from the group consisting of a pharmaceutical composition, a food product, a food extract, drink, food additive, a pet care product, a nutraceutical, and a nutritional supplement.

In one highly preferred embodiment, the composition is a maternal supplement. The supplement is preferably taken throughout pregnancy to build up maternal stores of the various constituent components, although supplementation in the second and more particularly the third trimesters is believed to be particularly advantageous. Likewise supplementation may continue after birth either via continued consumption of the composition by the mother if the baby is to be breast fed, or by administering a similar composition directly to the baby, for example by way of an infant formula used to feed the baby.

In one highly preferred embodiment, the composition is for use in conjunction with an infant formula and/or starter infant formula/and or growing up milk which is administered to the infant after birth. The composition can also be used in conjunction with baby food and/or a fortifier. Preferably, an infant formula and/or starter infant formula/and or growing up milk and/or baby food and/or fortifier also comprises a fatty acid, preferably DHA and/or ARA, or a mixture thereof, which further promotes, supports or optimizes one or more of the following: (i) de novo myelination; (ii) brain structure; (iii) brain connectivity; (iv)intellectual potential; (v) cognitive potential; and (vi) learning potential; (vii) cognitive function in the infant.

The expression "infant formula" means a foodstuff intended for particular nutritional use by infants during the first four to six months of life and satisfying by itself the nutritional requirements of this category of person (Article 1.2 of the European Commission Directive 91/321/EEC of May 14, 1991 on infant formulae and follow-on formulae).

The expression "starter infant formula" means a foodstuff intended for particular nutritional use by infants during the first four months of life.

The expression "follow-on formula" means a foodstuff intended for particular nutritional use by infants aged

over four months and constituting the principal liquid element in the progressively diversified diet of this category of person.

Within the context of the present invention, the term "Growing up milk (GUM)" indicates nutritional formula which may be given to children after stopping the infant formula. The "growing-up milks" (or GUMs) are given from one year onwards. It is generally a milk-based beverage adapted for the specific nutritional needs of young children.

The expression "baby food" means a foodstuff intended for particular nutritional use by infants during the first years of life.

The expression "fortifier" refers to liquid or solid nutritional compositions suitable for mixing with breast milk or infant formula.

The term "weaning period" means the period during which the mother's milk is substituted by other food in the diet of an infant.

The "mother's milk" refers to the breast milk or colostrum of the mother (= Human Breast Milk= HBM).

The compositions of the invention may further comprise any other additional ingredients or excipients known to be employed in the type of composition in question. Non-limiting examples of such additional ingredients include: proteins, amino acids, carbohydrates, oligosaccharides, lipids, prebiotics or probiotics, essential fatty acids, nucleotides, nucleosides, other vitamins, minerals and other micronutrients.

The type of protein is not believed to be critical to the present invention. Thus, protein sources based on whey, casein and mixtures thereof may be used, for example. As far as whey proteins are concerned, acid whey or sweet whey or mixtures thereof may be used as well as alpha-lactalbumin and betalactoglobulin in whatever proportions are desired. The whey protein may be modified sweet whey. Sweet whey is a readily available by-product of cheese making and is frequently used in the manufacture of infant formulae based on cows' milk. However, sweet whey includes a component which is undesirably rich in threonine and poor in tryptophan called caseino-glyco-macropeptide (CGMP). Removal of the CGMP from sweet whey results in a protein with a threonine content closer to that of human milk. This modified sweet whey may then be supplemented with those amino acids in respect of which it has a low content (principally histidine and tryptophan). A process for removing CGMP from sweet whey is described in EP 880902 and an infant formula based on this modified sweet whey is described in WO 01/11990. The proteins may be intact or hydrolysed or a mixture of intact and hydrolysed proteins. It may be desirable to supply partially hydrolysed proteins (degree of hydrolysis between 2 and 20%), for example for subjects believed to be at risk of developing cows' milk allergy. If hydrolysed proteins are required, the hydrolysis process may be carried out as desired and as is known in the art. For example, a whey protein hydrolysate may be prepared by enzymatically hydrolysing the whey fraction in two steps as described in EP 322589. For an extensively hydrolysed protein, the whey proteins may be subjected to triple hydrolysis using Alcalase 2.4L (EC 940459), then Neutrase 0.5L (obtainable from Novo Nordisk Ferment AG) and then pancreatin at 55°C. If the whey fraction used as the starting material is substantially lactose free, it is found that the protein suffers much less lysine blockage during the hydrolysis process. This enables the extent of lysine blockage to be reduced from about 15% by weight of total lysine to less than about 10% by weight of lysine; for example about 7% by weight of lysine which greatly improves the nutritional quality of the protein source.

Any suitable dietary protein may be used for exampleanimal proteins (such as milk proteins, meat proteins and egg proteins); vegetable proteins (such as soy protein, wheat protein, rice protein, and pea protein); mixtures of free amino acids; or combinations thereof. Non-limiting examples of proteins include casein, alpha-lactalbumin, whey, soy protein, rice protein, corn protein, oat protein, barley protein, wheat protein, rye protein, pea protein, egg protein, sunflower seed protein, potato protein, fish protein, meat protein, lactoferrin, serum albumin, immunoglobins, and combinations thereof. Milk proteins such as casein and whey, and soy proteins are particularly preferred.

The compositions of the present invention may comprise one or more amino acids. Non-limiting examples of amino acids include leucine, threonine, tyrosine, Isoleucine, arginine, alanine, histidine, isoleucine, proline, valine, cysteine, glutamine, glutamic acid, glycine, serine, arginine, lysine, methionine, phenylalanine, tryptophane, asparagine, aspartic acid, and combinations thereof.

The compositions of the present invention may contain a carbohydrate source. Any carbohydrate source may be used, such as lactose, saccharose, maltodextrins, fructose, glucose, honey, sucrose, corn syrup solids, starch, and combinations thereof.

The compositions of the present invention may contain a lipid source. The lipid source may be any lipid. The lipid source preferably provides 5% to 40% of the energy of the composition, for example 20% to 30% of the energy. A suitable fat profile may be obtained using a blend of canola oil, corn oil and high-oleic acid sunflower oil. Preferred fat sources include milk fat and vegetable oils. The essential fatty acids linoleic and $\alpha$-linolenic acid may also be added. In one embodiment, small amounts of oils containing high quantities of preformed arachidonic acid (AA) and docosahexaenoic acid (DHA) such as fish oils or microbial oils may be added. The lipid source preferably has a ratio of n-6 to n-3 fatty acids of about 5:1 to about 15:1; for example about 8:1 to about 10:1. Non-limiting examples of lipids include: palm oil, palm olein, high oleic sunflower oil, high oleic safflower oil, canola oil, fish oil, coconut oil, bovine milk fat, and combinations thereof.

In addition to ARA and/or DHA, the compositions of the present invention may further comprise one or more

additional essential fatty acids. Non-limiting examples of essential fatty acids include: linoleic acid (LA), α-linolenic acid (ALA) and polyunsaturated fatty acids (PUFAs). The compositions of the invention may further contain gangliosides monosialoganglioside-3 (GM3) and disialogangliosides 3 (GD3), other phospholipids such as phosphatidylcholine, phosphatidylethanolamine, phosphatidylinositol, phosphatidylserine, and combinations thereof.

The compositions may also contain at least one prebiotic, preferably in an amount of about 0.3 to about 10%. A prebiotic is a non-digestible food ingredient that beneficially affects the host by selectively stimulating the growth and/or activity of one or a limited number of bacteria in the colon, and thus improves host health. Such ingredients are non-digestible in the sense that they are not broken down and absorbed in the stomach or small intestine and thus pass intact to the colon where they are selectively fermented by the beneficial bacteria. Examples of prebiotics include certain oligosaccharides. Non-limiting examples of prebiotics include: oligosaccharides optionally containing fructose, galactose, mannose; dietary fibres, in particular soluble fibres, soy fibres; inulin; and combinations thereof. Preferred prebiotics are fructo-oligosaccharides (FOS), galacto-oligosaccharides (GOS), isomalto-oligosaccharides (IMO), xylo-oligosaccharides (XOS), arabino-xylo oligosaccharides (AXOS), mannan-oligosaccharides (MOS), oligosaccharides of soy, glycosylsucrose (GS), lactosucrose (LS), lactulose (LA), palatinose-oligosaccharides (PAO), maltooligosaccharides, gums and/or hydrolysates thereof, pectins and/or hydrolysates thereof, and combinations of the foregoing.

Further examples of oligosaccharides are described in Wrodnigg, T. M.; Stutz, A.E. (1999) Angew. Chem. Int. Ed. 38:827-828 and in WO 2012/069416 which is incorporated herein by reference.

The compositions may also comprise at least one probiotic bacterial strain. A probiotic is a microbial cell preparation or components of microbial cells with a beneficial effect on the health or well-being of the host. Non-limiting examples of probiotics include: *Bifidobacterium, Lactobacillus, Lactococcus, Enterococcus, Streptococcus, Kluyveromyces, Saccharoymces, Candida,* in particular selected from the group consisting of *Bifidobacterium longum, Bifidobacterium lactis, Bifidobacterium animalis, Bifidobacterium breve, Bifidobacterium infantis, Bifidobacterium adolescentis, Lactobacillus acidophilus, Lactobacillus casei, Lactobacillus paracasei, Lactobacillus salivarius, Lactobacillus lactis, Lactobacillus rhamnosus, Lactobacillus johnsonii, Lactobacillus plantarum, Lactobacillus salivarius, Lactococcus lactis, Enterococcus faecium, Saccharomyces cerevisiae, Saccharomyces boulardii* or mixtures thereof, preferably selected from the group consisting of *Bifidobacterium longum* NCC3001 (ATCC BAA-999), *Bifidobacterium longum* NCC2705 (CNCM 1-2618), *Bifidobacterium longum* NCC490 (CNCM I-2170), *Bifidobacterium lactis* NCC2818 (CNCM 1-3446), *Bifidobacterium breve* strain A, *Lactobacillus paracasei* NCC2461 (CNCM 1-2116), *Lactobacillus johnsonii* NCC533 (CNCM 1-1225), *Lactobacillus rhamnosus GG* (ATCC53103), *Lactobacillus* rhamnosus NCC4007 (CGMCC 1.3724), Enterococcus faecium SF 68 (NCC2768; NCIMB10415), and combinations thereof. The amount of probiotic, if present, preferably varies as a function of the age of the person or animal. For example the probiotic may be present in amounts of: about 5 million to about 2500 million, about 10 million to about 2500 million, about 30 million to about 2500 million, about 50 million to about 2500 million, about 50 million to about 1000 million, about 75 million to about 2500 million, about 75 million to about 1000 million, about 100 million to about 2500 million, about 100 million to about 1000 million, about 250 million to about 2500 million, about 250 million to about 1000 million, about 500 million to about 2500 million, about 500 million to about 1000 million, about 750 million to about 2500 million or about 750 million to about 1000 million, about 1 billion to about 2.5 billion, about 1.5 to about 2.5 billion bacteria per dosage form.

The compositions of the invention may further comprise dietary fibre. Dietary fibre passes through the small intestine undigested by enzymes and functions as a natural bulking agent and laxative. Dietary fibre may be soluble or insoluble and in general a blend of the two types is preferred. Suitable sources of dietary fibre include soy, pea, oat, pectin, guar gum, gum Arabic, fructooligosaccharides, galacto-oligosaccharides, sialyl-lactose and oligosaccharides derived from animal milks. A preferred fibre blend is a mixture of inulin with shorter chain fructooligosaccharides. Preferably, if fibre is present, the fibre content is between 10 and 40 g/1 of the formula as consumed.

The composition may also contain minerals and micronutrients such as trace elements and vitamins in accordance with the recommendations of Government bodies such as the USRDA. For example, the compositions may also contain one or more vitamins and minerals understood to be essential in the daily diet and in nutritionally significant amounts. Minimum requirements have been established for certain vitamins and minerals. Non-limiting examples of vitamins and minerals include: vitamin A, vitamin B1, vitamin B2, vitamin B6, vitamin K, vitamin C, vitamin D, inositol, niacin, biotin, pantothenic acid, choline, calcium, phosphorous, iodine, magnesium, copper, manganese, chloride, potassium, sodium, selenium, chromium, molybdenum, taurine, L-carnitine, and combinations thereof. Minerals are usually added in salt form.

If the composition of the invention comprises B12 and/or folic acid, it is particularly beneficial if it further comprises B6. The presence and amounts of specific minerals and other vitamins will vary depending on the numerous factors, such as age weight and condition of the person or animal the composition is administered to.

For example, the composition may contain per daily dose one or more of the following micronutrients in the ranges given:- 300 to 500 mg calcium, 50 to 100 mg magnesium, 150 to 250 mg phosphorus, 5 to 20 mg iron, 1 to 7 mg zinc, 0.1 to 0.3 mg copper, 50 to 200 [mu]g iodine, 5 to 15 [mu]g selenium, 1000 to 3000 [mu]g beta carotene, 10 to 80 mg Vitamin C, 1 to 2 mg Vitamin BI, 0.5 to 1.5 mg Vitamin B6, 0.5 to 2 mg Vitamin B2, 5 to 18 mg niacin, 0.5 to

2.0 [mu]g Vitamin B 12, 100 to 800 [mu]g folic acid, 30 to 70 [mu]g biotin, 1 to 5 [mu]g Vitamin D, 3 to 10 IU Vitamin E.

One or more food grade emulsifiers may be incorporated into the formula if desired; for example diacetyl tartaric acid esters of mono- and di- glycerides, lecithin and mono- and di-glycerides. Similarly suitable salts and stabilisers may be included.

The formula is preferably enterally administrable; for example in the form of a powder or a liquid concentrate for re-constitution with milk or water, a solid product or a ready-to-drink beverage.

The compositions may optionally contain other substances which may have a beneficial effect such as nucleotides, nucleosides, and the like. Non-limiting examples of nucleotides include: cytidine monophosphate (CMP), uridine monophosphate (UMP), adenosine monophosphate (AMP), guanosine monophosphate (GMP), and combinations thereof.

Other suitable and desirable ingredients of compositions that may be employed in the composition of the invention may be described in guidelines issued by the Codex Alimentarius with respect to the type of nutritional composition in question.

The compositions, for use in the invention may be prepared in any suitable manner. For example, the composition may be prepared by blending together the protein source, the carbohydrate source, and the fat source in appropriate proportions. If used, the emulsifiers may be included in the blend. The vitamins and minerals may be added at this point but are usually added later to avoid thermal degradation. Any lipophilic vitamins, emulsifiers and the like may be dissolved into the fat source prior to blending. Water, preferably water which has been subjected to reverse osmosis, may then be mixed in to form a liquid mixture. The liquid mixture may then be thermally treated to reduce bacterial loads. For example, the liquid mixture may be rapidly heated to a temperature in the range of about 80°C to about 110°C for about 5 seconds to about 5 minutes. This may be carried out by steam injection or by heat exchanger; for example a plate heat exchanger. The liquid mixture may then be cooled to about 60°C to about 85°C; for example by flash cooling. The liquid mixture may then be homogenised; for example in two stages at about 7 MPa to about 40 MPa in the first stage and about 2 MPa to about 14 MPa in the second stage. The homogenised mixture may then be further cooled to add any heat sensitive components; such as vitamins and minerals. The pH and solids content of the homogenised mixture is conveniently standardised at this point. The homogenised mixture is transferred to a suitable drying apparatus such as a spray drier or freeze drier and converted to powder. The powder should have a moisture content of less than about 5% by weight. If it is desired to add probiotic(s), they may be cultured according to any suitable method and prepared for addition to the formula freeze-drying or spray-drying for example. Alternatively, bacterial preparations can be bought from specialist suppliers such as Christian Hansen and Morinaga already prepared in a suitable form for addition to food products. Such bacterial preparations may be added to the formula by dry mixing.

The fatty acid may be added at any stage during this procedure, but is preferably added after the heating step.

In one preferred embodiment, the composition comprises triglycerides with high sn-2 palmitate, preferably triglycerides having more than 33% of the palmitic acids in sn-2 position.

In some preferred embodiments, palmitic acid comprises from about 15 to about 25%, such as from about 15 to about 20%, of the total fatty acids content of the formula, by weight, and at least from about 30%, for example, from about 35 to about 43% of the total palmitic acid content is in the sn-2 position.

A commercially available composition sold by Lipid Nutrition is BetapolTM B-55, which is a triglyceride mixture derived from vegetable oil in which at least 54% of the palmitic acid is in the sn-2 position of the glycerol molecule. In one preferred embodiment, the fat content of the composition of the invention is about 40-50% BetapolTM B-55 by weight, for example, from about 43% to about 45% by weight. Those skilled in the art will appreciate that the percentage of the high *sn*-2 fat used and the total amount of sn-2 palmitate in the formula may vary, and that a different high sn-2 palmitate oil may be used, without departing from the spirit and scope of the invention.

In another embodiment, a conventional food product such as a yoghurt, or a breakfast cereal may be enriched with a fatty acid, preferably DHA and/or ARA, or a mixture thereof.

In yet a further embodiment, a composition containing a fatty acid, preferably DHA and/or ARA, or a mixture thereof, in an amount sufficient to achieve the desired effect in an individual can be prepared. This composition may be in the form of tablets, capsules, pastilles or a liquid for example. The supplement may further contain protective hydrocolloids (such as gums, proteins, modified starches), binders, film forming agents, encapsulating agents/materials, wall/shell materials, matrix compounds, coatings, emulsifiers, surface active agents, solubilizing agents (oils, fats, waxes, lecithins etc.), sweeteners, texturizers, adsorbents, carriers, chelating agents, fillers, co-compounds, dispersing agents, wetting agents, processing aids (solvents), flowing agents, taste masking agents, weighting agents, jellifying agents, gel forming agents, antioxidants and antimicrobials. The composition may also contain conventional pharmaceutical additives and adjuvants, excipients and diluents, including, but not limited to, water, gelatine of any origin, vegetable gums, ligninsulfonate, talc, sugars, starch, gum arabic, vegetable oils, polyalkylene glycols, flavouring agents, thickeners, preservatives, stabilizers, emulsifying agents, buffers, lubricants, colorants, wetting agents, fillers, and the like.

**Health Benefits**

The composition of the invention has a positive effect on denovo myelination, in particular the denovo myelination trajectory, in the brain of the infants or young children whose mothers are administered such compositions, before or during pregnancy, or during postpartum lactation.

Such positive effects can comprise the promotion and/or support of an optimal denovo myelination, in particular de novo myelination trajectory, which may determine appropriate cognitive, intellectual and learning potential, the development of cognitive skills and abilities and learning in the infant or young children. An optimal de novo myelination trajectory may also prevent development of cognitive impairment or delay.

The health effect can be observed after a few days, weeks, months or years of use of the composition comprising choline.

The effect of the invention can be preventive (for example, avoiding a suboptimal de novo myelination, in particular de novo myelination trajectory in the brain, brain structure, brain connectivity, cognitive, intellectual and/or learning potential or cognitive function) or curative (for example, restoring an optimal de novo myelination, in particular de novo myelination trajectory in the brain, brain structure, brain connectivity, cognitive, intellectual and/or learning potential or cognitive function).

The health effect related to the infant can be measured by various methods as illustrated in the example below.

As evident from the above disclosure, the composition of the invention may be used to promote, support or optimize de novo myelination, in particular the de novo myelination trajectory, and/or brain structure, in particular the amount and/or spatial distribution of myelinated matter in the brain, and/or brain connectivity and/or intellectual potential, and/or cognitive potential and/or learning potential and/or cognitive functioning in a subject whose mother receives such a composition, before or during pregnancy, or during postpartum lactation.

In another aspect of the present invention there is provided a method of promoting, supporting or optimizing de novo myelination, in particular the de novo myelination trajectory, and/or brain structure , in particular the amount and/or spatial distribution of myelinated matter in the brain, and/or brain connectivity and/or intellectual potential, and/or cognitive potential and/or learning potential and/or cognitive functioing in a subject, in particular a formula fed subject, said method comprising feeding to the mother of said subject a composition comprising choline in accordance with the present invention, before, during or after pregnancy during lactation.

The effects of the composition of the invention described herein may have long term health benefits. Dementia e.g. Alzheimer's disease, causes a decrease in a subject's ability to think and remember, as well as emotional and language problems. The risk of a subject suffering from dementia, in particular Alzheimer's disease is often associated with a person's intellectual ability or intelligence. Accordingly, by optimizing a subject's intellectual, cognitive and or learning potential the risk of a subject developing dementia in particular Alzheimer's disease may be reduced.

A variety of other psychiatric and/or neurological disorders e.g. autism and schizophrenia, are also linked to brain structure, and in particular to the amount and/or spatial distribution of white matter throughout the brain. By promoting, supporting or optimizing de novo myelination, in particular the de novo myelination trajectory, and/or brain structure in a subject, it may be that psychiatric and/or neurological disorders e.g. autism, are prevented or that the risk of them developing is reduced, or that the severity of said condition(s) is reduced.

As will be evident to the skilled person, the same benefits as disclosed herein may be obtainable by the mother taking choline directly as opposed to in the form of a composition. Accordingly, choline may be employed directly in place of the composition of the present invention in any method or use set out herein.

As would be further evident to the skilled person, it may be particularly beneficial if said choline is administered separately, sequentially and/or simultaneously to one or more of the following ingredients: a vitamin, a mineral, a fatty acid derivative and a phospholipid or metabolite thereof, or a metabolic precursor thereof.

As used herein "simultaneously" means administering the additional ingredient concurrently with the choline. Preferably, the additional ingredient is administered simultaneously as part of the composition according to the invention.

As used herein "sequentially" means administering the additional ingredient within a certain time period before or after the choline. The time delay will vary depending on the nature of the additional ingredient and/or the choline.

As used herein "separately" means that there is a substantial gap between administering the additional agent and the choline, for example, such that the choline may no longer be present in the bloodstream of the female subject in a therapeutically effective amount when the additional ingredient is administered.

In one preferred embodiment, the additional ingredient is administered at least 2 hours, or at least 4 hours, or at least 8 hours, or at least 12 hours, or at least 24 hours, or at least 48 hours, before the choline.

In another preferred embodiment, the additional ingredient is administered at least 2 hours, or at least 4 hours, or at least 8 hours, or at least 12 hours, or at least 24 hours, or at least 48 hours, after the choline.

All particulars of the invention apply equally to the composition comprising choline, and to the direct use of choline and/or any other ingredient.

In one embodiment, the invention provides a means of promoting, supporting or optimizing de novo myelination

in infants or young children showing a sub-optimal de novo myelination trajectory in the brain which may result in cognitive deficits, impaired cognitive abilities and/or sub-optimal cognitive development. Such infants can be preterm or low birth weight infants or infants born small for gestational age.

The invention is also suitable for promoting, supporting or optimizing myelination in infants or young children that are born at term. All infants can benefit from the invention as all infants are or can be susceptible to develop a sub-optimal myelination trajectory in the brain.

In such infants or young children, acquiring a myelination trajectory in the brain that is close to that of breast fed infant (preferably exclusively breast fed infants for the first months of life) of a well-nourished or nutritionally replete mother, is of particular interest. Indeed it provides them with a health status in terms of cognitive abilities which are matching those observed in infants which are breast fed by a well-nourished or nutritionally replete mother.

In one embodiment, the infants and young children are 0-3 months, 0-6 months, or 0-12 months or 0-36 months of age or 0-60 months of age.

**Administration**

The composition of the invention can be administered to the female subject pre-pregnancy, during pregnancy, or during lactation, or a combination thereof.

In one embodiment, the compositions of the invention are administered prenatally to a female subject and thereby indirectly transmitted to the developing embryo or fetus e.g. via the placenta or amniotic fluid. In other words, the exposure of the offspring to the compositions of the invention is *in utero* when the composition is administered to the mother during pregnancy. Prenatal administration of the compositions may prevent the onset of, or reduce the risk, of suboptimal myelination in the offspring, and the effects associated therewith.

In another alternative embodiment, the compositions of the invention are administered postnatally to a lactating female, and are thereby indirectly transmitted to the neonate or infant via the ingestion of maternal milk, i.e. the exposure of the offspring to the compound is solely via the mother's milk.

In one highly preferred embodiment, the composition is for administration to the female subject pre-pregnancy, for example, in female subjects desiring to get pregnant. Pre-pregnancy administration of compositions according to the invention may impact the intrauterine environment.

Preferably, in any aspect or any embodiment of the present invention, pre-pregnancy supplementation or administration refers to administration from about 1-24 months, 1-18 months, 1-12 months, 1-6 months, or 1-3 months prior to pregnancy.

In one preferred embodiment, the composition is administered for at least 1 week, or for at least 2 weeks, or for at least 3 weeks, or for at least 1 month, or for at least 2 months, or for at least 3 months, or for at least 4 months, or for at least 5 months, or for at least 6 months, or for at least 12 months, or at least 18 months or 24 months prior to conception, for example, in women who are intending to, or trying to, become pregnant.

In another preferred embodiment, the composition is for administration to the female subject during pregnancy, e.g. prenatally. As used herein, "prenatally" refers to the period before birth, during or relating to pregnancy.

As used herein, unless otherwise indicated, a reference to administration of a composition according to the invention during pregnancy (i.e. prenatal administration), particularly refers to administration during any part of, or the whole of, the gestation period. In a preferred embodiment of the invention, administration of the composition is initiated as soon as possible following conception until at least the end of the period of embryogenesis. For example, in humans, the embryogenesis period encompasses the first 8 weeks of development (10 weeks of gestation).

In one preferred embodiment, the composition is administered throughout the whole gestation period.

In another preferred embodiment, the compositions of the invention are administered during a substantial part of the gestation period. In any embodiment of the present invention, administration can be within: the first week, the first two weeks, the first month, the first trimester, the second trimester or the third trimester of pregnancy. Preferably, the administration may be continued until at least the birth of the offspring. In one preferred embodiment, the composition is administered as soon as possible from conception until birth, i.e. during the full gestation period. In humans, the administration is preferably for a period of from: about 1 week to birth, about 2 weeks to birth, about 4 weeks to birth, about 8 weeks to birth, about 12 weeks to birth, about 18 weeks to birth, , about 24 weeks to birth.

In any embodiment of the present invention, the composition of the invention may additionally be administered pre-pregnancy to the female subject as well as during pregnancy and/or during lactation.

In one highly preferred embodiment, the composition is for administration to the female subject after birth, for example, during postpartum lactation. During this period, the infant can receive the beneficial effects of the composition via breast milk from the mother. After birth, the composition can be administered for part or all of the lactation period.

Hence, in one embodiment, the present invention relates to a composition for a lactating postpartum female subject, the composition comprising a fatty acid derivative, preferably DHA and/or ARA, or mixture thereof, for promoting, supporting or optimizing one or more of the following:

(i) de novo myelination;
(ii) brain structure;
(iii) brain connectivity;
(iv) intellectual potential;
(v) cognitive potential;
(vi) learning potential; and
(vii) cognitive functioning

in an infant being breast-fed by said female subject.

Reference to a lactating female subject refers to a female subject who is exclusively or partially breast feeding her offspring.

As used herein, unless otherwise indicated, a reference to administration of a composition according to the invention during lactation includes administration of the compound postnatally at any time during which the offspring is exclusively or partially ingesting the maternal milk (the subject's breast milk). For example, administration during lactation may be for the period starting from onset of lactation until the end of the weaning process, i.e. when the offspring has ceased to ingest the maternal milk. During this period, the offspring may be exclusively or partially ingesting the maternal milk.

Preferably, the administration of the composition during lactation includes administration for a period of 1-24 months, 2-20 months, 3-18 months, 6-12 months, 4-12 months or 4-8 months following the onset of lactation during which the offspring is exclusively or partially ingesting the maternal milk. It is foreseen that the composition of the invention may be particularly beneficial when administered to infants just after birth (0-4 weeks, 0-8, 0-12, 0-24 weeks) because it is at that time that myelination process has started and significantly develops.

In one particularly preferred embodiment, the composition of the invention is administered during lactation for two weeks following the onset of lactation when the offspring is exclusively or partially ingesting the maternal milk.

In one preferred embodiment, administration of the compositions to the female subject is initiated postpartum, i.e. not during pregnancy, but instead from the onset of lactation.

The composition can be advantageously used for promoting, supporting or optimizing one or more of the above aspects in the infant wherein the composition is administered to the mother of the infant during lactation postpartum and the advantageous effect in the infant can be observed later in life, e.g. during childhood and/or adolescence.

In another embodiment, the composition of the invention is administered both prenatally, i.e. at any period from conception to birth, as well as postnatally during lactation, i.e. at any period from birth until the end of the weaning process, i.e. when the offspring has ceased to ingest the maternal milk. Further, in any embodiment of the present invention the composition may be administered both prenatally for any period as defined above in relation to prenatal administration, as well as postnatally for any period as defined above in relation to the lactation period, and any combination of these periods as described above.

In another embodiment, the compositions of the invention are administered as a pre-pregnancy, pregnancy or lactation dietary supplement in a female subject to promote de novo myelination. Further, when employed as a pre-pregnancy supplement alone, or in combination as a pregnancy and/or lactation supplement, the compositions of the invention may provide health benefits to future offspring.

In one preferred embodiment, the composition of the invention is administered to the female subject for a period of from 2 to 52 weeks. In one preferred embodiment, it is administered to the female subject for a period of from 2 to 24 weeks, or 2 to 12 weeks.

In a more preferred embodiment, the composition of the invention is administered to the female subject for 2 to 52 weeks and started shortly after the infant or young children are born or breastfeeding is interrupted. In one embodiment, composition of the invention is fed to the mother for 2 to 24 weeks, or 2 to 12 weeks, and started shortly after the infants or young children are born or breastfeeding is interrupted.

In one embodiment, the maternal supplement is used in conjunction with an infant formula and/or starter infant formula/and or growing up milk which is administered to the infant after birth. The composition can also be used in conjunction with baby food and/or a fortifier. Preferably, an infant formula and/or starter infant formula/and or growing up milk and/or baby food and/or fortifier also comprises a fatty acid, preferably DHA and/or ARA, or a mixture thereof, which further promotes, supports or optimizes one or more of the following: (i) de novo myelination; (ii) brain structure; (iii) brain connectivity; (iv)intellectual potential; (v) cognitive potential; and (vi) learning potential; (vii) cognitive functioning in the infant.

It should be appreciated that all features of the present invention disclosed herein can be freely combined and that variations and modifications may be made without departing from the scope of the invention as defined in the claims. Furthermore, where known equivalents exist to specific features, such equivalents are incorporated as if specifically referred to in this specification.

There now follows a series of non-limiting examples that serve to illustrate the invention.

## Experimental section:

### Methods, definitions and materials

MRI (Magnetic Resonance Imaging): MRI brain scans of infants and children between 0 and 5 years were acquired using a white matter imaging technique. This technique provides a quantitative measure, the Myelin Water Fraction (MWF), which is a surrogate marker of myelin content in the brain. When mapped as a function of time across early childhood, myelination trajectories can be generated.

Infant formula composition: six infant formulas fed to infants participating in a study were analyzed for their composition/level of myelin-relevant nutrients.

Nutritional compositions were tested in standard, commercially-available infant formulas of different brands/suppliers and showing variable levels on the nutrients therein contained. Cognitive abilities: Age-standardized (T)- scores of gross motor, visual reception and language (expressive and receptive) derived from the Mullen Scales of Early Learning, a standardized and validated measurement tool of early cognitive development for infants and children 6 years of age or younger.

Maternal nutritional intake: Maternal nutritional intake during pregnancy was assessed at 34 weeks of gestation using the Automated Self-Administered 24-hour (ASA24) dietary assessment tool, a web-based tool that enables multiple, automatically coded, self-administered 24-hour recalls.

## Nutrient Analysis

Nutrients in each of the 6 infant formula compositions are shown in table 1.

Table 1

| Nutrient | Unit | Concentration range across the 6 infant formulas included in the analysis |
|---|---|---|
| Alpha Lactalbumin | g/100g protein | ND-1.01 |
| Fat | g/100g | 26.3 - 29.5 |
| AA | mg/100g | 94.2 -180 |
| DHA | mg/100g | 42.3 -89.8 |
| Iron | mg/100g | 8.42 -11.7 |
| Calcium | mg/100g | 397 - 566 |
| Phosphorus | mg/100g | 234 -358 |
| Sodium | mg/100g | 135 -189 |
| Potassium | mg/100g | 593 - 834 |
| Copper | mcg/100g | 471 - 834 |
| Zinc | mg/100g | 4.23 -7.25 |
| Magnesium | mg/100g | 38.4 - 53.9 |
| Magnese | mcg/100g | 60.6 - 140.3 |
| Vitamin B12 | mcg/100g | 4.93 - 8.34 |
| Folic acid | mcg/100g | 98.8 -306 |
| Choline | mg/100g | 35.7 - 170 |
| Beta lactoglobulim | g/100g | ND - 4.21 |
| Phospahtidylcholine | mg/kg | 397 - 1287 |
| Phosphatidylinositole | mg/kg | 266 - 788 |
| Phosphatidylserine | mg/kg | <LQ (144)- 977 |
| Phosphatidylethanolamine | mg/kg | <LQ(174) |

(continued)

| Nutrient | Unit | Concentration range across the 6 infant formulas included in the analysis |
|---|---|---|
| Sphingomyelin | mg/kg | <LQ(100) - 480 |

## Clinical study 1

### Infant Participants

Infants included in this study were drawn from a larger longitudinal study of normal brain and behavioral development: the Brown University Assessment of Myelination and Behavior Across Maturation (BAMBAM). To focus on neurotypical development, children with known potential risk factors for learning, neurologic, or psychiatric disorders were specifically excluded during recruitment and enrollment. Thus, children with *in utero* alcohol or illicit substance exposure, premature (<37 weeks gestation) or multiple birth, fetal ultrasound abnormalities, complicated pregnancy (e.g., preeclampsia), APGAR scores < 8, NICU admission, neurological disorder (e.g., head injury, epilepsy), psychiatric or developmental disorders in the infant, parents or siblings (including maternal depression requiring medication) were excluded. Ongoing screenings, such as the MCAT for autism, or CBCL for behavioral problems, were further used to remove enrolled children with clinically concerning behaviors or overt medical conditions (such as autism spectrum disorders).

A combination of retrospective and prospective data were acquired from parents via detailed medical histories and parental interview on the type of infant formula used, percentage of breastfeeding to formula feeding, and length of exclusive breastfeeding. This information was updated at each study visit, which occurred approximately every 6 months for children under 2 years, and yearly for older children. Using this information, children were categorized into one of 2 groups: #1. Exclusively formula-fed; and #2. Exclusively breastfed for at least 90 days (3 months). Children who were fed a combination of breastmilk and formula within 3 months were excluded from our analysis. Infants within the exclusively formula-fed group were further subdivided based on parental reports of the main infant formula used throughout the first 3 months. Main formula was defined as that given 90% of the time or more (in the case were parents used an alternate brand during vacation, for example).

Using these criteria, 94 exclusively formula-fed infants and young children were selected into group #1. These included 13 children who received formula #2; 28 who received formula #5; 8 who received formula #3 ; 39 who received formula #4; 5 who received formula #1 and 1 who received formula #6. A sample of 52 exclusively breast-fed infants were also selected and matched to the over formula-fed group with regards to mean age, gestation duration, birth weight, male:female ratio, ethnicity ratio, maternal education, family size, and number of languages spoken in the home (in addition to English). Groupings for each formula are provided in **Table 1a.**

**Table 1a.** Data breakdown for longitudinal and nutritional analysis

| | Formula 1 | Formula 2 | Formula 5 | Formula 3 | Formula 4 | Formula 6 | Breast-fed |
|---|---|---|---|---|---|---|---|
| $N_{children}$ | 5 | 13 | 28 | 8 | 39 | 1 | 52 |
| $N_{Measurements}$ | 11 | 27 | 56 | 14 | 64 | 3 | 106 |

### Imaging Methods and Analysis

Each infant was scanned using the mcDESPOT (multicomponent Driven Equilibrium Single Pulse Observation of $T_1$ and $T_2$) white matter imaging technique Deoni et al. (Magn. Reson. Med. 2008, 60:1372-1387), which provides a quantitative measure of the myelin water fraction (MWF)- a measure of myelin content - at each point throughout the brain. All infants were scanned during natural *(i.e. non-sedated)* sleep using acoustically-muffled mcDESPOT imaging protocols. Total imaging times ranged from 19 minutes for the youngest toddlers to 24 minutes for the older 4 year-old children.

All data were acquired on a Siemens 3T Tim Trio scanner equipped with a 12 channel head RF array. To minimize intra-scan motion, children were swaddled with a pediatric MedVac vacuum immobilization bag (CFI Medical Solutions, USA) and foam cushions. Scanner noise was reduced by lessening the peak gradient amplitudes and slew-rates, and using a noise-insulating scanner bore insert (Quiet Barrier HD Composite, UltraBarrier, USA). MiniMuff pediatric ear covers and electrodynamic headphones (MR Confon, Germany) were also used. Children were continuously monitored with a pediatric pulse-oximetry system and infrared camera. All children remained asleep for the duration of the

MRI scan and no motion-artifacts were present in the analyzed data.

Following image alignment, non-brain signal removal, and correction for main and transmit magnetic field ($B_0$ and $B_1$) inhomogeneities, a three-pool signal model (comprising the myelinassociated water; intra-extra axonal water; and a non-exchanging free-water pool) was fit to the mcDESPOT data to derive voxel-wise MWF maps.

Each child's map was then non-linearly aligned to a study specific template. White matter masks, corresponding to 5 bilateral regions (frontal, temporal, occipital, parietal, and cerebellar WM) as well as the body, genu, and splenium of the corpus callosum were created from common databases, registered to the common template, and superimposed onto each child's MWF map. Mean values for each region were then determined for each child and used for subsequent developmental analysis and trajectory modeling.

*Developmental Differences:*

To examine developmental differences between the breastmilk and formula-fed infants, as well as between the different formula-fed infants, a non-linear mixed effects modeling approach was used. Modified Gompertz growth models were fit to groups #1 and #2, and each formula subgroup independently. Each of the four Gompertz model parameters were then compared between the breast and formula-fed groups using an unpaired t-test, and between the 4 formula sub-groups using an analysis of variance followed by post-hoc Tuckey tests to determine which of the formula groups differed.

**Cognitive Assessments and Analysis**

Alongside MR imaging, general cognitive ability and skills were evaluated in each child within 7 days of scanning using the Mullen Scales of Early Learning, MSEL (Mullen EM, 1995). The MSEL provide a broad assessment of behavioral development in the domains of fine and gross motor control, receptive and expressive language, and visual reception. Age-normalized T-scores from these domains can be combined into three composite scores: the early learning composite (ELC, comprising fine motor, visual reception, expressive and receptive language); the non-verbal development quotient (NVDQ, comprising fine motor and visual reception scores); and the verbal development quotient (VDQ, comprising the expressive and receptive language scores).

As with the MWF MRI data, potential group mean differences in ELC, VDQ and NVDQ between the breastmilk and formula-fed infants, as well as between the different formula sub-groups were examined. In addition to mean comparisons, longitudinal changes in these three composite values were investigated using mixed effects modeling assuming a linear trend.

**Example 1**

**Nutritional Drivers identification from cross-sectional analyses**

From the cohort described above, children up to 5 years of age that were fed different infant formulas during infancy were included in a large correlation analysis to examine the relationship between formula nutrient composition and brain myelination. The 5 most frequently used formulas in that cohort were analyzed for their nutritional composition. A single general linear model (GLM) was constructed that modeled all quantified nutrients and child age.

Spearman rank correlations correlations were then calculated between the nutrient content and myelin content value (adjusted for child age) at each image voxel, or point within the brain. Significance was defined as $p < 0.05$ corrected for type 1 error using a cluster based correction approach. An association or trend was defined as $p<0.15$. In initial analysis, inclusion of all 22 nutrients shown in Table 1 resulted in an underpowered model. To reduce the number of nutritional components in the model, we examined the inter-nutritional correlation. Using a conservative threshold of 0.9, we excluded nutritional components that were highly correlated with each other across the various formulas. This yielded a final model that included iron, sphingomyelin, folic acid, choline, DHA, zinc, and phosphatidylcholine.

$P<0.05$: iron, sphingomyelin, folic acid, choline, DHA.

$P<0.15$: zinc, and phosphatidylcholine.

Nutritional components that were found to be highly correlated with each other were:

Folic acid and vitamin B12.
DHA and AA.
Zinc, calcium, magnesium, copper, and phosphorus.
Phosphatidylcholine, phosphatidylserine and phosphatidylinositol.

For DHA, an association with myelination (myelin water fraction) was observed over time in the brain, in particular

in the cerebellum, primary & secondary motor cortices, internal capsule, visual Cortex, frontal Cortex. Results are reported in Fig.1b.

For AA, an association with myelination (myelin water fraction) was observed over time in the brain, in particular in the cerebellum, internal capsule, parietal lobe, motor and sensory cortices, visual Cortex, frontal Cortices. Results are reported in Fig.1c.

For folic acid, an association with myelination (myelin water fraction) was observed over time in the brain, in particular in the cerebellum, motor cortex, visual cortex. Results are reported in Fig.1d.

For vitamin B12, an association with myelination (myelin water fraction) was observed over time in the brain, in particular in the cerebellum, visual Cortex, Motor & Somatosensory Cortices.

Results are reported in Fig.1e

For iron, an association with myelination (myelin water fraction) was observed over time in the brain, in particular in the cerebellum, visual cortex, internal capsule, motor & somatosensory cortices, corpus callosum, frontal cortex, temporal white matter. Results are reported in Fig.1f.

For zinc, an association with myelination (myelin water fraction) was observed over time in the brain, in particular in the cerebellum, visual cortex, internal capsule, motor & somatosensory cortices, corpus callosum, frontal cortex, temporal white matter. Results are reported in Fig.1g.

For calcium, an association with myelination (myelin water fraction) was observed over time in the brain, in particular in the cerebellum, visual cortex, Motor & Somatosensory Cortices, Corpus Callosum, Frontal Cortex, Temporal White Matter. Results are reported in Fig.1h.

For phosphorus, an association with myelination (myelin water fraction) was observed over time in the brain, in particular in the cerebellum, visual cortex, motor & somatosensory cortices, prefrontal cortex. Results are reported in Fig.1i.

For magnesium, an association with myelination (myelin water fraction) was observed over time in the brain, in particular in the cerebellum, visual cortex, internal capsule, corpus callosum, frontal cortex, motor cortex. Results are reported in Fig.lJ.

For sphingomyelin, an association with myelination (myelin water fraction) was observed over time in the brain, in particular in the cerebellum, visual cortex, internal capsule, frontal lobe, parietal lobe, temporal lobe. Results are reported in Fig.1k.

For phosphatidylinositol, an association with myelination (myelin water fraction) was observed over time in the brain, in particular in the cerebellum, visual cortex, motor cortex, frontal cortex. Results are reported in Fig.1L.

For phosphatidylcholine, an association with myelination (myelin water fraction) was observed over time in the brain, in particular in the cerebellum, visual cortex, internal capsule, frontal lobe, parietal lobe, temporal lobe. Results are reported in Fig.1M.

For choline, an association with myelination (myelin water fraction) was observed over time in the brain, in particular in the cerebellum, visual cortex, thalamus, parietal cortex, and frontal lobe. Results are reported in Fig.1n.

**Example 2**

**a) Whole Brain Myelination trajectory from longitudinal study**

From the available data, trajectories of longitudinal myelin development (de novo myelination) were calculated using repeated MWF data from children for whose infant formulas contained a differing amount of DHA (composition of such formulas is reported below in Table 2). Trajectories were calculated using a longitudinal nonlinear mixed effects approach. Modified Gompertz growth models were fitted to the data of children for each formula group. Results are reported in Fig.1.

**Table 2:**

| | (low DHA content) | (high DHA content) |
|---|---|---|
| Docosahexaenoic acid (DHA). | 43.2 mg/100g | 89.8 mg/100g |

**b) Mean Regional brain Myelination trajectory from longitudinal study**

From the available data, a mean regional trajectory of longitudinal myelin development (de novo myelination) was calculated using repeated MWF data from children for whose infant formulas contained a differing amount of DHA and AA (composition of such formulas is reported below in Table 2a). Trajectories were calculated using a longitudinal nonlinear mixed effects approach and modified Gompertz growth models were fitted to the data of children for each

formula group. Results are reported in Fig.1a.

**Table 2a:**

| | (low DHA/AA content) | (high DHA/AA content) |
|---|---|---|
| Docosahexaenoic acid (DHA). | 67.7mg/100g | 81.5mg/100g |
| Arachidonic acid (AA) | 108mg/100g | 172 mg/100g |

**Clinical Study 2**

**Participants: Mother-Infant Pairs**

The available data set of 21 mother-child pairs were taken from a hybrid cross-sectional/longitudinal cohort at the Children's Hospital Colorado, US.

Pregnant women were drawn from the local community with the following inclusion criteria: At least 18 years of age, English-speaking (primary language), singleton pregnancy, no reports of smoking, alcohol or illicit drug use during pregnancy, no abnormalities on fetal ultrasound, no history of high blood pressure or diabetes before or during pregnancy, no personal history of major psychiatric or learning disorder (including major depressive disorder) or use of psychiatric medication, no reports of major psychiatric or learning disorders in other children, suspicion of metal in body or other MRI contraindications. These criteria are meant to provide a generally healthy cohort devoid of major risk factors for abnormal or altered neurodevelopment. Inclusion criteria for the infants were: English-speaking parents (primary language), singleton pregnancy, born at full-term (after 37 weeks gestation), no exposure to smoking, alcohol or illicit drugs during pregnancy, no abnormalities on fetal ultrasound, no visit to the NICU, APGAR scores >= 8, no history of high blood pressure or diabetes before or during pregnancy in mother, no history of major psychiatric or learning disorder in parents or siblings, no history of neurological trauma to the infant, no other neurological conditions (e.g., epilepsy), suspicion of metal in body or other MRI contraindications. Informed consent was obtained from all parents, consisting of a description of the US, MRI and other lab facilities, the overall study protocol, and specifics regarding each measurement that will be obtained.

**Imaging Methods and Analysis**

Each infant was scanned using the mcDESPOT (multicomponent Driven Equilibrium Single Pulse Observation of $T_1$ and $T_2$) white matter imaging technique Deoni et al. (Magn. Reson. Med. 2008, 60:1372-1387), which provides a quantitative measure of the myelin water fraction (MWF)- a measure of myelin content - at each point throughout the brain. All infants were scanned during natural *(i.e. non-sedated)* sleep using acoustically-muffled mcDESPOT imaging protocols. Wholebrain, ($1.7\times1.7\times1.7$) mm3 data was acquired using optimized protocols that include additional steps for main and transmit magnetic field inhomogeneity correction (Deoni et al., 2008; Deoni, 2011; Deoni et al., 2013). Following acquisition and processing (including motion correction, skullstripping, B0 and B1 map calibration, and voxel-wise parameter estimation), calculated T1, T2 and MWF 'maps' were non-linearly aligned to MNI-space using a multi-step registration process (Deoni et al., 2012; Dean et al., 2014c; 2014d).

Children included in the analyses had one MRI scan between 90 and 180 days of age, with an average of 117days +/- 71.

*Correlational Analyses:*

To examine the association between maternal nutritional intake data at 34 weeks of gestation and myelin content in the infants' brain at around 3 to 6 months, correlational analyses (Pearson) were performed.

**Example 3**

From the cohort described above, maternal nutritional intake data was associated with infant brain myelin content. The following nutrients showed significance at the level of $p<0.001$: DHA, choline, iron, B12, and folic acid.

Using the identified areas of significance of masks and then looking at the correlation between the myelin water fraction in those masks vs. maternal nutrient values (actually, the partial correlation since we included the effect of age), we find the following:

B12: $r^2 = 0.39$

Choline: $r^2 = 0.33$
DHA: $r^2 = 0.44$
Folic Acid: $r^2 = 0.173$
Iron: $r^2 = 0.18$

For DHA, choline, iron, B12, and folic acid associations with maternal intake and myelination (myelin water fraction) were observed for the following brain areas:

B12: Cerebellum, temporal lobes, frontal lobe
Folic Acid: Cerebellum, brainstem
Iron: Cerebellum, brainstem
Choline: Cerebellum, Internal Capsules, Motor Cortex
DHA: Cerebellum, Internal Capsule, Temporal lobe, Frontal cortex, Motor Cortex

Results are reported in Figures.50a to 50e.

**Example 4**

**Vendors and** stock **solutions**

| Compound | Company | Cat# | Cas# | Stock |
|---|---|---|---|---|
| Octanoic Acid | sigma | O3907 | 12407-2 | 50mM |
| Nervonic Acid | Fluka | 87117 | 50637-6 | 10 mM |
| Stearic Acid | Fluka | 85679 | 57-114 | 10 mM |
| Sphingomyelin | sigma | S0756 | 8518710-6 | 10mM |

**Vehicles and doses**

| Compound | Vehicle/dissolved in | Dose 1 | Dose 2 | Dose 3 |
|---|---|---|---|---|
| Octanoic Acid | DMSO | 10μM | 50μM | 250μM |
| Nervonic Acid | DMSO | 10nM | 100nM | 1μm |
| Stearic Acid | DMSO | 100nM | 1μm | 10μM |
| Sphingomyelin | Dissolved in ETOH/diluted in DMSO | 10nM | 100nM | 1μm |

**Media compositions and culture methods**

1) Neurobasal complete media

Neurobasal media (LIFE TECHNOLOGIES CORP, # 21103-049)
50X B27 supplement (LIFE TECHNOLOGIES CORP, #12587-010)
2 mM L-Glutamine(LIFE TECHNOLOGIES CORP, #25030-149)
1X Pen-Strep (LIFE TECHNOLOGIES CORP,#15140-122)

2) Neurobasal media complete with growth factors (GF)

Above recipe with GF mix
1M Tris (MW 121.14, Fisher SCI BP152)

Heparin (sigma H3149)
BSA(Sigma A7030)
DNAse, RNase, Protease free water(Fisher SCI AC327390010)
EGF (GIBCO PHG0311)
bFGF vial (GIBCO PHG0021)

3)Neurobasal media no Choline (Life technologies, formulated custom, no L-Glutamine, no phenol red)

Note: complete and complete with GF made the same as above.
Generation of neural progenitor cell (NPCs) libraries:
dissociation of E14 mouse neocortex
Reagents needed:

DPBS(1X) + 10% Pen/Strep

Neurobasal media/ 10% Pen/Strep/ 10X Hepes

**Procedure:**

E14 pup brains were harvested and placed in ice cold DPBS(1X) + 10% Pen/Strep, then they were dissected using a dissecting microscope. From each pup, one brain hemisphere was placed in 2 ml of Neurobasal media/ 10% P/S/ 10X Hepes and another brain hemisphere was placed in another tube.
The tissue from each tube was aseptically and manually dissociate into single cells, neurobasal complete medium was added and centrifuged at 130G for 5 min. The tissue was then resuspended in neurobasal complete media with GF and placed in a corning suspension culture dish 100mm X 20mm (# 430591). Cells were passage twice using a 1:3 ratio, after what they were centrifuged (130g 5min), resuspended in freezing media (10%DMSO and neurobasal complete media, no GF) and frozen in liquid nitrogen (LN2).

Thawing cells for compound screen

Vials were remove from LN2, quickly defrosted, and cells were transferred, dropwise, to a 15mL conical.10 mls of complete neurobasal media was added. Cells were transferred to a suspension culture dish, and placed in an incubator for 2 hours. At 1.5 hours, cells were examined. Based on the health and number, the number of plates needed was estimated and the appropriate amount of complete neurobasal media was warmed. After 2 hours, cells were put in a 15mL conical tube and spun at 130G, 5 min. Cells were then resuspended in Neurobasal media complete with GF (3ul of GF for every 10mL media). Cells were then grown overnight, and then use in the experiments.

Plating cells in 96-well plates for neurosphere counts and diameters only

Corning Costar 3474, 96 well plate, Ultra low attachment

Dissociation and Plating of cells

3-4 mLs of cells were taken out of the tilted plate and add to a 15 ml conical. Some of the remaining media was used to rinse down the plate. All remaining media was drawn up and put into a 15 ml conical tube, and Spun at 130G for 5 min. All media was removed. The cells were gently resuspended in 5mls of warm PBS, spun again. PBS was then removed and the cells were then gently resuspended in 500 μl of Accutase(Corning™ Accutase™ Cell Detachment Solution, # 25058CI). The cells were then Pipetted gently with a 1000μl tip to break up pellet, and then they were placed in a shaking water bath for 5-10 minutes, after which time they were swirled by hand frequently.
Media was prepared as indicated below, all media had GF:

| **Compound** | | | | | | |
|---|---|---|---|---|---|---|
| Octanoic Acid | Control/Vehicle | Low choline/Vehicle | 1%BSA | low | Med | 1:5 Oct:dec |
| Nervonic Acid | Control/Vehicle | Low choline/Vehicle | Med choline/Vehicle | low | Med | High |
| Stearic Acid | Control/Vehicle | Low choline/Vehicle | Med choline/Vehicle | low | Med | high |

(continued)

| **Compound** | | | | | | |
|---|---|---|---|---|---|---|
| Sphingomyelin | Control/Vehicle | Low choline/Vehicle | Med choline/Vehicle | low | Med | high |

Control and compound media was made with #2 media and contain 29uM Choline, Low (5uM) and medium (70uM) choline media was made with #3 media.

| Compound | Company | Cat | cas # | Stock | Vehicle/dissolved | Dose 1 | Dose 2 |
|---|---|---|---|---|---|---|---|
| Choline chloride | Sigma | 26978 | 67-48-L | 7mM | PBS | 5 μm | 70 μm |

The media was Pipetted GENTLY using a 1000 μl tip and a then a 200ul tip to further disperse cells.
Clumps were no bigger than ~3-5 cells. 5-10 mls of warm media (GF) was added to dilute enzyme. 2 mls of media was added. This was pipetted with a 1000 ul pipette, then 3 mls with added with a serological pipette. Cells were strained through a cell culture approved 40uM strainer before they were plated.
1ml was taken off to count cells. The cells were spun again. Media was removed from the cell pellet.1 mls of prepared media (no GF) was added. The cells were pipetted with a 1000ul pipette. Cell dilutions (24,000 cells/well) in 250uL of appropriate media were made. Cells were swirled daily and grown for 2 days.

Fixation and Staining

1. The cells were fixed in the hood. For fixation and subsequent immunohistochemical analysis 100ul of medium was removed and 100ul 4% PFA in 1X PBS was added to fix the cells whilst counting the neurospheres by hand, then the cells were washed twice with 1X PBS for 5 min, and left in 1X PBS, wrapped in foil and left overnight at 4°C, or Dapi staining was carried out. 100uL of PBS was removed and 100uL

of antibody (AB) staining solution (1% Goat serum, 1xPBS, and 0.1% triton X) block was added at room temp for 1 hour. AB staining solution was removed. The cells were then Stained with Dapi 1:5000 in AB staining solution, 100ul per well, the cells were then incubated at room temp for15 min in the dark. The cells were then washed 2 times
in AB staining solution for five min. Imaging was carried out using a GE Cytell imager or LSM 710, Zeiss confocal microscope and the diameters of neurospheres with ImageJ software (National Institutes of Health) was analysed.

Plating cells in 24 well plate for monolayer differentiation or EdU incorporation assays

| **Compound** | | | | | | |
|---|---|---|---|---|---|---|
| Octanoic Acid | Control/Vehicle | Low choline/Vehicle | Med choline/Vehicle | low | Med | High |
| Nervonic Acid | Control/Vehicle | Low choline/Vehicle | Med choline/Vehicle | low | Med | High |
| Stearic Acid | Control/Vehicle | Low choline/Vehicle | Med choline/Vehicle | low | Med | high |
| Sphingomyelin | Control/Vehicle | Low choline/Vehicle | Med choline/Vehicle | low | Med | high |

24 well glass bottom plates (Mat Tek P24G-1.0-13-F Case, glass bottom 24 well plates) were coated with poly-L-ornithin (Sigma P4957) and Fibronection (Sigma F1141) before use in the assay below.
See Dissociation and Plating of cells above.
Cells were plated (10,000 cells per well) in Complete media with GF for 24 hours (500ul per well). Once cells had been attached they were switched to choline deficient media, other compound media, or appropriate media.

Differentiation assay: quantification of neuronal, glial and NPC marker expression

After 24 hours, it was ensured that cells were attached to the plate, then the medium was carefully removed.

500 μL of compound media containing 2% Nu Serum (serum substitute) (Corning™ Nu-Serum Growth Medium Supplement, #CB55004), control, low choline, or medium choline media, was added. Note: medium contains no GF.

Control and compound media were made with #2 media and contain 29uM Choline,

Low (5uM) and medium (70uM) choline media was made with #3 media.

The cells were culture for 9 d in media plus 2% Nu Serum, the medium was changed every 2nd day. For fixation and subsequent immunohistochemical analysis the medium was removed, the cells were rinsed once with 1X PBS for 5 min, and fixed with 4% PFA in 1X PBS for 15min at 4°C. The cells were then washed twice with 1X PBS for 5 min, left in 1X PBS, wrapped in foil and left overnight at 4C, or they were immediately primary antibody staining was carried out.

Staining for differentiation

PBS was removed and enough AB staining solution (1% Goat serum, 1xPBS, and 0.1% triton X) was added to cover the bottom, the block was kept at room temp for 1 hour.

Primary antibody dilutions were made in appropriate amount of AB staining solution, 250 ul per well (the antibodies were only kept out on ice for a short time, mouse anti-MAP2 or TUJ1 1:500 (neuron marker),rabbit anti-GFAP(glial marker) 1:1000, chicken anti-Nestin CFP (EGFP antibody (progenitor cell marker)) 1:1000. AB staining solution was removed and a solution of primary antibodies was added to each chamber. The cells were wrapped in foil and kept overnight 4°C. The cells were then washed with 400ul of

AB staining solution for 5 min once to remove primary antibodies. Secondary antibody solutions were made (enough for 250ul of each chamber) (Goat anti-mouse alexa 488 1:2000, anti-rabbit Cy3 (1:500), anti-chicken alexa 647 1:500, and 1:5000 Dapi).

The cells were incubated at room temp for1 hour in the dark and washed 2 time in AB staining solution for five mins. They were then kept at 4°C or Imaged using a GE Cytell imager or LSM 710, Zeiss confocal microscope and analyze with ImageJ software(National Institutes of Health).

Microtubule-associated protein 2 (MAP2), Neuronal beta-tubulin III(TuJ1), glial fibrillary acidic protein (GFAP), and Nestin CFP (EGFP antibody).

Each marker expression was measured on collected images (integrated density

measure in ImageJ) and normalized to DAPI fluorescence, marking all nuclei (integrated density measure).

Octanoic Acid were labeled with neuronal beta-tubulin III

(TuJ1), subsequent compounds were labeled with Microtubule-associated protein 2 (MAP2).

Monolayer culture NPC proliferation assay (incorporation of EdU - S phase marker)

After 24 h, it was ensured that cells had attached to the plate then the medium was carefully removed. 500 μL of compound media plus GF.was added. The cells were cultured for 3 days in appropriate media.

Control and compound media was made with #2 media and contains 29uM Choline,

Low (5uM) and medium (70uM) choline media was made with #3 media.

EDU incorporation was measured using Click-iT® EdU Alexa Fluor® 555 Imaging Kit (Life technologies, # c10338).

At the end of day 3 EdU was added to each well at 10μM for 30 minutes prior to fixation.

For fixation and subsequent immunohistochemical analysis, the medium was removed, the cells were rinsed once with 1X PBS for 5 min, and fixed with 4% PFA in 1X PBS for 15min at 4°C, then the cells were washed twice with 1X PBS for 5 min, left in 1X PBS, wrapped in foil and leave overnight at 4°C, or staining was proceeded with.

PBS was removed and enough AB staining solution (1% Goat

serum, 1xPBS, and 0.1% triton X) added to cover the bottom, the block was kept at room temp for 1 hour. The cells were stained for EDU. The cells were Incubated for 30min at room temp, in the dark. The cells were washed with 1X PBS and stained with Dapi 1:5000ul for 15 min. The cells were washed once with 1X PBS, then left in PBS at 4°C, or Imaged immediately using a GE Cytell imager (cell viability application), or LSM 710, Zeiss confocal microscope (with ImageJ software (National Institutes of Health) analysis).

Results are shown in Tables 3 -7 and figures 2 to 5.

Table 3 - Effect of Nervonic acid on Neuronal Cell density and astrocyte cell density

| | neuronal | astrocytic |
|---|---|---|
| control | 0.436189 | 0.642448 |
| lowNA | 0.467588 | 0.621784 |
| medNA | 0.56563 | 0.721512 |

(continued)

| | neuronal | astrocytic |
|---|---|---|
| highNA | 0.539448 | 0.70279 |

Table 4 Effect of Stearic acid on Neuronal Cell density and astrocyte cell density

| | Neuronal | astrocytic |
|---|---|---|
| control | 0.44 | 0.64 |
| lowSA | 0.54 | 0.68 |
| medSA | 0.66 | 0.81 |
| highSA | 0.64 | 0.85 |

Table 5 Effect of Octanoic acid on Neuronal Cell density and astrocyte cell density

| | Neuronal | Astrocytic |
|---|---|---|
| Control | 1.1 | 4.1 |
| lowOA | 1.2 | 3.7 |
| medOA | 1.8 | 6.1 |
| HighOA | 1.7 | 7.9 |

Table 6 Effect of Sphingomyelin on number of neurospheres

| Sphingomyelin | Control | Low | Med | High |
|---|---|---|---|---|
| Average (Mean) | 103 | 94 | 121 | 219 |

Table 7 Effect of Sphingomyelin on neuronal proliferation

| | DAPI |
|---|---|
| control | 262 |
| lowSM | 280 |
| medSM | 314 |
| highSM | 305 |

**Example 5**

**Experimental part**

**Samples**

Ingredient C2 a whey protein concentrate enriched in alpha lactalbumin(Sample Manager ID: K2Q-00030); first infant milk containing whey protein concentrate enriched in alpha Lactalbumin (Sample Manager ID: K2Q-00032); cow's milk (whole milk); human breast milk (quality control pool of 6 individual samples, collected after week 4 following child birth; Lee Biosolutions, St Louis, MI, USA).

**Extraction of phospholipids from milk products**

*Milk powder:* A quantity of 1g of homogenized powder was weighed into a 50-mL glass flask and diluted into

20 mL of pure distilled water. The solution was heated at 40 °C for 30 min in a water bath. A volume of 500 μL of this solution was put in a 10-mL glass tube.

*Cow milk and Human milk:* A quantity of 500 μL of homogenized liquid was aliquoted to a 10-mL glass tube.

Analytes were extracted following the MP on quantification of human breast milk by UPLC-MS/MS (RDLS-MP-80138-Rev01) in triplicate using 9.5 mL of a mixture of chloroform/methanol (2+1). Briefly, the tubes were shaken and placed in an ultrasonic bath at 40 °C for 15 min, followed by centrifugation for 10 min at 2500 rpm. A volume of 2 mL of potassium chloride solution (0.88%, m/m) was added to the liquid phase then shaken and centrifuged for 10 min at 2500 rpm. The lower organic phase was transferred into a glass vial, evaporated to dryness under gentle $N_2$ stream and reconstituted in 500 μL of chloroform/methanol (9+1) before injection into the LC-MS.

## Analysis of phospholipids by liquid chromatography coupled to mass spectrometry (LC-MS)

Analyses were performed on a Q Exactive Plus Orbitrap (Thermo Fisher Scientific, Bremen, Germany) equipped with a Thermo Scientific Dionex UltiMate 3000 Rapid Separation LC system. Separation was performed on an HILIC column (100 x 2.1 (i.d.) mm; 1.7 μm) with a mobile phase composition of (A) ammonium acetate 10 mM and (B) acetonitrile. The injection volume was set to 10 μL and the gradient started from 95% B to 70% B over 15 min, maintained 1 min at 70% B, returned to initial conditions in 3 min and equilibrated for 6 min.

Q Exactive Plus Orbitrap was equipped with an atmospheric pressure chemical ionization (APCI) probe operated in the positive ion mode. APCI and MS parameters were as follows: corona discharge current 4.0 μA, sheath gas and auxiliary gas 24 and 5 arbitrary units, respectively; capillary and vaporizer temperatures 320 and 390 °C, respectively, sweep gas flow rate was 0 arbitrary unit and s-lens RF level was 80. Automatic gain control (AGC) target value was set at $1 \times 10^6$ charges and maximum injection time at 100 ms with resolution of 35'000 and 1 microscan *per* full MS. AGC was set to $1 \times 10^6$ charges and maximum injection time of 250 ms with resolution of 17'500 with 1 microscan in the data independent fragmentation mode. An inclusion list of selected parent ions was used with normalized collision energy of 30%. Data were acquired over the mass range 133-2000 Da in profile mode. External mass calibration was applied. The system was controlled by Xcalibur 3.0 (Thermo Fisher Scientific).

SM species were extracted from total ion chromatogram using accurate mass. Parent ions corresponded to in-source loss of phosphatidylcholine into ceramide. An inclusion list was used for specific fragmentation of 57 SM regioisomers built on parent ions corresponding to *m/z* of ceramide with loss of water [Cer-$H_2O$+$H^+$], based on LipidView database and literature (Trenerry V.C., Akbaridoust G., Plozza T., Rochfort S., Wales W.J., Auldist M., Ajilouni S. Ultra-highperformance liquid chromatography-ion trap mass spectrometry characterisation of milk polar lipids from dairy cows fed different diets. Food Chemistry 2013, 141, 1451-1460; Godzien J., Ciborowski M., Martinez-Alcazar M.P., Samczuk P., Kretowski A., Barbas C. Rapid and reliable identification of phospholipids for untargeted metabolomics with LC-ESI-QTOF-MS/MS. Journal of Proteome Research 2015, 14, 3204-3216).

## Analysis of fatty acid methyl ester (FAME) by gas chromatography with flame ionization detector (GC-FID)

SM fractions were collected between 8.5 and 10 min into glass tubes 5 times for each sample. After solvent evaporation under $N_2$ stream, FAME analyses were conducted in triplicate following the MP for quantification of fatty acid in human milk by gas chromatography (RDLS-MP-8980-00030-Rev01-FAME_Human milk fat 2012, Vers. 1.0).

## Result and Discussion

Hydrophilic interaction liquid chromatography (HILIC) was used to separate PL classes *(i.e.* phosphatidylinositol (PI), phosphatidylserine (PS), phosphatidylethanolamine (PE), phosphatidylcholine (PC) and SM). The number of carbons and unsaturation within the individual SM species was assigned based on the accurate mass of the pseudo-molecular ion detected in the Orbitrap mass spectrometer. Relative abundance of SM species was determined for comparison between ingredient, infant formula, cow's milk and human milk.

## SM species in different milk products

45 SM species were detected in the analysed samples (Table 8).

**Table 8:** SM species detected (indicated by x) in ingredient, infant formula, cow's milk and human milk samples. SM species that were only detected in human milk are indicated in bold.

| SM | Ingredient | Infant Formula | Cow's Milk | Human Milk | SM | Ingredient | Infant Formula | Cow's Milk | Human Milk |
|---|---|---|---|---|---|---|---|---|---|
| **SM 24: 1** | | | | X | SM 37: 1 | X | X | X | X |
| SM25:0 | X | X | X | X | SM 37: 0 | X | X | X | X |
| SM28:1 | X | X | X | X | **SM 38: 4** | | | | X |
| SM 28: 0 | X | X | X | X | **SM 38: 3** | | | | X |
| SM 30: 2 | X | X | X | X | SM 38: 2 | X | X | X | X |
| SM 30: 1 | X | X | X | X | SM 38: 1 | X | X | X | X |
| SM 30: 0 | X | X | X | X | SM 38: 0 | X | X | X | X |
| SM 32: 3 | X | X | X | X | SM 39: 1 | X | X | X | X |
| SM 32: 2 | X | X | X | X | SM 39: 0 | X | X | X | X |
| SM 32: 1 | X | X | X | X | SM 40: 2 | X | X | X | X |
| SM 32: 0 | X | X | X | X | SM 40: 1 | X | X | X | X |
| SM 33: 1 | X | X | X | X | SM 40: 0 | X | X | X | X |
| SM 34: 3 | X | X | X | X | SM 41: 2 | X | X | | |
| SM 34: 2 | X | X | X | X | SM 41: 1 | X | X | X | X |
| SM 34: 1 | X | X | X | X | SM 41: 0 | X | X | X | X |

(continued)

| SM | Ingredient | Infant Formula | Cow's Milk | Human Milk | SM | Ingredient | Infant Formula | Cow's Milk | Human Milk |
|---|---|---|---|---|---|---|---|---|---|
| SM 34: 0 | X | X | X | X | **SM 42: 4** | | | | X |
| SM 35: 2 | X | X | X | X | SM 42: 3 | X | | X | |
| SM 35: 0 | X | X | X | X | SM 42: 2 | X | X | X | X |
| SM 36: 4 | X | X | X | X | SM 42: 1 | X | X | X | X |
| SM 36: 3 | X | X | X | X | SM 42: 0 | X | X | X | X |
| SM 36: 2 | X | X | X | X | SM 44: 3 | X | | X | |
| SM 36: 1 | X | X | X | X | SM 44: 1 | X | X | X | X |
| SM 36: 0 | X | X | X | X | | | | | |

The species SM 24:1, SM 38:4, SM 38:3 and SM 42:4 were only found at trace levels in human milk.

**Relative abundance of SM species**

The relative abundance (%) of SM within different milk products was estimated based on the peak area divided by the sum of all peak area corresponding to SM species in the chromatogram *per* each sample. Figure 6 shows the relative abundance of the main SM species in ingredient, infant formula, cow's milk and human milk.

The relative abundance of SM species present in ingredient and infant formula was comparable to that of cow's milk, and slightly different from human milk portion of some species (e.g. SM 32:1, SM 32:0, SM 33:1; SM 34:1, SM 38:0, SM 39:1, SM 39:0 and SM 41:1) were lower in human milk than in ingredient, infant formula and cow's milk. Whereas SM 36:2, SM 36:1, SM 36:0, SM 37:1, SM 38:2, SM 38:1, SM 40:1, SM 42:2 and SM 42:1 had higher relative abundance in human milk compared to the other milk products.

Human milk sample consisted of a quality control pool of 6 individual samples collected at or later than 4 weeks after child birth. Knowing that SM abundance in human milk varies in function of the diet and lactation time, this can partly explain the observed differences. Nevertheless, despite the variations in the relative abundance of some SM species, >85 % of the SM species that were detected in human milk were also identified in infant formula and in cow's milk.

It is noteworthy that for a given m/z extracted from the MS trace, different LCB-FA combinations could be suggested (e.g. SM 34:1 could correspond to SM d18:1/16:0, d18:0/16:1, dl6:1/18:0etc.). Therefore, we evaluated GC FA profile to gather more information on the SM molecular structures between the different milk products.

**Fatty acid profile in SM fraction from different milk products**

Regioisomeric structure of SM was investigated by first fractionating the SM and then analysing the FA present in the fractions by GC-FID. Fractionation of SM was performed as described above for LC-MS analysis, but in this case the effluent was directed into a 5-mL glass tube instead of the MS. Each fraction was then subjected to methylation procedure before subsequent GC analysis . The relative abundance of FAs within the SM fraction is represented in

Figure 7.

As shown in Figure 7, SM fraction contained mostly saturated FAs (i.e. myristic acid 14:0, pentadecylic acid 15:0, palmitic acid 16:0, stearic acid 18:0, arachidic acid 20:0, behenic acid 22:0, tricosylic acid 23:0 and lignoceric acid 24:0). A higher proportion of SFA was observed in SM fraction from all milk products (Table 9). This is in agreement with literature, revealing high distribution of SFA with carbon chain higher than 18 in SM fraction. This high amount of SFA reflects the structural role of SM, namely lessen fluidity and maintain rigidity of the milk fat globule membrane.

**Table 9:** Percentage of SFA, MUFA and PUFA detected in SM fraction from different milk products.

| | FA | Ingredient | Infant Formula | Cow's Milk | Human Milk |
|---|---|---|---|---|---|
| SFA % | 14:0-16:0; 18:0; 20:0-24:0 | 95.1 ± 2.6 | 92.8 ± 3.9 | 93.1 ± 8.4 | 87.9 ± 2.7 |
| MUFA % | 18:1n-9 | 2.5 ± 0.7 | 3.6 ± 0.9 | 5.3 ± 1.5 | 4.4 ± 1.9 |
| | 24:1n-9 | 1.7 ± 0.3 | 1.9 ± 1.7 | 1.2 ± 0.1 | 5.9 ± 0.6 |
| PUFA % | 18:2n-6 | 0.8 ± 0.4 | 1.7 ± 0.2 | 0.3 ± 0.4 | 1.7 ± 0.1 |

Monounsaturated FAs (MUFAs) represented about 4-11% of the FA in the SM fraction. Oleic acid 18:1n-9 and nervonic acid 24:1n-9 were the 2 MUFAs detected. Interestingly, 24:1n-9 was found in relative higher proportion in human milk compared to the other milk products and this is in agreement with the literature. The only PUFA linoleic acid 18:2n-6 was found relatively higher in the tested infant formula and human milk compared to the other products. Finally, omega-3 PUFAs were not detected in SM fraction. This is also in accordance with data found in the literature showing that arachidonic acid (AA, 20:4n-6), eicosapentaenoic acid (EPA, 20:5n-3) and docosahexaenoic acid (DHA, 22:6n-3) are mainly present in PE, PI and PS.

**Example 6**

An examples composition in accordance with the invention is set out in Table 10. Said composition could be a pre-pregnancy, pregnancy or lactation supplement.

Table 10

Table 10

| Ingredient | Amount per daily dose |
|---|---|
| Vitamin B12 | 5.2 µg |
| Zinc | 10 mg |
| Folic acid | 400 µg |
| Iron | 5mg |
| Calcium | 500 mg |
| Phosphorus | 500mg |
| Zinc | 3mg |
| copper | 0.2mg |
| Magnesium | 100 mg |
| DHA | 800mg |
| ARA | 400mg |
| Choline | 300mg |
| Phosphatidylcholine | 300mg |
| Phosphatidylinositol | 250mg |
| Phosphatidylserine | 400mg |

(continued)

| Ingredient | Amount per daily dose |
|---|---|
| Sphingomyelin | 650mg |

Example 7

Co culture of neurons and OL

Neurons / Oligodendrocytes were cultured as previously described by Charles et al., 2000. Pregnant female rats of 17 days gestation were killed by cervical dislocation (Rats Wistar) and the foetuses removed from the uterus. The Forebrains were removed and placed in ice-cold medium of Leibovitz (L15) containing 2% of Penicillin-Streptomycin (PS) and 1% of bovine serum albumin (BSA). Forebrains were dissociated by trypsinisation for 20 min at 37ºC (Trypsin EDTA 1X). The reaction was stopped by the addition of Dulbecco's modified Eagle's medium (DMEM) containing DNAase I grade II (0.1 mg/ml) and 10% of foetal calf serum (FCS). Cells were then mechanically dissociated by 3 passages through a 10 ml pipette. Cells were then centrifuged at 180 x g for 10 min at 4°C temperature on a layer of BSA (3.5%) in L15 medium. The supernatant was discarded and the cells of pellet were resuspended in DMEM containing 10% of FCS. Cells were then centrifuged at 515 x g for 10 min at 4°C. The supernatant was discarded and the cells of pellet were re-suspended in a culture medium consisting of Neurobasal supplemented with 2% of B27, 2 mM of L-glutamine (L Glu), 2% of PS solution, 1 % of FCS and 10 ng/ml of platelet-derived growth factor (PDGF-AA). Viable cells were counted in a Neubauer cytometer using the trypan blue exclusion test. The cells were seeded at a density of 20000 cells/well in 96 well-plates precoated with poly-L-lysine and laminin.

The day following seeding (day 1 of culture), cells were incubated with a test compound (selected from those listed in table 11), or estradiol. Control cells were not incubated with a test compound or estradiol. Estradiol was used as positive control. Estradiol is known to induce OPC proliferation. The positive effect of estradiol on OL differentiation has also been demonstrated, as has its effect on the early myelination process. The positive effect of estradiol on neurite outgrowth was also published (for review see Alevaro et al., 2010). The plates were maintained at 37°C in a humidified incubator, in an atmosphere of air (95%)-CO2 (5%). Half of the medium was replaced every other day with fresh medium and test compound or control compound. The test or control compounds were maintained at the defined concentration for the duration of the experiments. Compounds were tested on 1 culture (6 wells per conditions). Cells were then used on day 12, 18 or 30 of culture to measure one of either proliferation of OPC, differentiation of OPC into OL and early myelination process (myelin wrapping), or maturation of OL (myelin maturation) and mature myelination process (myelin wrapping).

Proliferation of OPC - Measurement of A2B5 positive cells and total axonal length (NF)

On day 12 of culture, cells were fixed by a cold mixture of absolute ethanol (95%) and pure acetic acid (5%) for 5 min. The cells were then permeabilized and non-specific sites were blocked with a solution of phosphate buffered saline (PBS) containing 0.1% of saponin and 1% FCS for 15 min at room temperature.

Cells were then incubated with Monoclonal Anti-A2B5 conjugated alexa fluor® 488 produced in mouse at dilution of 1/200 in PBS containing 1% FCS, 0.1 % saponin, for 2 h at room temperature and with anti-NF (Neurofilament 200 phosphorylated and non-phosphorylated) produced in rabbit at dilution of 1/500 in PBS containing 1% FCS, 0.1 % saponin for 2 h at room temperature. This antibody was revealed with Alexa Fluor 568 goat anti-rabbit at the dilution of 1/400 in PBS with 1% FCS, 0.1 % saponin, for 1 h at room temperature.

The total number of OPC (number of A2B5 positive cells) was quantified (to evaluate the proliferation), the axonal network was measured (total axonal length (NF)) to assess the effect of the compound on the neuronal network (the quality of the myelination is directly linked to the quality of the axonal network).

Differentiation of OPC into OL and myelination process (myelin wrapping) - Measurement of number and area of MAG positive cells, overlap MAG/NF wrapping, and total axonal length (NF)

On day 18 of culture, cells were fixed by a cold mixture of absolute ethanol (95%) and pure acetic acid (5%) for 5 min. The cells were then permeabilized and non-specific sites were blocked with a solution of phosphate buffered saline (PBS) containing 0.1% of saponin and 1% FCS for 15 min at room temperature.

Cells were then incubated with Monoclonal Anti-MAG produced in mouse at dilution of 1/400 in PBS containing 1% FCS, 0.1 % saponin, and with anti-NF (Neurofilament 200 phosphorylated and non-phosphorylated) produced in rabbit at dilution of 1/500 in PBS containing 1% FCS, 0.1 % saponin for 2 h at room temperature. These antibodies were

revealed with CF 488 A goat anti-mouse at the dilution of 1/800 in PBS with 1% FCS, 0.1 % saponin and Alexa Fluor 568 goat anti-rabbit at the dilution of 1/800 in PBS with 1% FCS, 0.1 % saponin, for 1 h at room temperature.

The total number of OL was quantified (number and area of MAG positive cells) (to evaluate the differentiation process), as well as the wrapping of OPC around axons (overlap MAG/NF wrapping) (myelination process). The axonal network was measured (total axonal length (NF) to assess the effect of the compounds on the neuronal network.

Maturation of OL (myelin maturation) - Measurement of number and area of MBP positive cells, overlap MBP/NF wrapping, and total axonal length (NF)

On day 30 of culture, cells were fixed by a cold mixture of absolute ethanol (95%) and pure acetic acid (5%) for 5 min. The cells were then permeabilized and non-specific sites were blocked with a solution of phosphate buffered saline (PBS) containing 0.1% of saponin and 1% FCS for 15 min at room temperature.

Cells were then incubated with Monoclonal Anti-MBP produced in mouse at dilution of 1/1000 in PBS containing 1% FCS, 0.1 % saponin, and with anti-NF (Neurofilament 200 phosphorylated and non-phosphorylated) produced in rabbit at dilution of 1/500 in PBS containing 1% FCS, 0.1 % saponin for 2 h at room temperature. These antibodies were revealed with CF 488 A goat anti-mouse at the dilution of 1/800 in PBS with 1% FCS, 0.1 % saponin and Alexa Fluor 568 goat anti-rabbit at the dilution of 1/400 in PBS with 1% FCS, 0.1 % saponin, for 1 h at room temperature.

The total number of OL was assessed (number and area of MBP positive cells) (to evaluate the OL maturation) as well as the wrapping of myelin around axon (overlap MBP/NF(wrapping)). The axonal network was measured (Total axonal length (NF)) to assess the effect of the compounds on the neuronal network.

For all measurements, once the culture was done (6 wells per conditions). For each condition tested, 30 pictures (each picture representing a field) per well were taken and analyzed using ImageXpress (Molecular devices) with 20x magnification equipped with LED lamp (excitation 360/480/565 and emission 460/535/620). The 30 pictures were automatically taken and represented 80% of the total surface of the culture well.

Results were expressed in terms of cumulated mean length in μm of neurite network, or myelin sheath labeled for a given marker (MAG or MBP) per field. The overlapping area between NF and MAG or MBP was measured to evaluate the wrapping.

To assess OPC population, MAG positive cell population, MBP positive cell population, an automatic counting of number of positive cells per picture (=field) was done. The results were expressed in mean number of positive cells per field.

All the images were taken under the same conditions.

Table 11

| PLATE 1 (A2B5 / NF) |
|---|
| Control |
| Estradiol (150 nM) |
| DHA (0.15 μM) |
| DHA (1.5 μM) |
| Stearic acid (50 μM) |
| Stearic acid (5 μM) |
| Stearic acid (0.5 μM) |
| B12 (100 nM) |
| B12 (10 nM) |
| B12 (1 nM) |
| Folic acid (250 nM) |
| Folic acid (50 nM) |
| Folic acid (6 nM) |
| Choline (20 μM) |
| Iron (1 μM) |
| Iron (0,1 μM) |

(continued)

| |
|---|
| PLATE 1 (A2B5 / NF) |
| Zinc (5 μM) |
| Zinc (0,5 μM) |
| Phosphorus (5 mM) |
| Phosphorus (1 mM) |
| Magnesium (25 mM) |
| Copper (0,5 μM) |
| Phosphatidylcholine (100 μM) |
| Phosphatidylinositol (5 μM) |
| Phosphatidylinositol (50 μM) |
| Phosphatidylserine (5 μM) |
| Phosphatidylserine (10 μM) |
| Phosphatidylserine (100 μM) |
| Sphingomyelin (5 μM) |
| Sphingomyelin (25 μM) |
| Ceramide(brain extract):DPPC (1:4) |
| galactoceramides (C18:1/24:1)/(C18:1/18:0):DPPC (1:4) |
| glucoceramides (C18:1/24:1)/(C18:1/18:0):DPPC (1:4) |
| D-erythro-dihydroceramide (C24:1/18:0)/(C18:0/18:1):DPPC (1: 4) |
| Ceramide-1-phosphate (C18:1/24:0):DPPC (1:4) |
| GM3:DPPC (1:4) |
| GD3:DPPC (1:4) |

Results are show in Figures 8 to 28

**Example 8**

Materials and methods

**1. Feeder layer preparation: Dissociation of neonatal cortices and maintenance of mixed glial cultures**

Freshly dissected brains were added to a 37°C water bath for 3 min, then cortices were diced through a P1000 pipette tip to generate smaller fragments. 75 μL of OPC papain solution per brain were added, then tissues were incubated in a 37°C water bath for 20 min. The tissue suspension was then additioned with mixed glial culture in order to allow inactivation of the OPC papain solution.

Tissue were subsequently triturated using a sterile flame-polished glass Pasteur pipette, then 4 mL of mixed glial culture media per brain was added. Cells were centrifuged at 1200 rpm (~300 g) for 5 min, then cells were resuspended in warm mixed glial culture media and plated into PLL-coated flask.

4 hours following plating, a full media change was performed in order to remove much of the debris caused by the trituration, and promote culture viability. After 3 days of culture, a 2/3 media change was performed, and no subsequent medium change was performed. Cells were then maintained in culture until confluency.

**2. Hippocampal neurons preparation**

Hippocampal neurons were isolated from embryonic (E18) pups of Sprague Dawley rats. Briefly, following

animal sacrifice, brains were isolated, meninges removed from the medial aspect of the cerebral hemispheres, then hippocampi dissected out and kept at 4°C until process completion.

Tissue were then incubated with 2.5% trypsin for 15 min in a water bath at 37°C, then gently washed and kept in culturing media. Hippocampal dissociation was performed by repeatedly pipetting them up and down with a functionalized sterile Pasteur pipette. Following mechanical dissociation, cells were plated at desired density in neuronal plating medium, let recover for 4 hours, then put in compete neuronal culturing medium.

**3. Purification of OPCs from mixed glial cultures for establishment of OL/hippocampal neurons o-cultures**

On Day 9 of the mixed glial culture, flasks were shaken at 50 rpm for 45 min on an orbital shaker in a 5% CO2 tissue culture incubator. The purpose of this shake was to remove any loosely adherent contaminating cells from the monolayer.

Media was then changed and replaced with 4 mL of fresh mixed glial culture media supplemented with 5 μg/mL insulin. Flasks were then repositioned onto the shaker, equilibrated for approximately 3 hours, then shaken for approximately 16 hours at 220 rpm (overnight).

The next morning, mixed glia culture medium containing microglia and OPCs cells were collected and preplated on P100 petri dish (not treated for culture) for 30 minutes in order to purify OPCs cells; microglia cells start immediately to adhere to petri while OPCs cells remained in the surnatant medium.

After 30 minutes of pre-plate, medium was collected and OLs were counted and seeded on hippocampal neurons in a final volume of 1 mL OL media.

A full OL media (minus CNTF) change was performed, then cells were maintained in culture until the appropriate experimental timings.

For maturation experiments, the experimental procedure was as follows:

a. Growth of OPCs on feeder layer of astrocytes for 10 DIV
b. Isolation of OPCs (Day 0)
c. Administration of compounds (Day 3)
d. Quantitative evaluation of maturation at Day 4, 7 and 10.

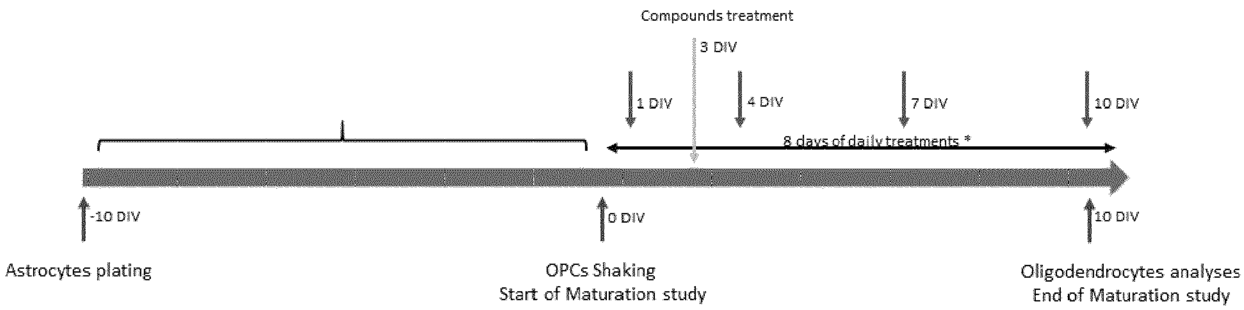


For myelination experiments, the experimental procedure was as follows:

a. Growth of hippocampal neurons until complete neuronal network maturation (14 DIV)
b. Concomitant growth of OPCs on feeder layer of astrocytes for 10 DIV
c. Isolation of OPCs and coculturing with neurons (Day 14)
d. Administration of compounds (Day 15)
e. Quantitative evaluation of myelination at Day 15 ( 1 day after coculture plating, before compound treatment), 18, 21/23 and 28/29 of coculturing

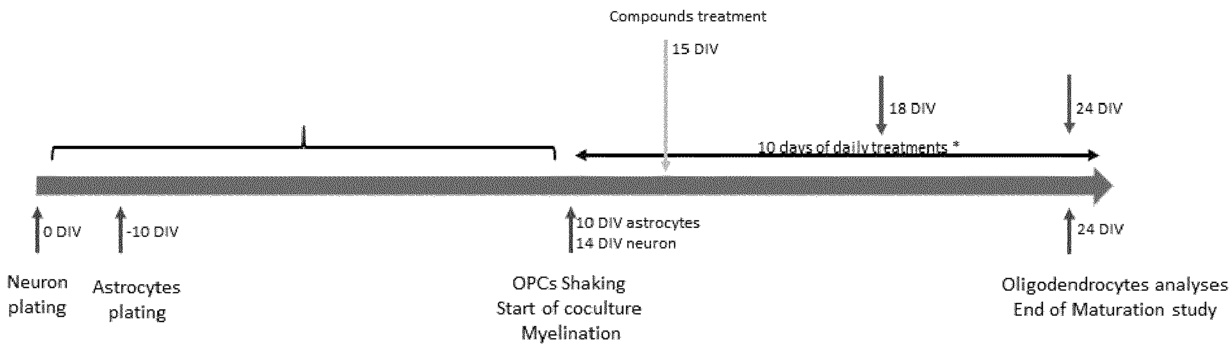


### 4. Acquisition of images

All cultures at the different experimental time points, were fixed in 4% paraformaldehyde and 4% sucrose at room temperature (RT) for 10 min. Primary and secondary antibodies were applied in GDB buffer (30 mM phosphate buffer, pH 7.4, containing 0.2% gelatin, 0.5% Triton X-100, and 0.8 M NaCl) for 2 h at room temperature. cells were stained with appropriate marker (primary antibody used: Anti-A2B5 antibody (ABCAM cat. ab53521), Rat anti MBP (BIO-RAD cat. aa82-87), Oligodendrocyte Marker O4 Antibody (R&D Systems cat. MAB1326), Anti-βIII Tubulin mAb (Promega cat. G7121); secondary antibody used: Alexa anti rat 555 (Life Tech A-21434), Alexa anti mouse 488 (Life Tech A-11009). Following immunocytochemical staining all images were acquired with Array Scan XTI (ThermoScientific); the objective was 20x at binning 2x2. For each condition and replica well (triplicate) a minimum of 15 images were taken.

For the analysis of all acquired images the HCS Studio Cell Analysis Software was used, in particular the "Scan" application.

### OPC papain solution (made up in MEM)

Papain solution 1.54 mg/mL

L-cysteine 360 μg/mL

DNase I 60 μg/mL

### Mixed glial culture media (made up in DMEM)

FBS 10%

Pen/Strep (0.33% from stock) 33 units/mL Penicillin and 33 μg/mL Streptomycin

GlutaMAX 1%

### OL media

DMEM
100X OL-Supplement
Bovine insulin (from 1 mg/mL stock)
GlutaMAX
Holo-transferrin (from 33 mg/mL stock)
B27 Supplement
FBS
CNTF (from 50 ng/μL stock)

Results are show in Figures 30 to 49.

**Claims**

1. A composition comprising one or more fatty acid derivatives for use in promoting, supporting or optimizing one or more of the following:

    (i) de novo myelination;
    (ii) brain structure;
    (iii) brain connectivity;
    (iv) intellectual potential;
    (v) cognitive potential;
    (vi) learning potential; and
    (vii) cognitive functioning

    in an offspring of a female subject, wherein said composition is for administration to the female subject and wherein the offspring is a fetus, neonate, infant or child and wherein said composition is for administration to the female subject pre-pregnancy, during pregnancy, or during lactation, or a combination thereof.

2. A composition according to claim 1 wherein said composition promotes, supports or optimizes the amount and/or spatial distribution of myelinated matter throughout the brain.

3. A composition according to any preceding claim wherein said composition promotes, supports or optimizes the de novo myelination trajectory.

4. A composition according to any preceding claim wherein the female subject is a human.

5. A composition according to any preceding claim wherein the fatty acid derivative comprises DHA, nervonic acid, and/or stearic acid.

6. A composition according to any preceding claim wherein said composition further comprises one or more of the following ingredients: a vitamin, a mineral, choline and a phospholipid, or a metabolite thereof, or a metabolic precursor thereof, or a mixture thereof.

7. A composition according to claim 6 wherein the vitamin is selected from vitamin B12, folic acid, and combinations thereof.

8. A composition according to claim 6 wherein the mineral is selected from iron, zinc, calcium, phosphorus, copper, magnesium, and combinations thereof.

9. A composition according to claim 6 wherein the phospholipid is selected from the group consisting of phosphatidyl choline, phosphatidyl inositole, phosphatidyl serine, phosphatidyl ethanolamine, sphingomyelin and mixtures thereof, and metabolic precursors and metabolites of any of the foregoing, and mixtures thereof.

10. A composition according to any preceding claim wherein the composition is in the form of a pre-pregnancy (pre-conception) supplement and/or a maternal supplement (pregnancy and/or lactation supplement).

11. One or more fatty acid derivatives for use in promoting, supporting or optimizing one or more of the following:

    (i) de novo myelination;
    (ii) brain structure;
    (iii) brain connectivity;
    (iv) intellectual potential;
    (v) cognitive potential;
    (vi) learning potential; and

(vii) cognitive functioning

in an offspring of a female subject, wherein said fatty acid is for administration to the female subject pre-pregnancy, during pregnancy or during lactation, or a combination thereof.

**12.** A method of promoting, supporting or optimizing one or more of the following:

(i) de novo myelination;
(ii) brain structure;
(iii) brain connectivity;
(iv) intellectual potential;
(v) cognitive potential;
(vi) learning potential; and
(vii) cognitive functioning

in an offspring of a female subject, said method comprising administering to the female subject a therapeutically effective amount of one or more fatty acid derivatives wherein said fatty acid derivative is administered to the female subject pre-pregnancy, during pregnancy or during lactation, or a combination thereof.

**13.** A method according to claims 12 wherein said fatty acid derivative is administered to the female subject separately, sequentially or simultaneously with one or more of the following ingredients: a vitamin, a mineral, choline, and a phospholipid, or a metabolite thereof, or a metabolic precursor thereof, or a mixture thereof.

**14.** Use of one or more fatty acid derivatives in the manufacture of a composition to promote, support or optimize one or more of the following:

(i) de novo myelination;
(ii) brain structure;
(iii) brain connectivity;
(iv) intellectual potential;
(v) cognitive potential;
(vi) learning potential; and
(vii) cognitive functioning

in an offspring of a female subject, wherein said composition is for administration to the female subject pre-pregnancy, during pregnancy or during lactation, or a combination thereof.

**15.** A composition comprising a fatty acid comprising DHA and choline and/or vitamin B12 and/or folic acid, and/or sphingomyelin and/or Iron wherein the composition is in the form of a pre-pregnancy (pre-conception) supplement and/or a maternal supplement (pregnancy and/or lactation supplement).

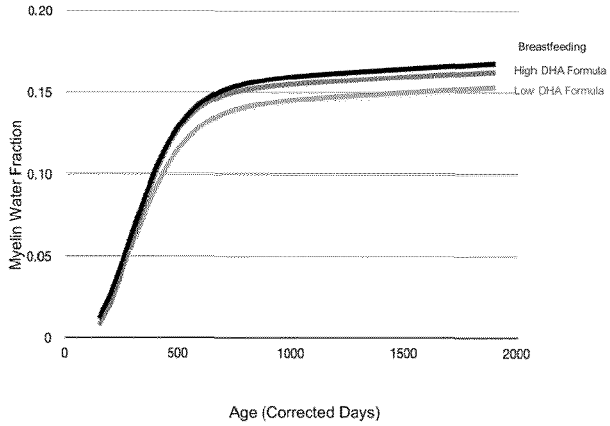


Fig. 1

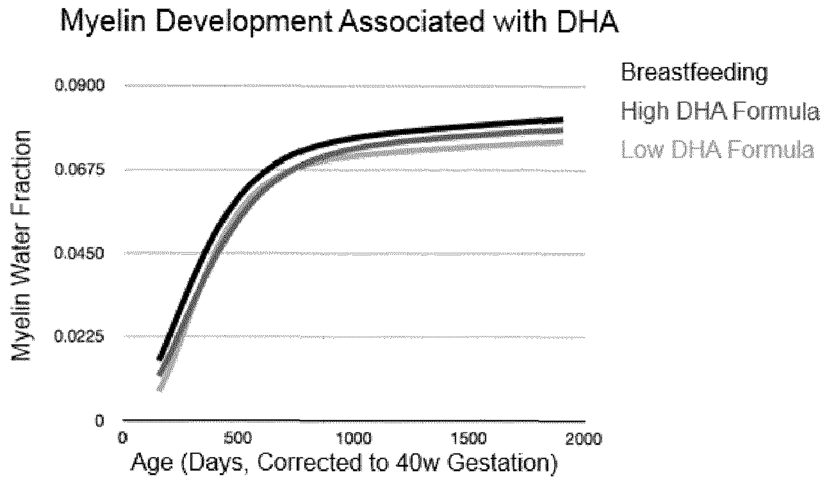


Fig.1a

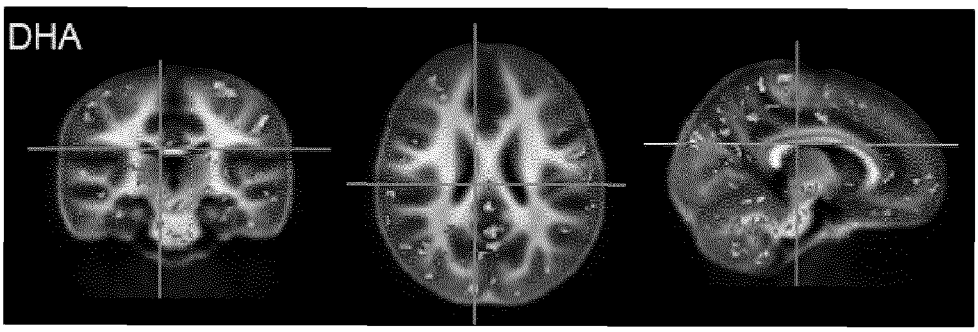


Fig.1b

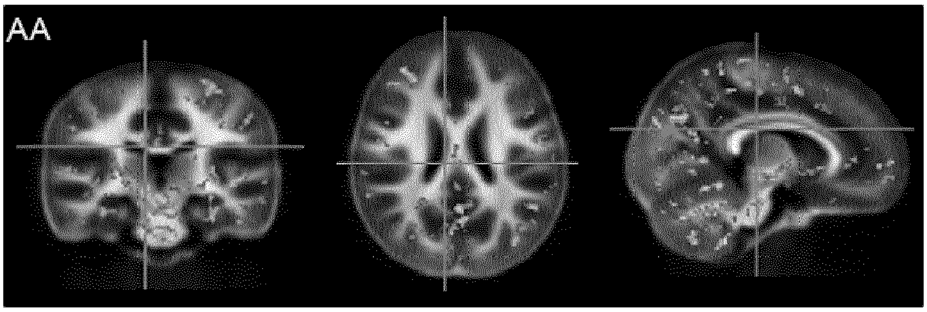


Fig.1c

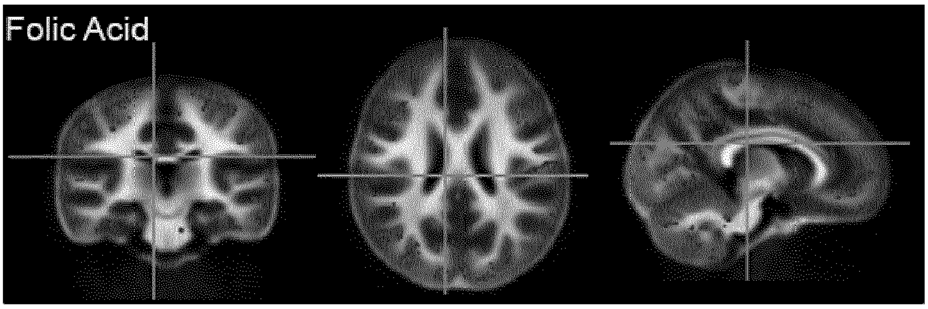


Fig.1d

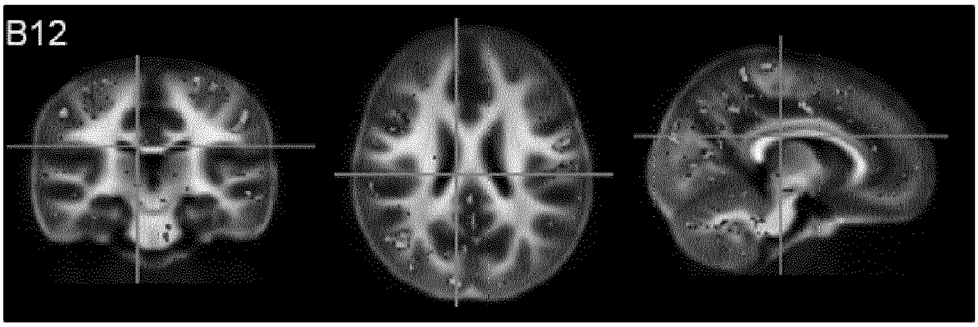


Fig.1e

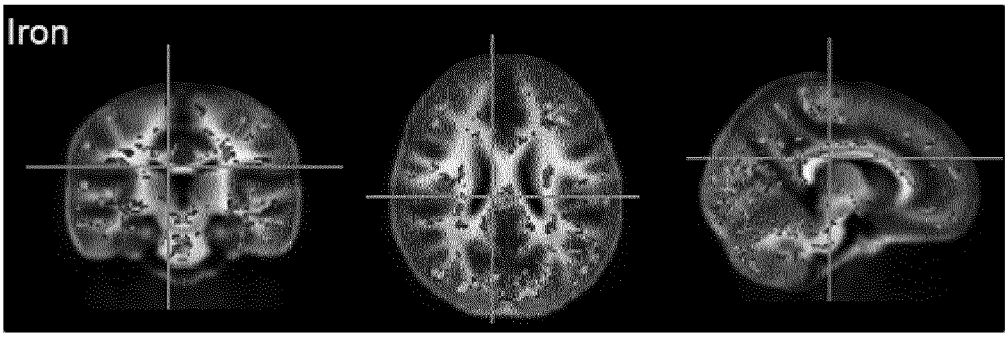


Fig.1f

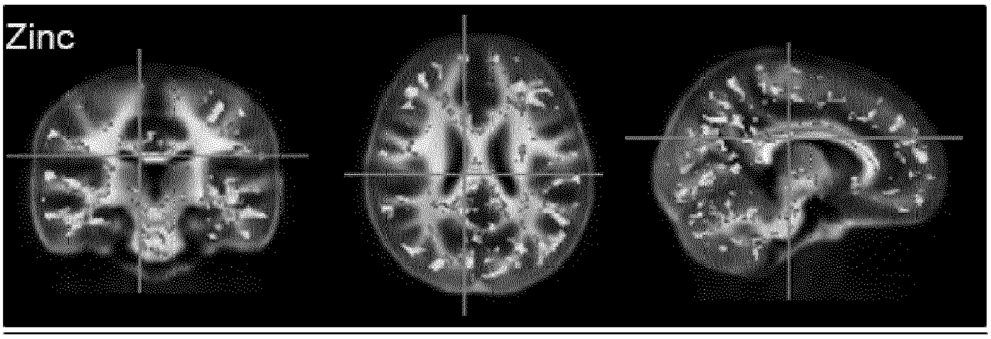


Fig.1g

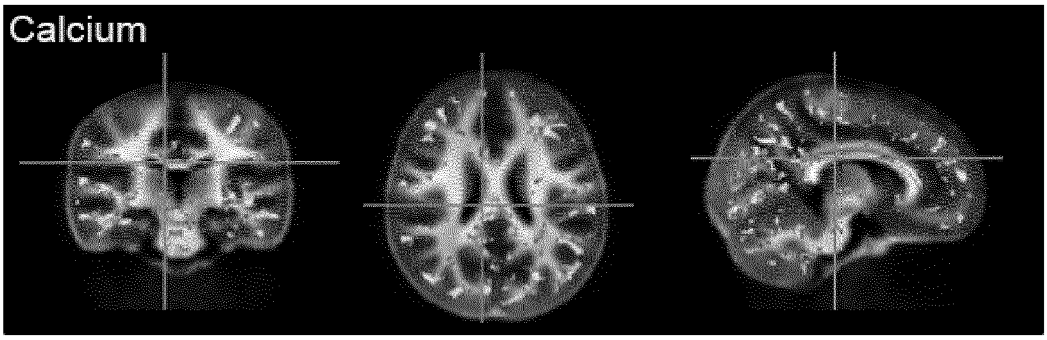


Fig.1h

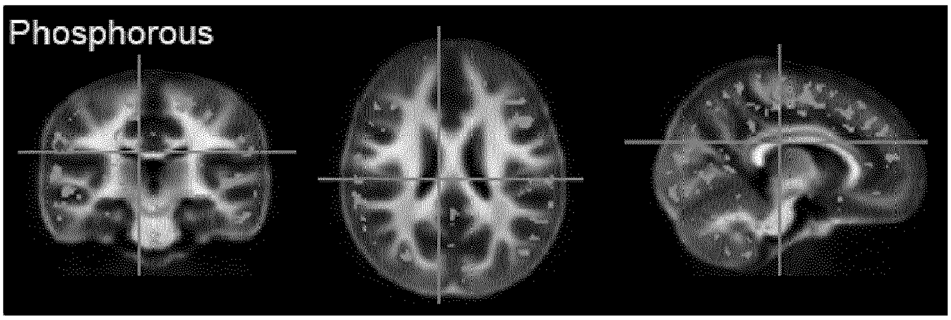

Fig.1I

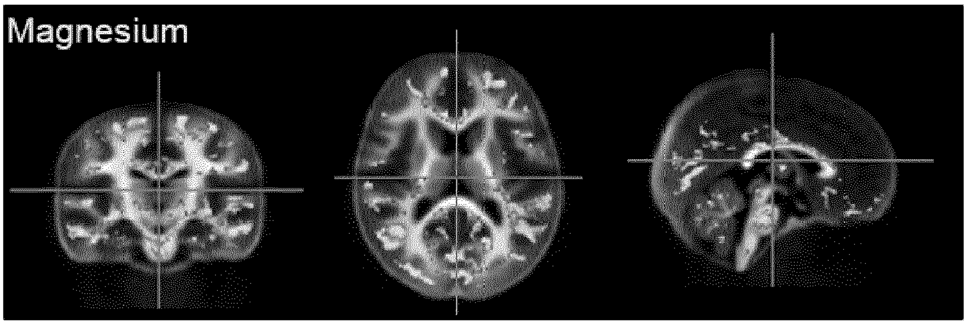


Fig.1J

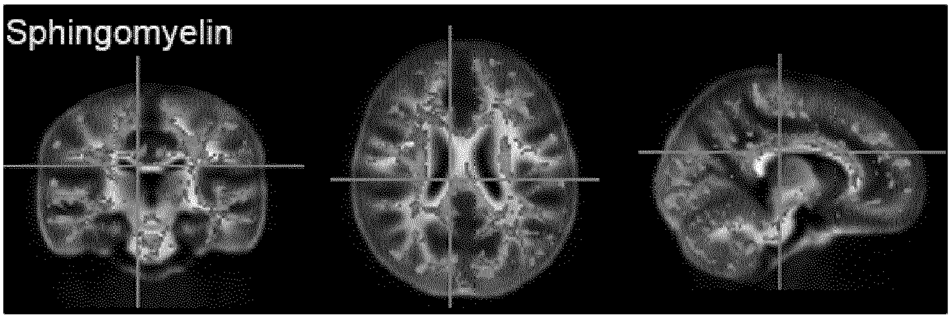


Fig.1K

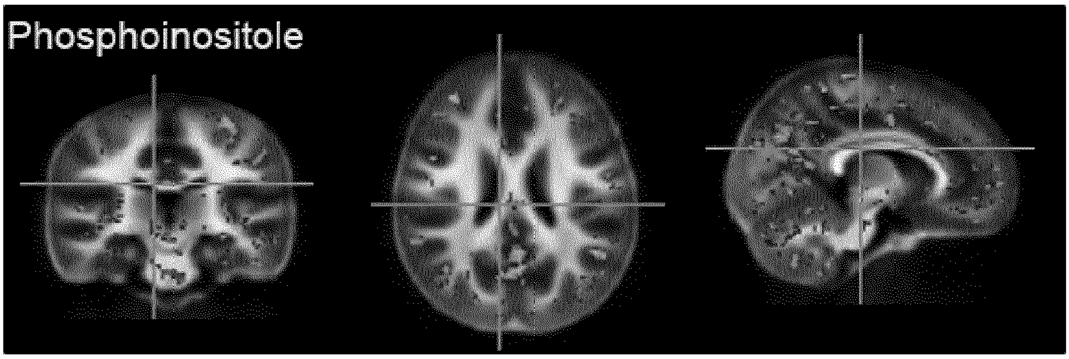


Fig.1L

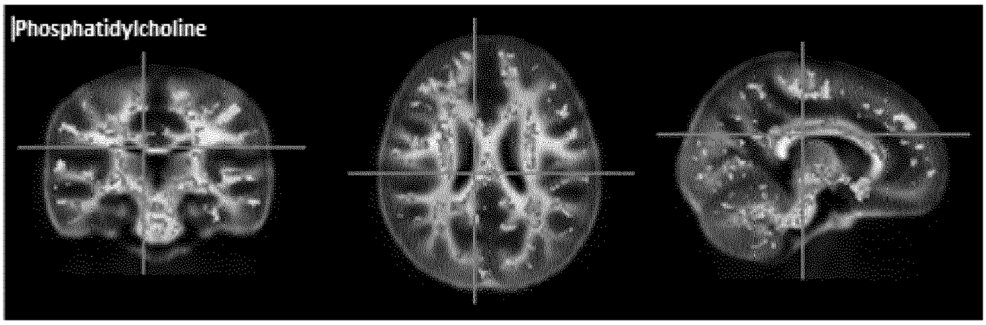


Fig.1M

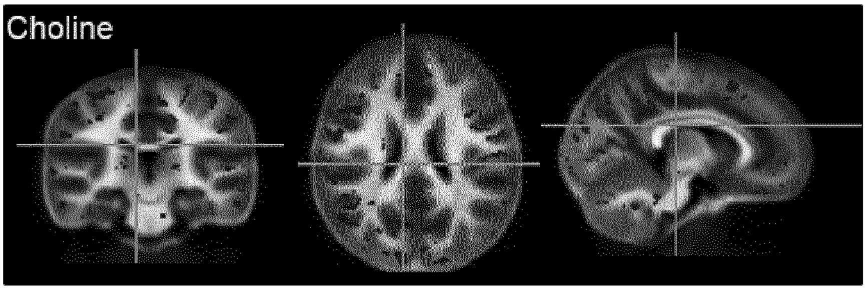


Fig.1n

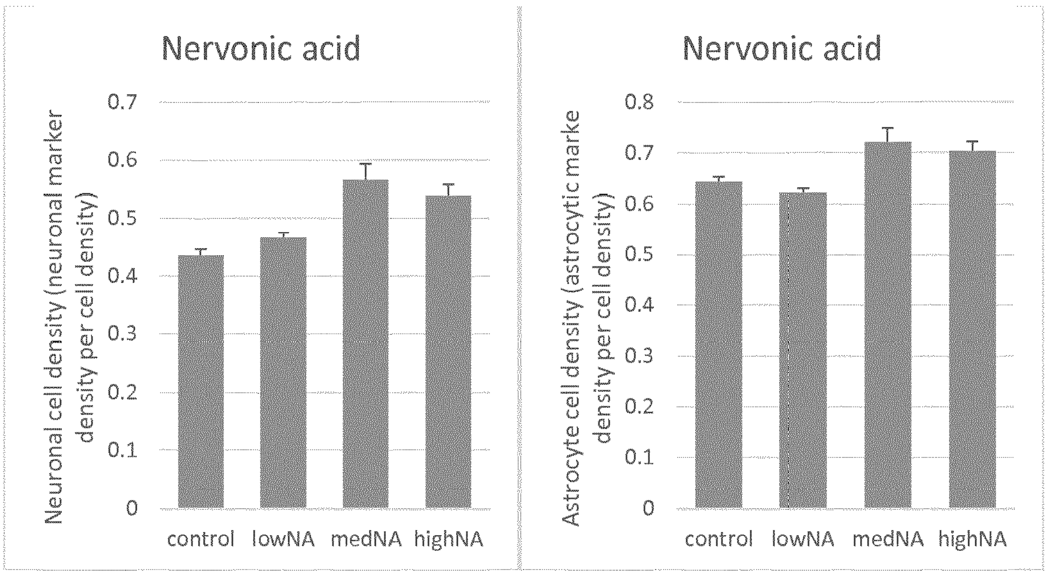


**Fig. 2**

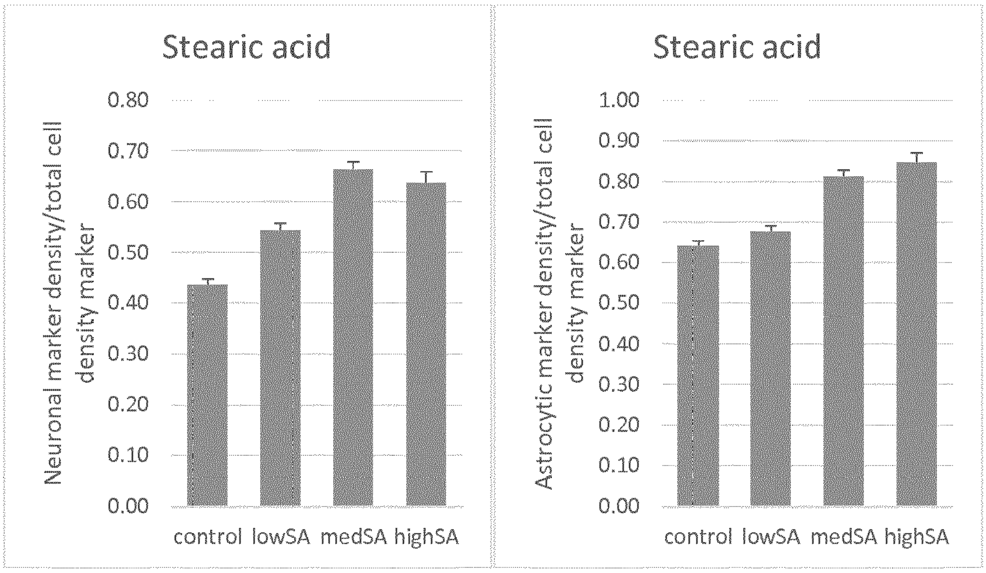
Stearic acid
Neuronal marker density/total cell density marker
0.80
0.70
0.60
0.50
0.40
0.30
0.20
0.10
0.00
control lowSA medSA highSA
Stearic acid
Astrocytic marker density/total cell density marker
1.00
0.90
0.80
0.70
0.60
0.50
0.40
0.30
0.20
0.10
0.00
control lowSA medSA highSA

**Fig. 3**

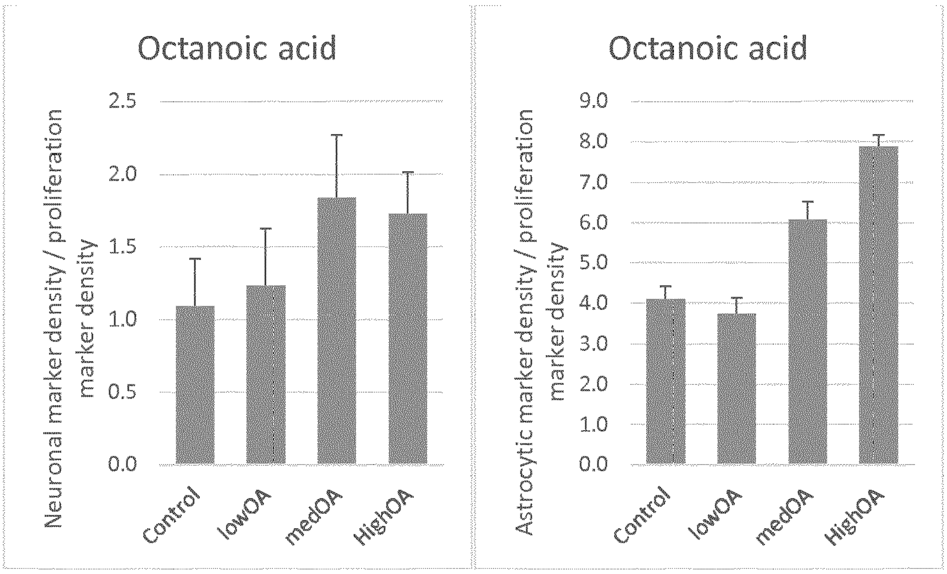
Octanoic acid
Neuronal marker density / proliferation marker density
2.5
2.0
1.5
1.0
0.5
0.0
Control
lowOA
medOA
HighOA
Octanoic acid
Astrocytic marker density / proliferation marker density
9.0
8.0
7.0
6.0
5.0
4.0
3.0
2.0
1.0
0.0
Control
lowOA
medOA
HighOA

**Fig. 4**

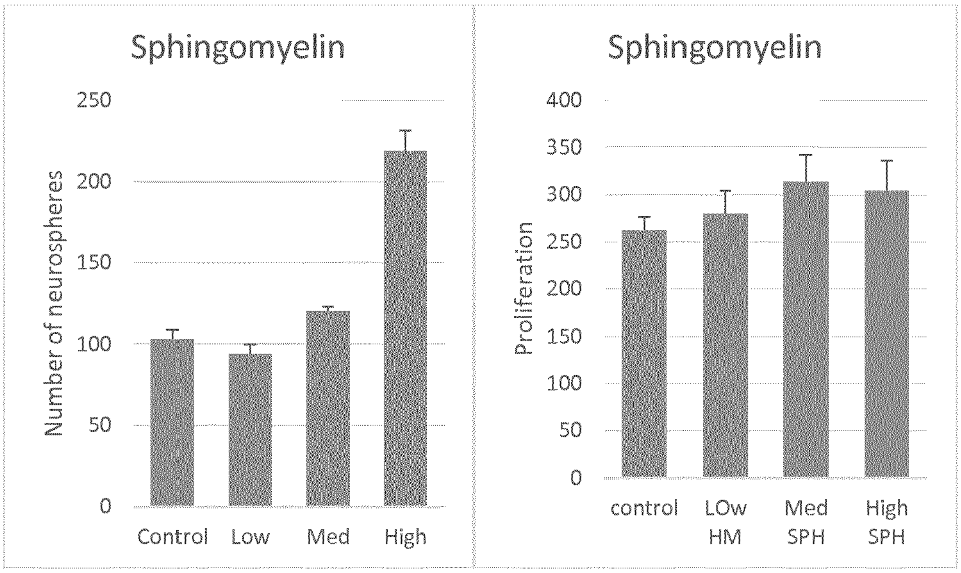
Sphingomyelin
Number of neurospheres
250
200
150
100
50
0
Control
Low
Med
High
Sphingomyelin
Proliferation
400
350
300
250
200
150
100
50
0
control
LOw HM
Med SPH
High SPH

Fig.5

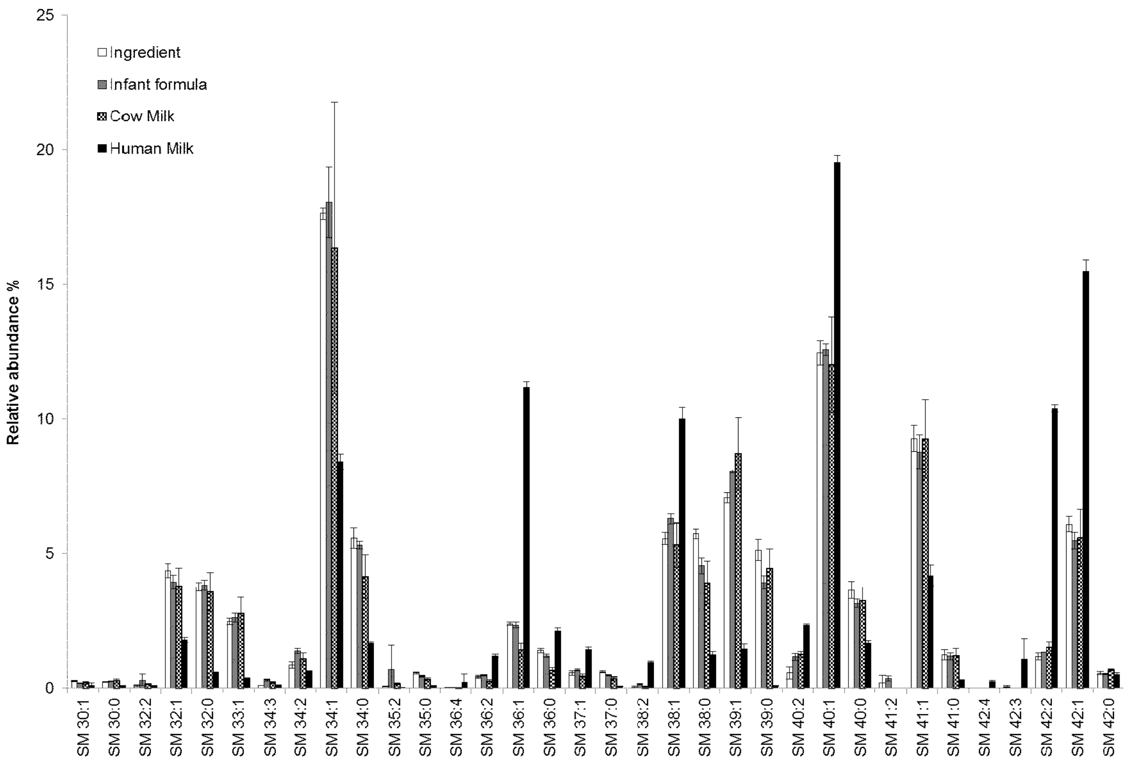
Ingredient
Infant formula
Cow Milk
Human Milk
Relative abundance %
25
20
15
10
5
0
SM 30:1
SM 30:0
SM 32:2
SM 32:1
SM 32:0
SM 33:1
SM 34:3
SM 34:2
SM 34:1
SM 34:0
SM 35:2
SM 35:0
SM 36:4
SM 36:2
SM 36:1
SM 36:0
SM 37:1
SM 37:0
SM 38:2
SM 38:1
SM 38:0
SM 39:1
SM 39:0
SM 40:2
SM 40:1
SM 40:0
SM 41:2
SM 41:1
SM 41:0
SM 42:4
SM 42:3
SM 42:2
SM 42:1
SM 42:0

Fig. 6

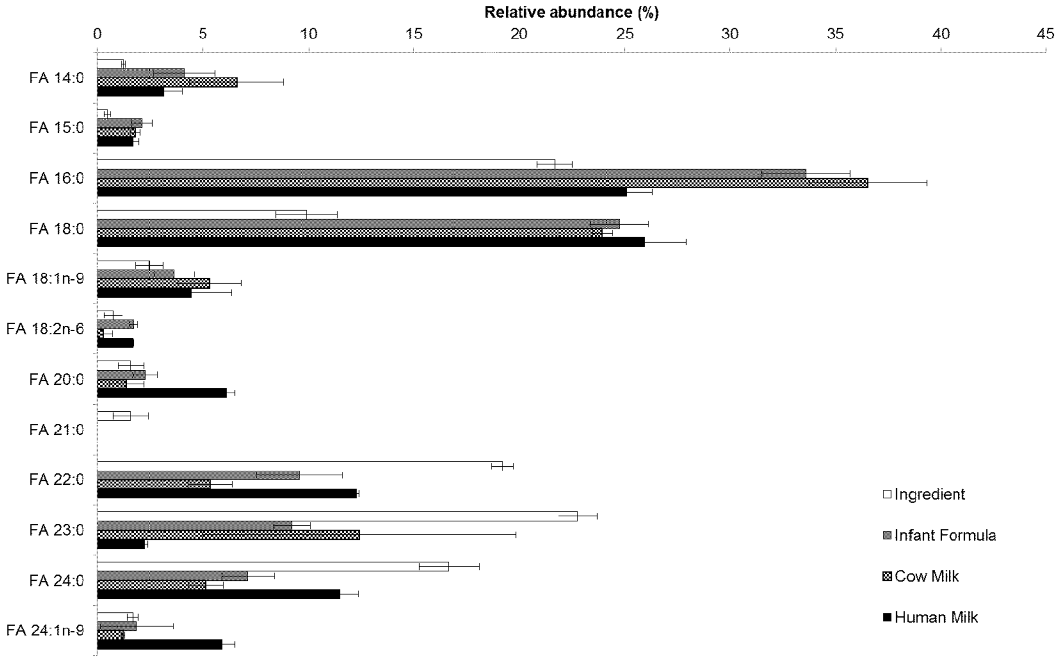
Relative abundance (%)
0
5
10
15
20
25
30
35
40
45
FA 14:0
FA 15:0
FA 16:0
FA 18:0
FA 18:1n-9
FA 18:2n-6
FA 20:0
FA 21:0
FA 22:0
FA 23:0
FA 24:0
FA 24:1n-9
Ingredient
Infant Formula
Cow Milk
Human Milk

Fig. 7

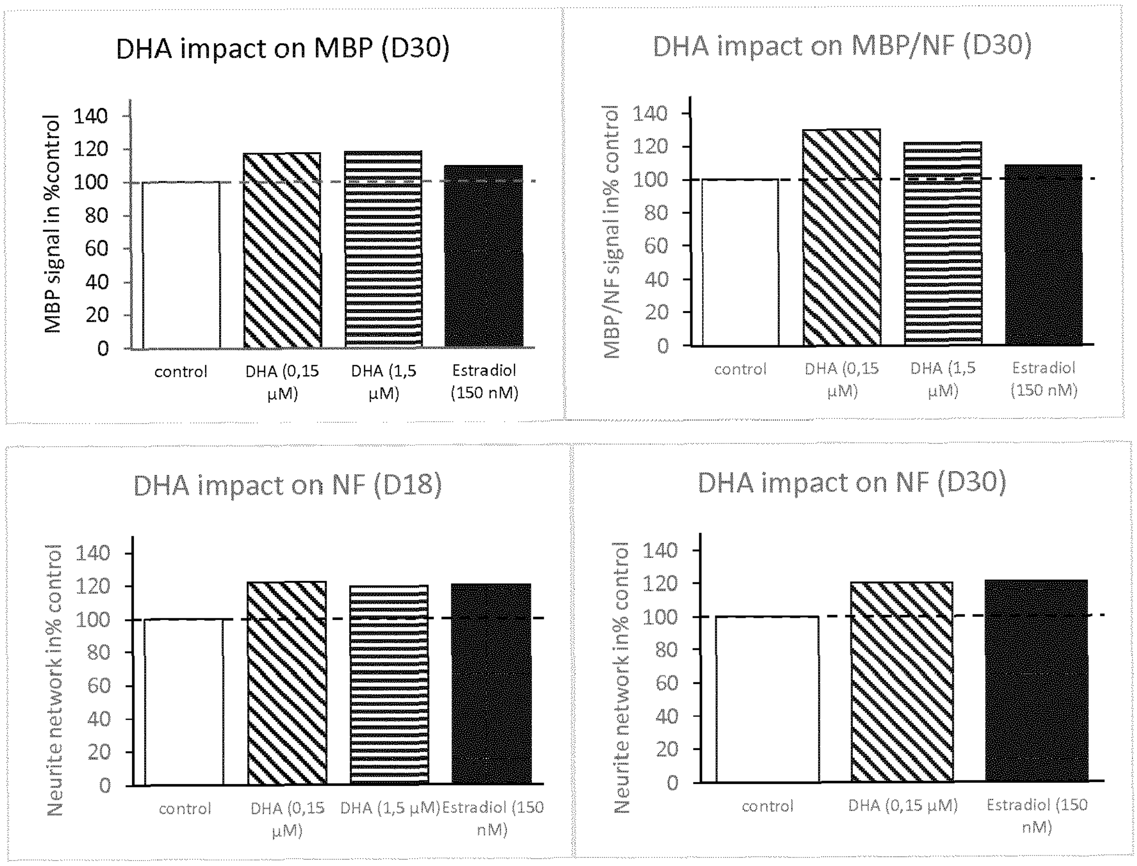
DHA impact on MBP (D30)
MBP signal in %control
140
120
100
80
60
40
20
0
control
DHA (0,15 µM)
DHA (1,5 µM)
Estradiol (150 nM)
DHA impact on MBP/NF (D30)
MBP/NF signal in% control
140
120
100
80
60
40
20
0
control
DHA (0,15 µM)
DHA (1,5 µM)
Estradiol (150 nM)
DHA impact on NF (D18)
Neurite network in% control
140
120
100
80
60
40
20
0
control
DHA (0,15 µM)
DHA (1,5 µM)
Estradiol (150 nM)
DHA impact on NF (D30)
Neurite network in% control
140
120
100
80
60
40
20
0
control
DHA (0,15 µM)
Estradiol (150 nM)

Fig.8

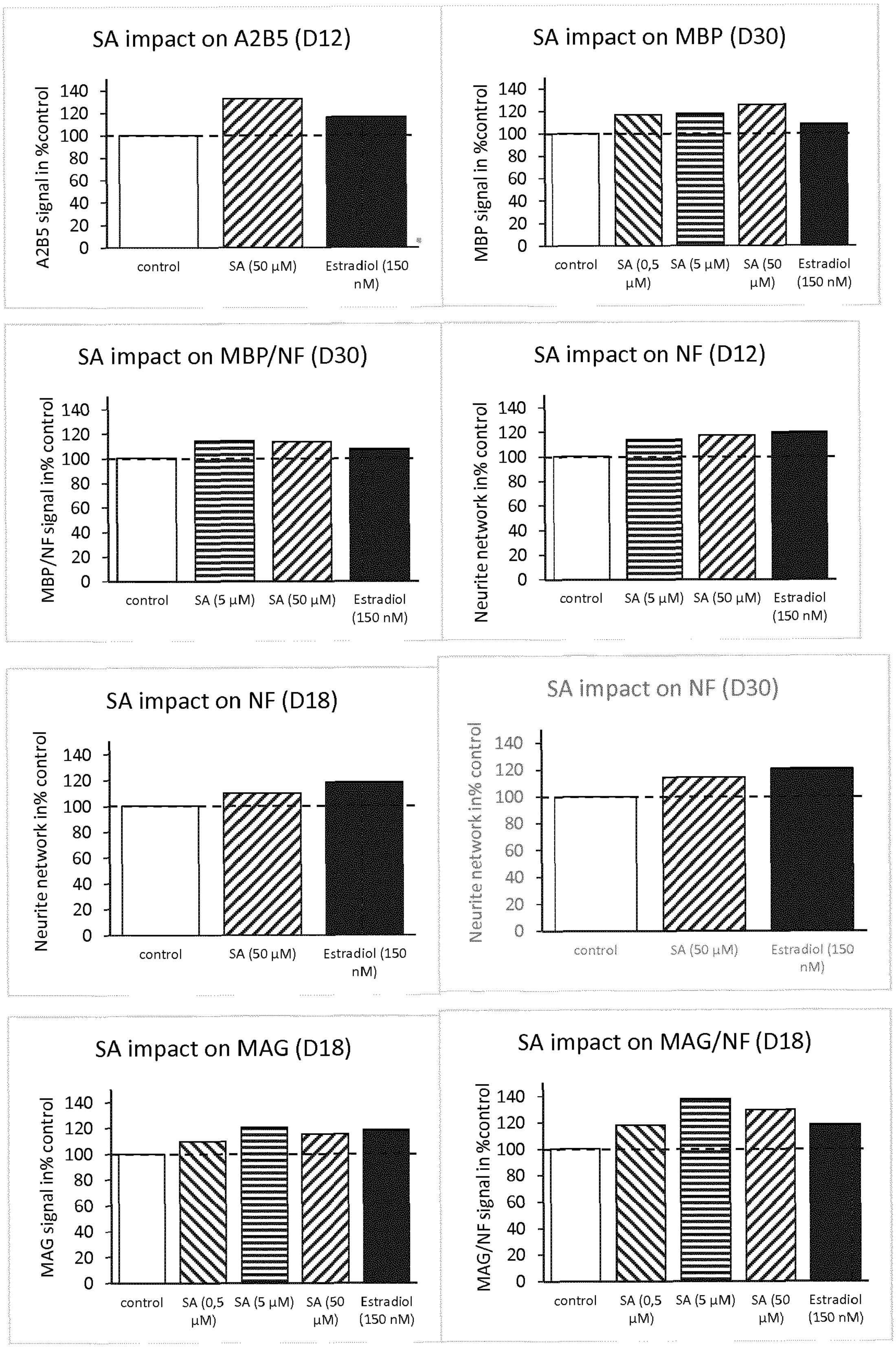
SA impact on A2B5 (D12)
A2B5 signal in %control
140
120
100
80
60
40
20
0
control
SA (50 µM)
Estradiol (150 nM)
SA impact on MBP (D30)
MBP signal in %control
140
120
100
80
60
40
20
0
control
SA (0,5 µM)
SA (5 µM)
SA (50 µM)
Estradiol (150 nM)
SA impact on MBP/NF (D30)
MBP/NF signal in% control
140
120
100
80
60
40
20
0
control
SA (5 µM)
SA (50 µM)
Estradiol (150 nM)
SA impact on NF (D12)
Neurite network in% control
140
120
100
80
60
40
20
0
control
SA (5 µM)
SA (50 µM)
Estradiol (150 nM)
SA impact on NF (D18)
Neurite network in% control
140
120
100
80
60
40
20
0
control
SA (50 µM)
Estradiol (150 nM)
SA impact on NF (D30)
Neurite network in% control
140
120
100
80
60
40
20
0
control
SA (50 µM)
Estradiol (150 nM)
SA impact on MAG (D18)
MAG signal in% control
140
120
100
80
60
40
20
0
control
SA (0,5 µM)
SA (5 µM)
SA (50 µM)
Estradiol (150 nM)
SA impact on MAG/NF (D18)
MAG/NF signal in %control
140
120
100
80
60
40
20
0
control
SA (0,5 µM)
SA (5 µM)
SA (50 µM)
Estradiol (150 nM)

Fig.9

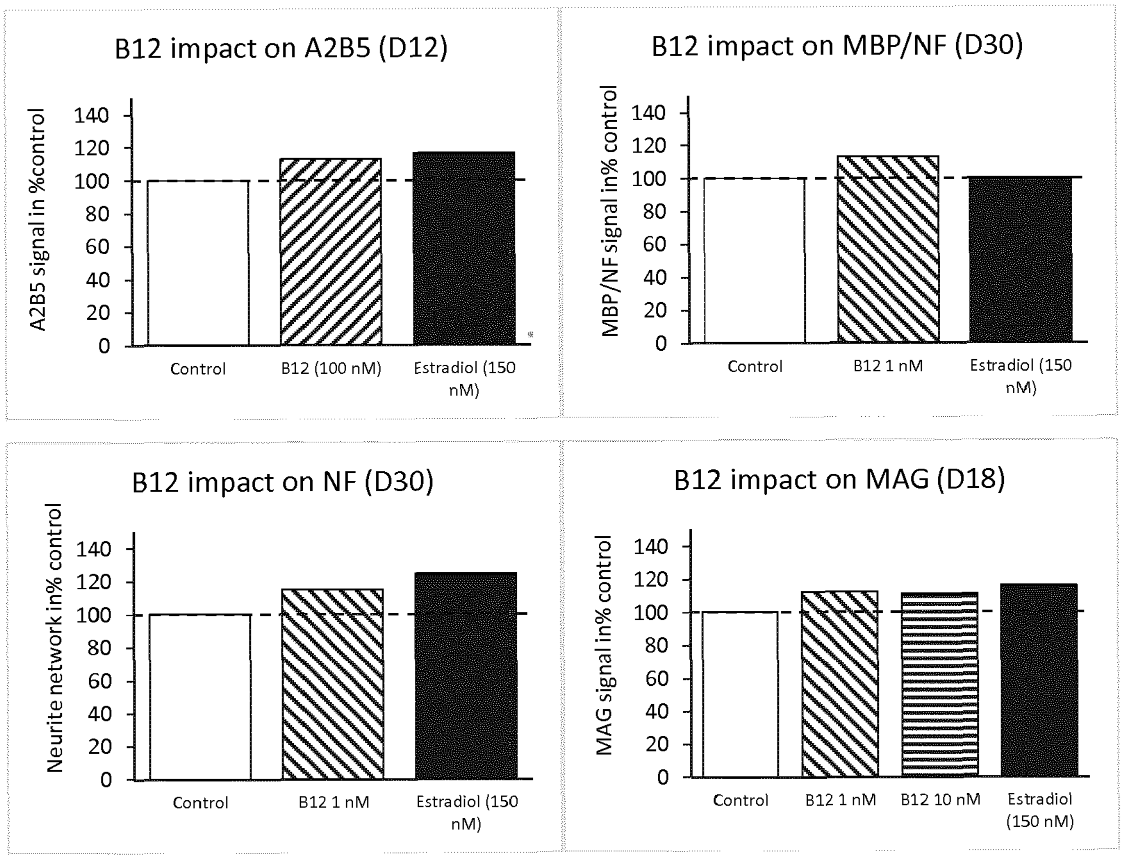
B12 impact on A2B5 (D12)
A2B5 signal in %control
140
120
100
80
60
40
20
0
Control
B12 (100 nM)
Estradiol (150 nM)
B12 impact on MBP/NF (D30)
MBP/NF signal in% control
140
120
100
80
60
40
20
0
Control
B12 1 nM
Estradiol (150 nM)
B12 impact on NF (D30)
Neurite network in% control
140
120
100
80
60
40
20
0
Control
B12 1 nM
Estradiol (150 nM)
B12 impact on MAG (D18)
MAG signal in% control
140
120
100
80
60
40
20
0
Control
B12 1 nM
B12 10 nM
Estradiol (150 nM)

Fig.10

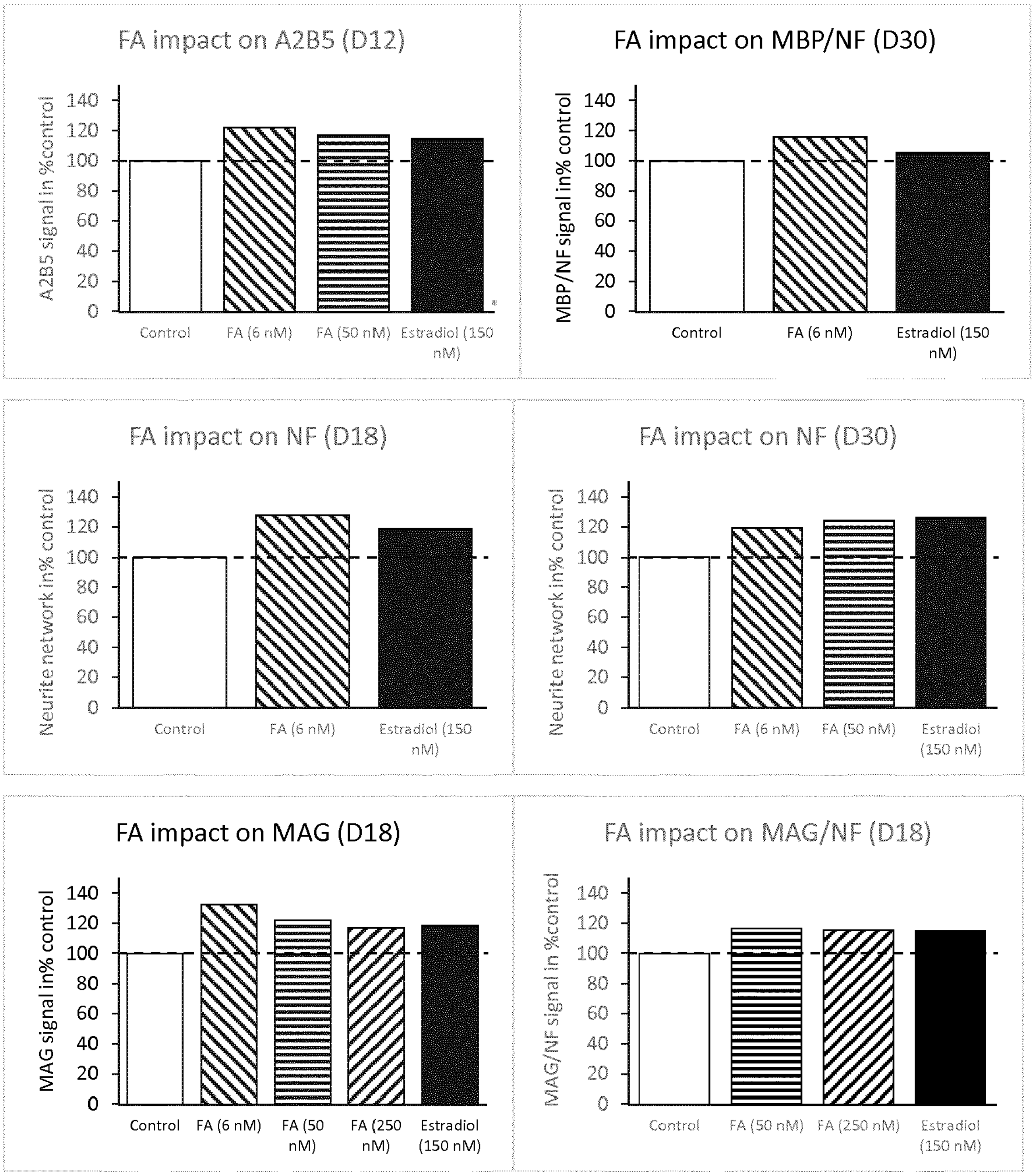
FA impact on A2B5 (D12)
A2B5 signal in %control
140
120
100
80
60
40
20
0
Control
FA (6 nM)
FA (50 nM)
Estradiol (150 nM)
FA impact on MBP/NF (D30)
MBP/NF signal in% control
Control
FA (6 nM)
Estradiol (150 nM)
FA impact on NF (D18)
Neurite network in% control
Control
FA (6 nM)
Estradiol (150 nM)
FA impact on NF (D30)
Neurite network in% control
Control
FA (6 nM)
FA (50 nM)
Estradiol (150 nM)
FA impact on MAG (D18)
MAG signal in% control
Control
FA (6 nM)
FA (50 nM)
FA (250 nM)
Estradiol (150 nM)
FA impact on MAG/NF (D18)
MAG/NF signal in %control
Control
FA (50 nM)
FA (250 nM)
Estradiol (150 nM)

Fig.11

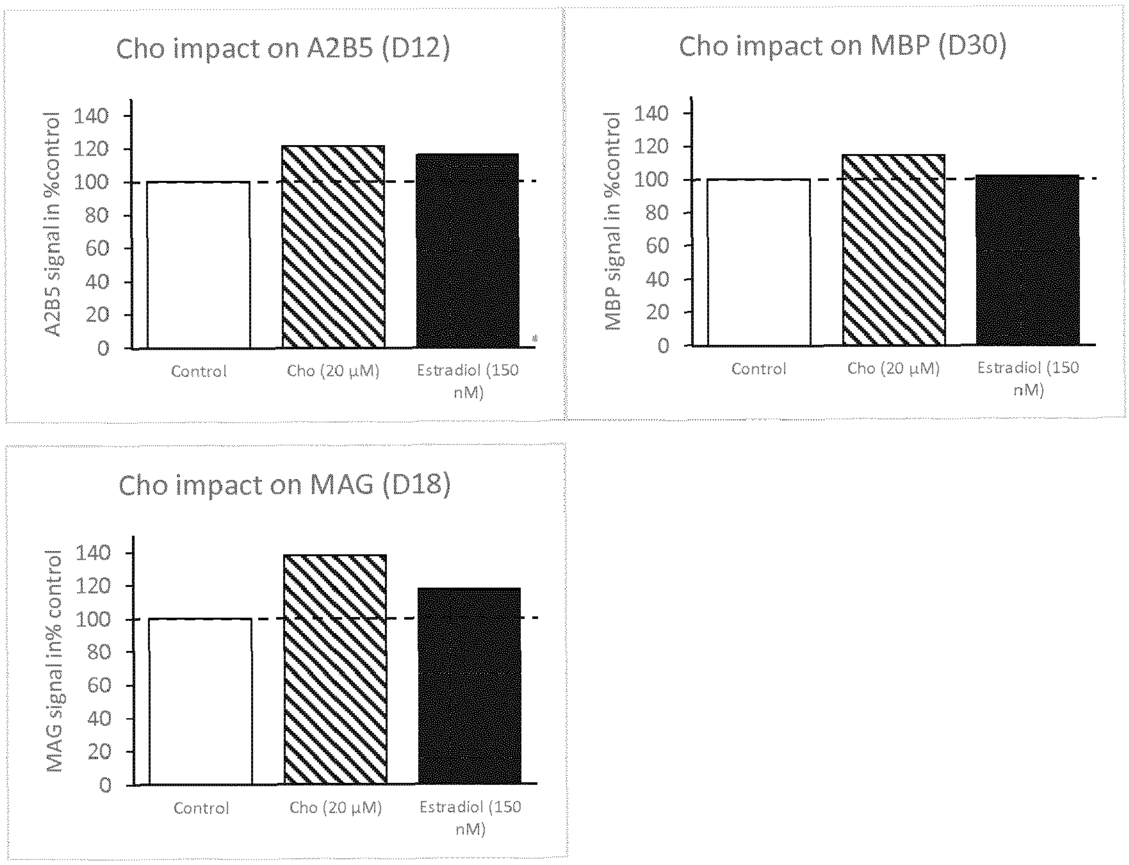
Cho impact on A2B5 (D12)
A2B5 signal in %control
140
120
100
80
60
40
20
0
Control
Cho (20 µM)
Estradiol (150 nM)
Cho impact on MBP (D30)
MBP signal in %control
140
120
100
80
60
40
20
0
Control
Cho (20 µM)
Estradiol (150 nM)
Cho impact on MAG (D18)
MAG signal in% control
140
120
100
80
60
40
20
0
Control
Cho (20 µM)
Estradiol (150 nM)

Fig.12

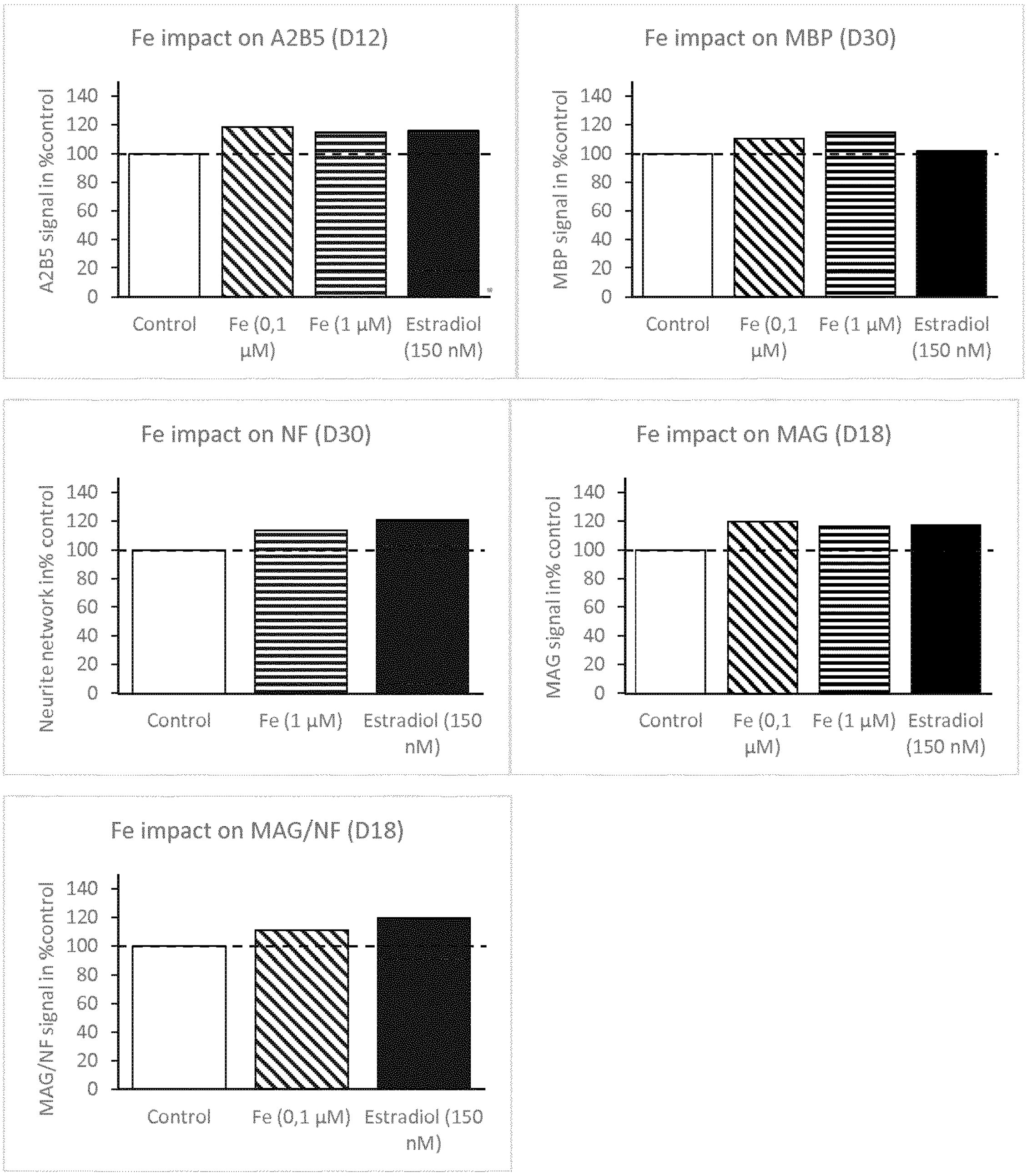

Fe impact on A2B5 (D12)
A2B5 signal in %control
140
120
100
80
60
40
20
0
Control
Fe (0,1 µM)
Fe (1 µM)
Estradiol (150 nM)
Fe impact on MBP (D30)
MBP signal in %control
140
120
100
80
60
40
20
0
Control
Fe (0,1 µM)
Fe (1 µM)
Estradiol (150 nM)
Fe impact on NF (D30)
Neurite network in% control
140
120
100
80
60
40
20
0
Control
Fe (1 µM)
Estradiol (150 nM)
Fe impact on MAG (D18)
MAG signal in% control
140
120
100
80
60
40
20
0
Control
Fe (0,1 µM)
Fe (1 µM)
Estradiol (150 nM)
Fe impact on MAG/NF (D18)
MAG/NF signal in %control
140
120
100
80
60
40
20
0
Control
Fe (0,1 µM)
Estradiol (150 nM)


Fig.13

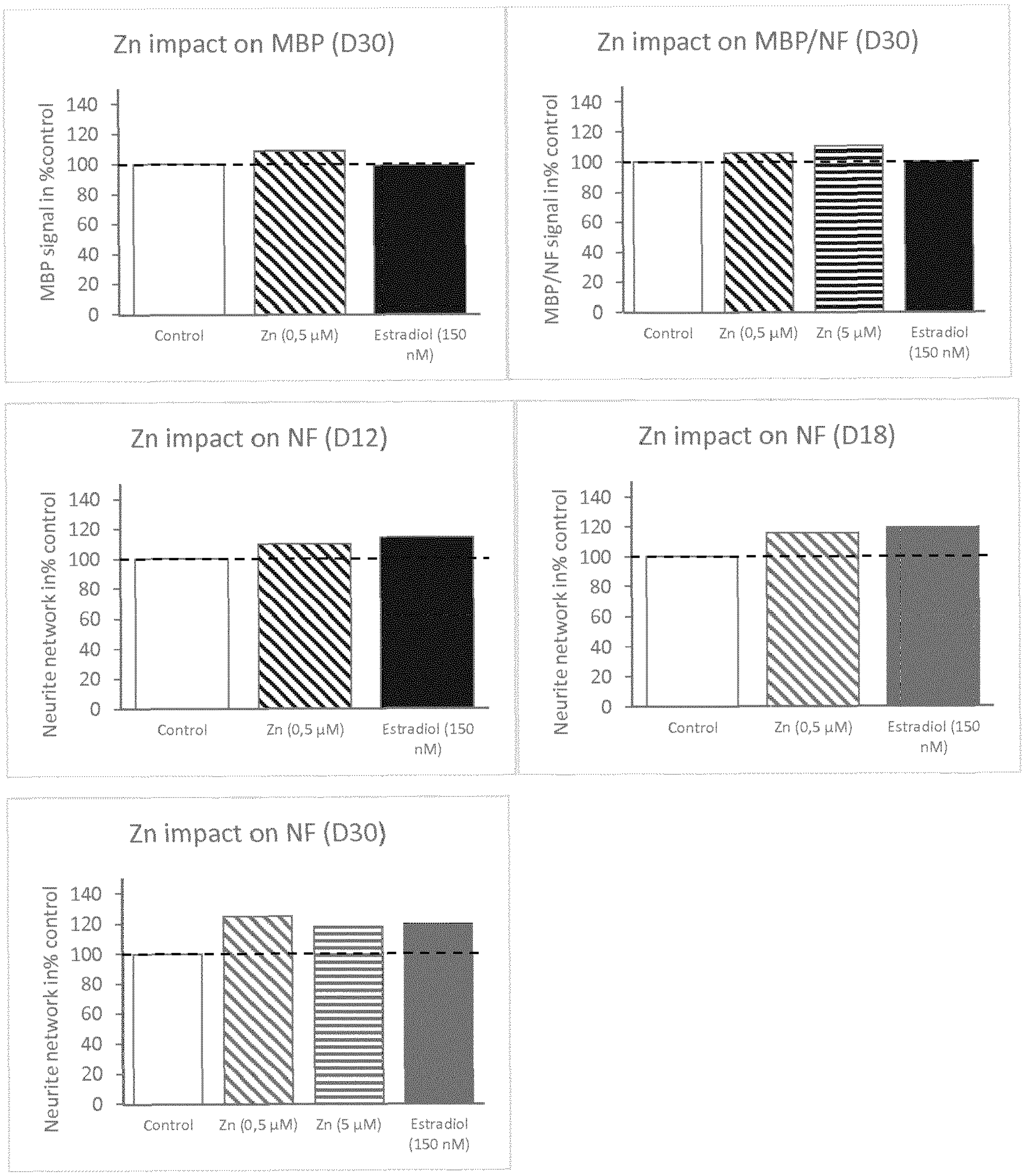
Zn impact on MBP (D30)
MBP signal in %control
140
120
100
80
60
40
20
0
Control
Zn (0,5 µM)
Estradiol (150 nM)
Zn impact on MBP/NF (D30)
MBP/NF signal in% control
140
120
100
80
60
40
20
0
Control
Zn (0,5 µM)
Zn (5 µM)
Estradiol (150 nM)
Zn impact on NF (D12)
Neurite network in% control
140
120
100
80
60
40
20
0
Control
Zn (0,5 µM)
Estradiol (150 nM)
Zn impact on NF (D18)
Neurite network in% control
140
120
100
80
60
40
20
0
Control
Zn (0,5 µM)
Estradiol (150 nM)
Zn impact on NF (D30)
Neurite network in% control
140
120
100
80
60
40
20
0
Control
Zn (0,5 µM)
Zn (5 µM)
Estradiol (150 nM)

Fig.14

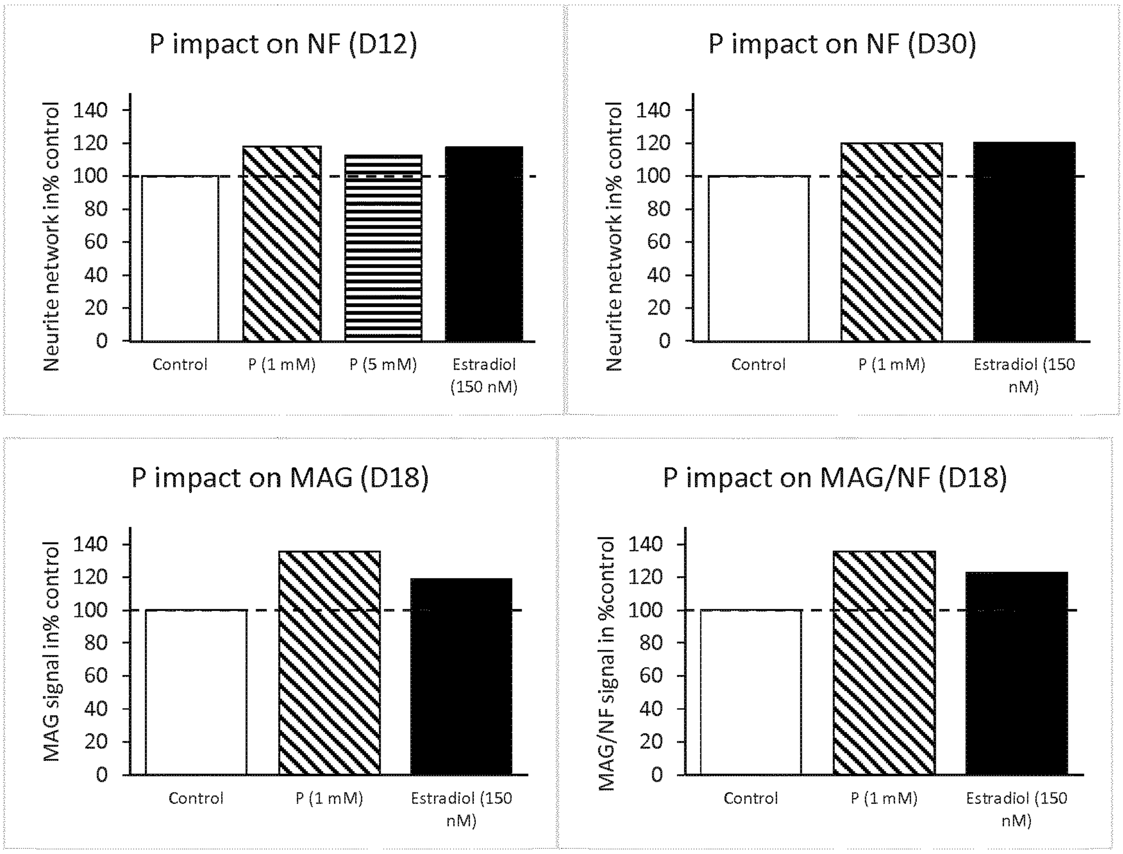
P impact on NF (D12)
Neurite network in% control
140
120
100
80
60
40
20
0
Control
P (1 mM)
P (5 mM)
Estradiol (150 nM)
P impact on NF (D30)
Neurite network in% control
140
120
100
80
60
40
20
0
Control
P (1 mM)
Estradiol (150 nM)
P impact on MAG (D18)
MAG signal in% control
140
120
100
80
60
40
20
0
Control
P (1 mM)
Estradiol (150 nM)
P impact on MAG/NF (D18)
MAG/NF signal in %control
140
120
100
80
60
40
20
0
Control
P (1 mM)
Estradiol (150 nM)

Fig.15

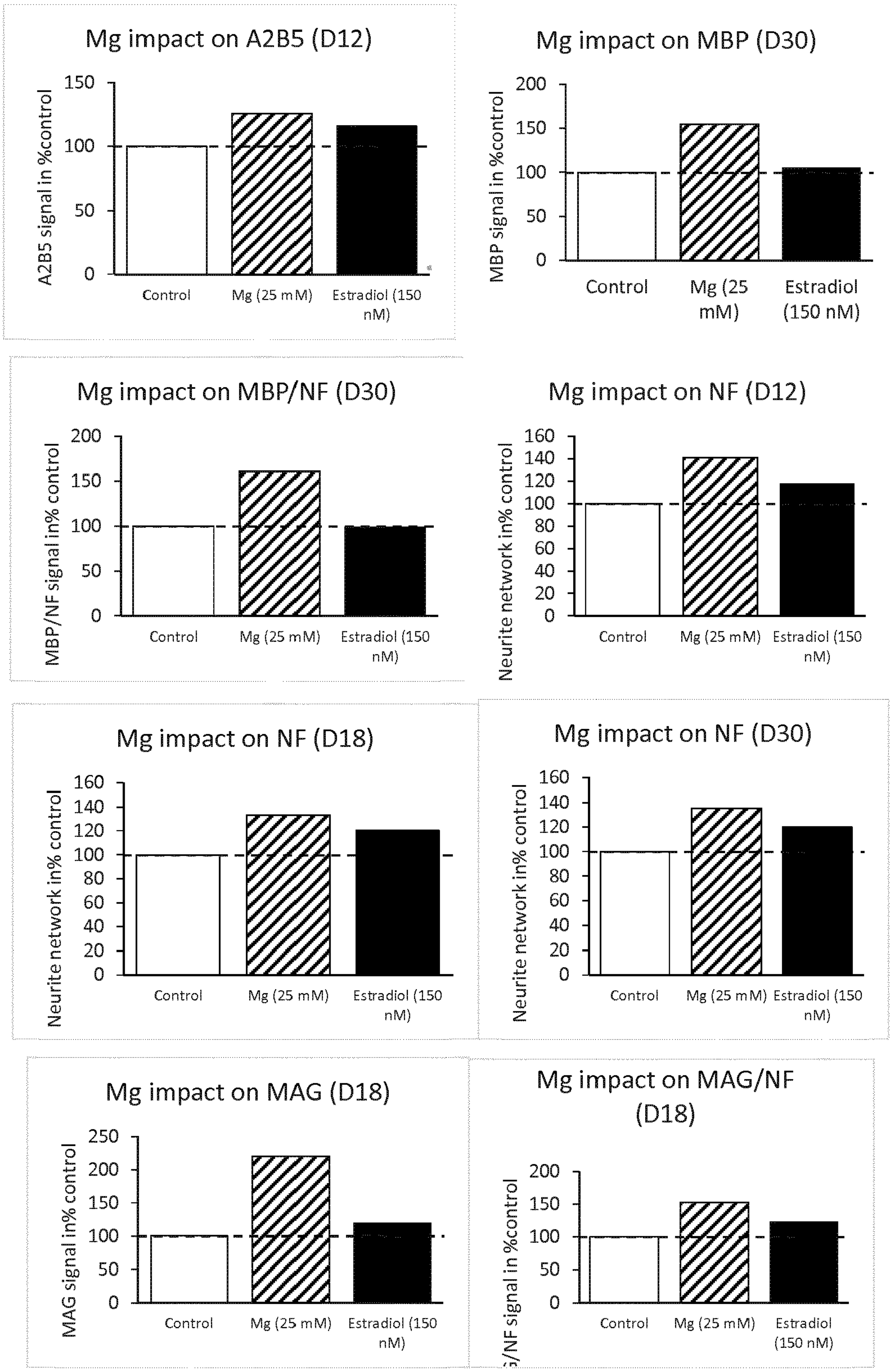
Mg impact on A2B5 (D12)
A2B5 signal in %control
150
100
50
0
Control
Mg (25 mM)
Estradiol (150 nM)
Mg impact on MBP (D30)
MBP signal in %control
200
150
100
50
0
Control
Mg (25 mM)
Estradiol (150 nM)
Mg impact on MBP/NF (D30)
MBP/NF signal in% control
200
150
100
50
0
Control
Mg (25 mM)
Estradiol (150 nM)
Mg impact on NF (D12)
Neurite network in% control
160
140
120
100
80
60
40
20
0
Control
Mg (25 mM)
Estradiol (150 nM)
Mg impact on NF (D18)
Neurite network in% control
160
140
120
100
80
60
40
20
0
Control
Mg (25 mM)
Estradiol (150 nM)
Mg impact on NF (D30)
Neurite network in% control
160
140
120
100
80
60
40
20
0
Control
Mg (25 mM)
Estradiol (150 nM)
Mg impact on MAG (D18)
MAG signal in% control
250
200
150
100
50
0
Control
Mg (25 mM)
Estradiol (150 nM)
Mg impact on MAG/NF (D18)
/NF signal in %control
200
150
100
50
0
Control
Mg (25 mM)
Estradiol (150 nM)

Fig.16

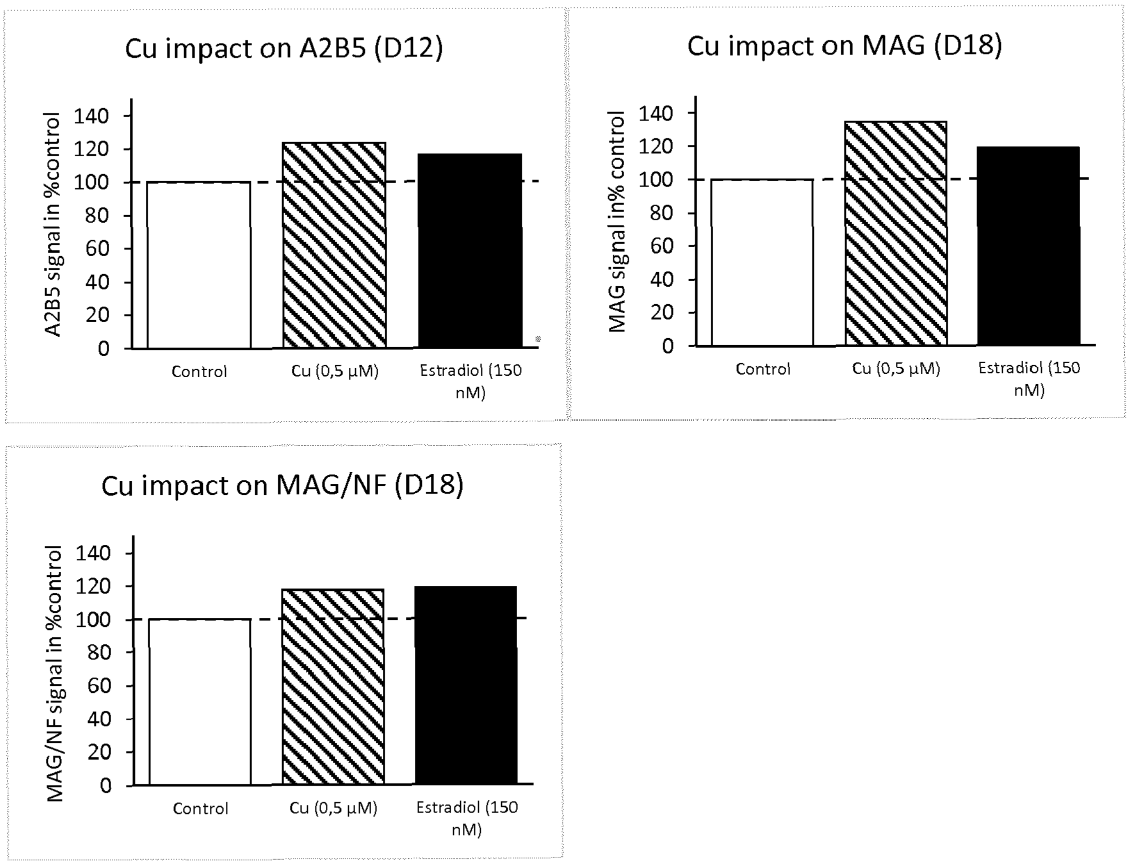
Cu impact on A2B5 (D12)
A2B5 signal in %control
140
120
100
80
60
40
20
0
Control
Cu (0,5 µM)
Estradiol (150 nM)
Cu impact on MAG (D18)
MAG signal in% control
140
120
100
80
60
40
20
0
Control
Cu (0,5 µM)
Estradiol (150 nM)
Cu impact on MAG/NF (D18)
MAG/NF signal in %control
140
120
100
80
60
40
20
0
Control
Cu (0,5 µM)
Estradiol (150 nM)

Fig.17

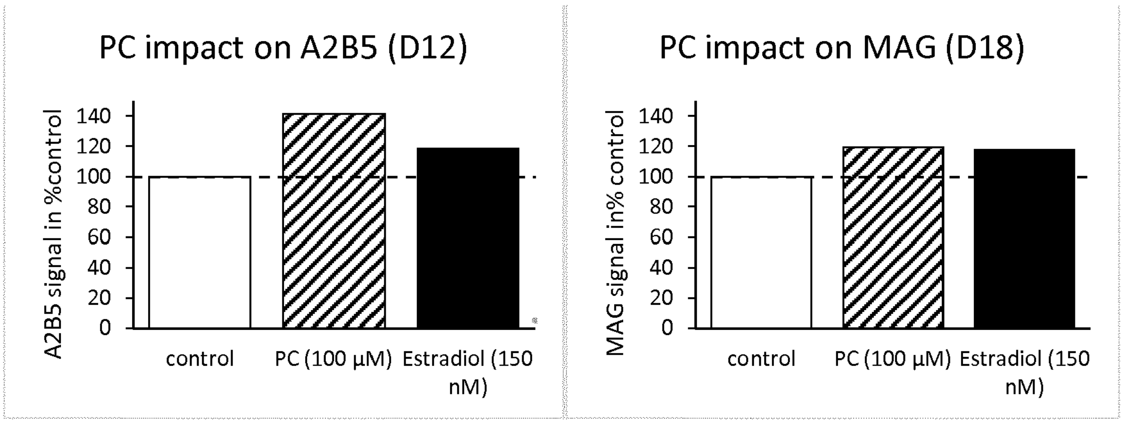
PC impact on A2B5 (D12)
A2B5 signal in %control
140
120
100
80
60
40
20
0
control
PC (100 µM)
Estradiol (150 nM)
PC impact on MAG (D18)
MAG signal in% control
140
120
100
80
60
40
20
0
control
PC (100 µM)
Estradiol (150 nM)

Fig.18

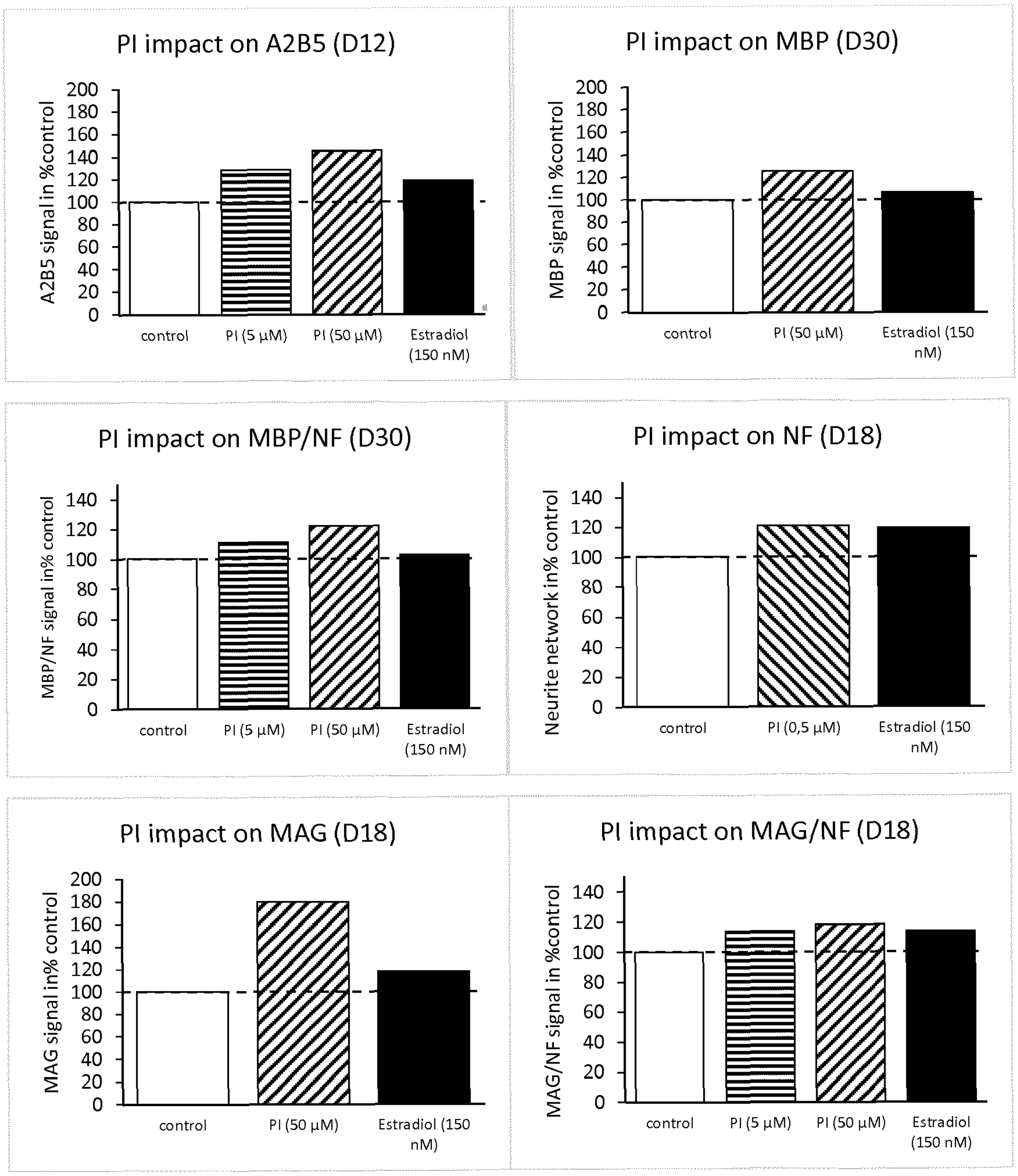
PI impact on A2B5 (D12)
A2B5 signal in %control
200
180
160
140
120
100
80
60
40
20
0
control
PI (5 µM)
PI (50 µM)
Estradiol (150 nM)
PI impact on MBP (D30)
MBP signal in %control
200
180
160
140
120
100
80
60
40
20
0
control
PI (50 µM)
Estradiol (150 nM)
PI impact on MBP/NF (D30)
MBP/NF signal in% control
140
120
100
80
60
40
20
0
control
PI (5 µM)
PI (50 µM)
Estradiol (150 nM)
PI impact on NF (D18)
Neurite network in% control
140
120
100
80
60
40
20
0
control
PI (0,5 µM)
Estradiol (150 nM)
PI impact on MAG (D18)
MAG signal in% control
200
180
160
140
120
100
80
60
40
20
0
control
PI (50 µM)
Estradiol (150 nM)
PI impact on MAG/NF (D18)
MAG/NF signal in %control
140
120
100
80
60
40
20
0
control
PI (5 µM)
PI (50 µM)
Estradiol (150 nM)

Fig.19

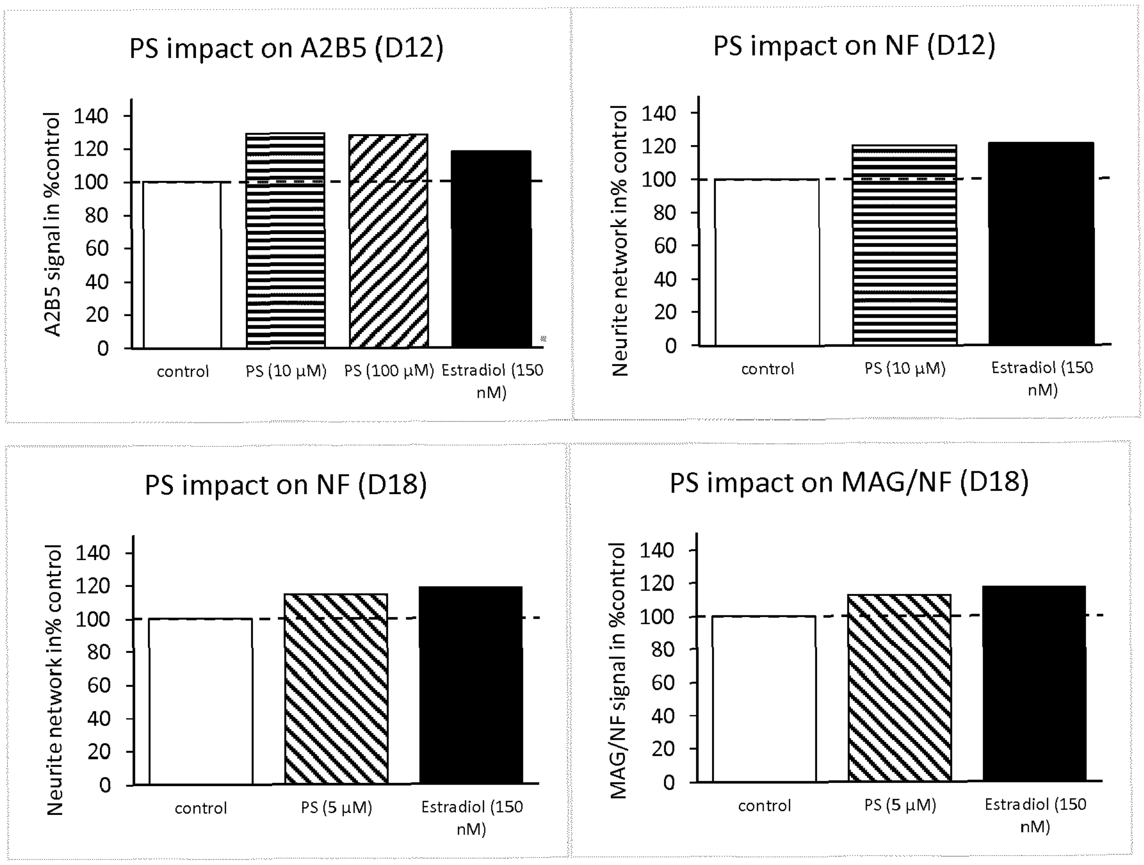

PS impact on A2B5 (D12)
A2B5 signal in %control
140
120
100
80
60
40
20
0
control
PS (10 µM)
PS (100 µM)
Estradiol (150 nM)
PS impact on NF (D12)
Neurite network in% control
140
120
100
80
60
40
20
0
control
PS (10 µM)
Estradiol (150 nM)
PS impact on NF (D18)
Neurite network in% control
140
120
100
80
60
40
20
0
control
PS (5 µM)
Estradiol (150 nM)
PS impact on MAG/NF (D18)
MAG/NF signal in %control
140
120
100
80
60
40
20
0
control
PS (5 µM)
Estradiol (150 nM)


Fig.20

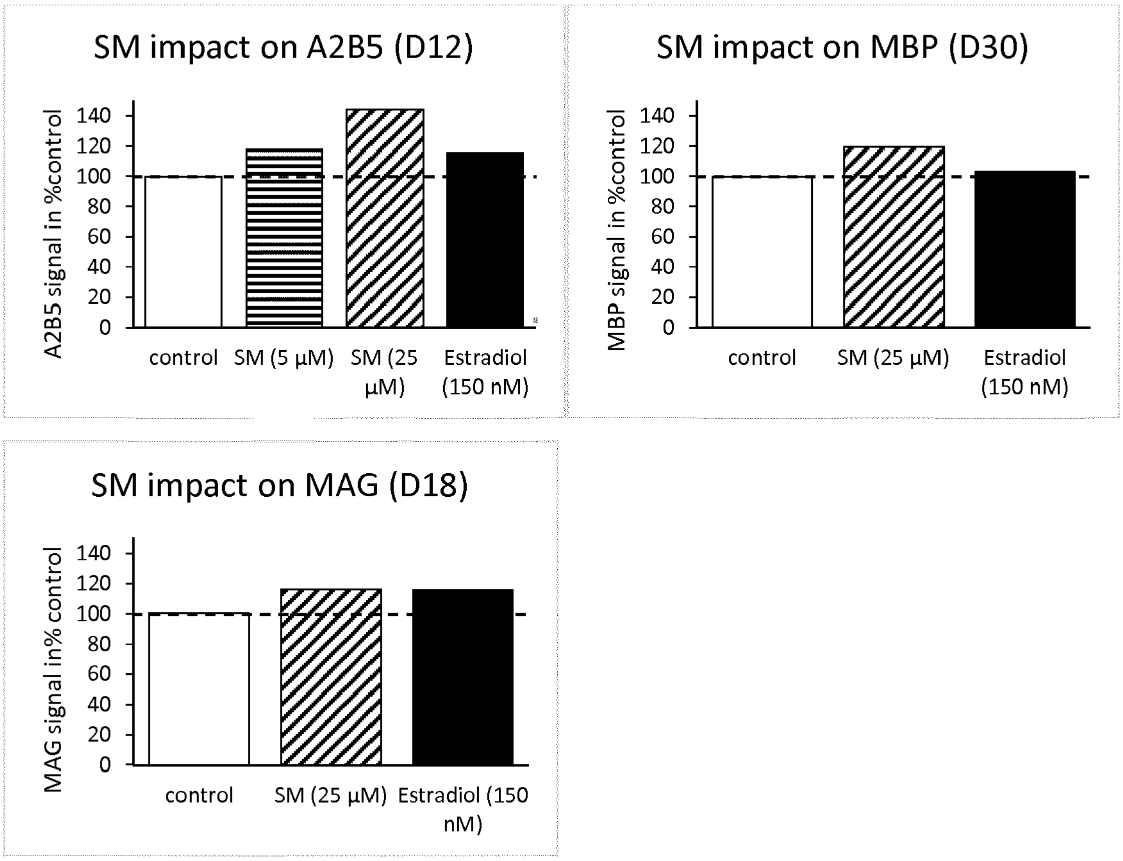
SM impact on A2B5 (D12)
A2B5 signal in %control
0
20
40
60
80
100
120
140
control
SM (5 µM)
SM (25 µM)
Estradiol (150 nM)
SM impact on MBP (D30)
MBP signal in %control
0
20
40
60
80
100
120
140
control
SM (25 µM)
Estradiol (150 nM)
SM impact on MAG (D18)
MAG signal in% control
0
20
40
60
80
100
120
140
control
SM (25 µM)
Estradiol (150 nM)

Fig.21

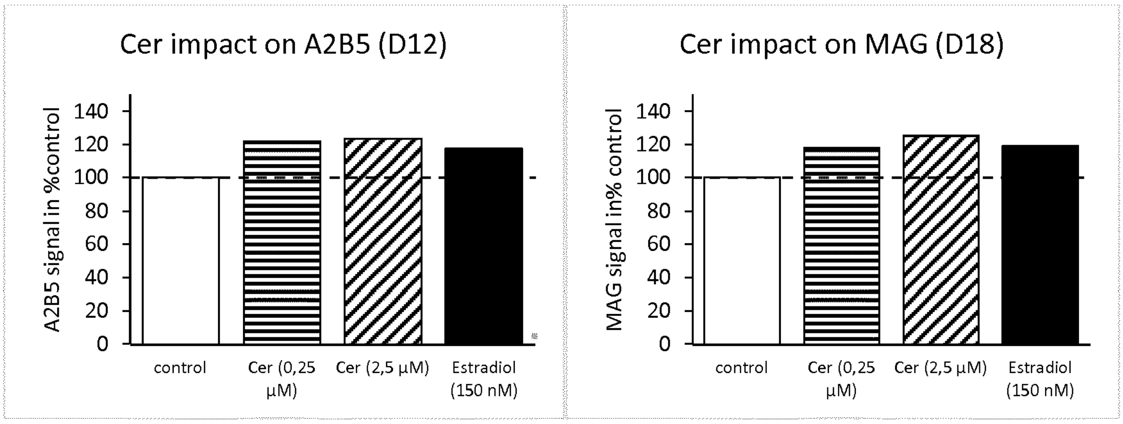

Cer impact on A2B5 (D12)
A2B5 signal in %control
140
120
100
80
60
40
20
0
control
Cer (0,25 µM)
Cer (2,5 µM)
Estradiol (150 nM)
Cer impact on MAG (D18)
MAG signal in% control
140
120
100
80
60
40
20
0
control
Cer (0,25 µM)
Cer (2,5 µM)
Estradiol (150 nM)


Fig.22

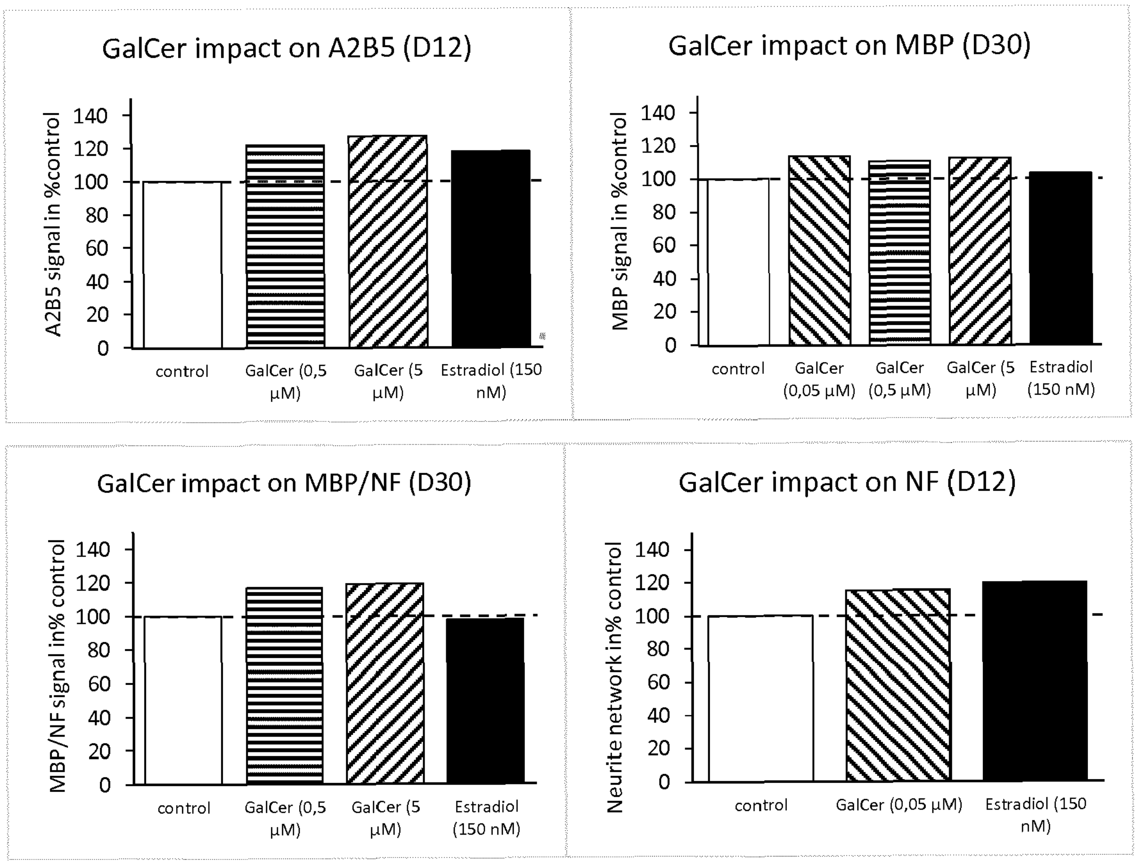
GalCer impact on A2B5 (D12)
A2B5 signal in %control
140
120
100
80
60
40
20
0
control
GalCer (0,5 µM)
GalCer (5 µM)
Estradiol (150 nM)
GalCer impact on MBP (D30)
MBP signal in %control
140
120
100
80
60
40
20
0
control
GalCer (0,05 µM)
GalCer (0,5 µM)
GalCer (5 µM)
Estradiol (150 nM)
GalCer impact on MBP/NF (D30)
MBP/NF signal in% control
140
120
100
80
60
40
20
0
control
GalCer (0,5 µM)
GalCer (5 µM)
Estradiol (150 nM)
GalCer impact on NF (D12)
Neurite network in% control
140
120
100
80
60
40
20
0
control
GalCer (0,05 µM)
Estradiol (150 nM)

Fig.23

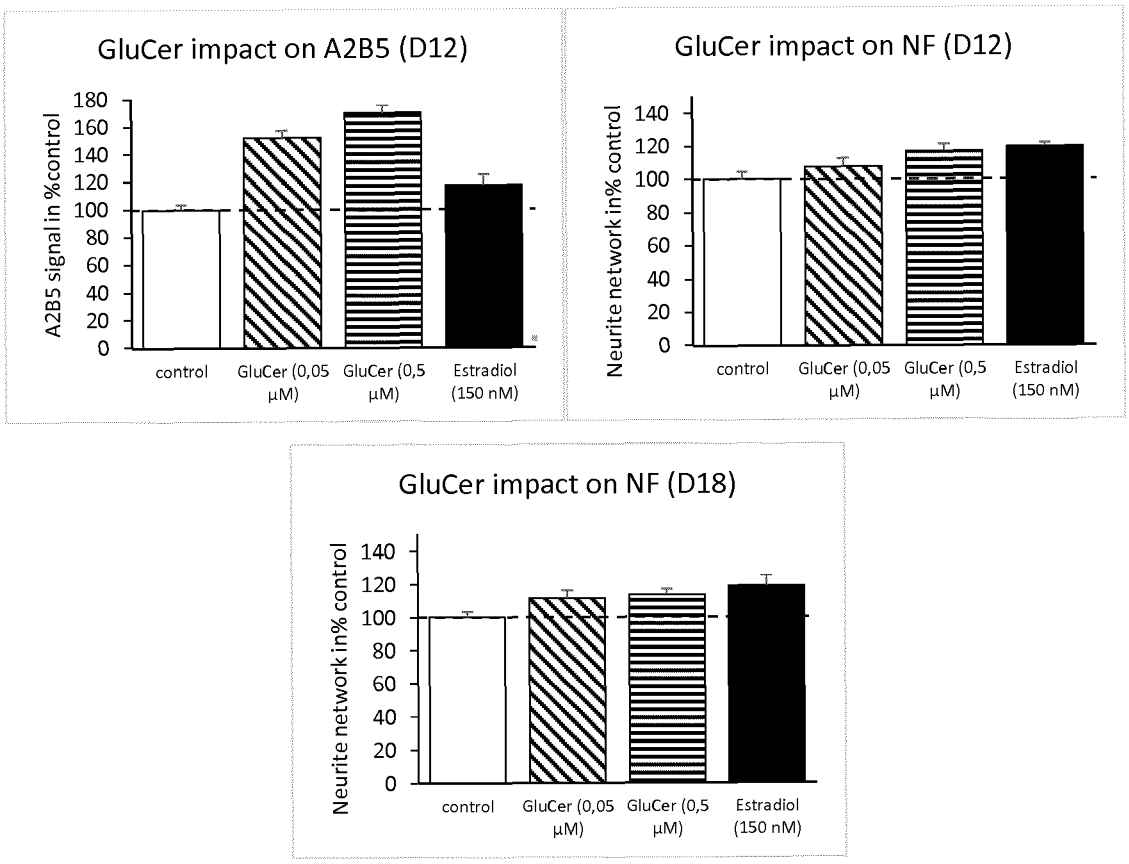
GluCer impact on A2B5 (D12)
A2B5 signal in %control
180
160
140
120
100
80
60
40
20
0
control
GluCer (0,05 µM)
GluCer (0,5 µM)
Estradiol (150 nM)
GluCer impact on NF (D12)
Neurite network in% control
140
120
100
80
60
40
20
0
control
GluCer (0,05 µM)
GluCer (0,5 µM)
Estradiol (150 nM)
GluCer impact on NF (D18)
Neurite network in% control
140
120
100
80
60
40
20
0
control
GluCer (0,05 µM)
GluCer (0,5 µM)
Estradiol (150 nM)

Fig.24

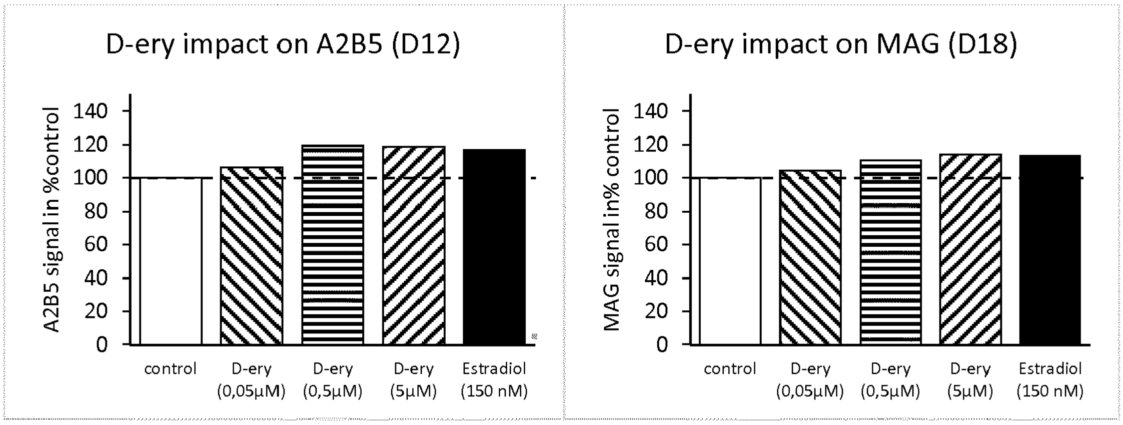
D-ery impact on A2B5 (D12)
A2B5 signal in %control
140
120
100
80
60
40
20
0
control
D-ery (0,05µM)
D-ery (0,5µM)
D-ery (5µM)
Estradiol (150 nM)
D-ery impact on MAG (D18)
MAG signal in% control
140
120
100
80
60
40
20
0
control
D-ery (0,05µM)
D-ery (0,5µM)
D-ery (5µM)
Estradiol (150 nM)

Fig.25

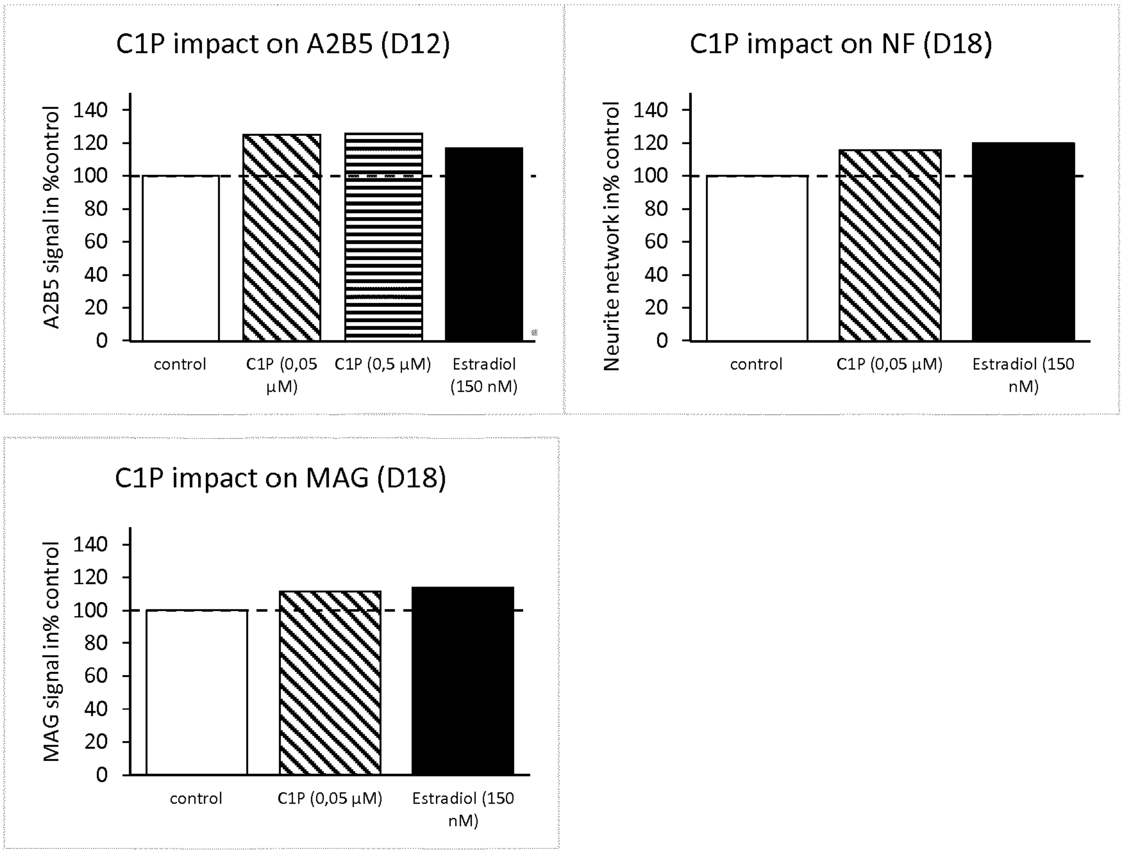
C1P impact on A2B5 (D12)
A2B5 signal in %control
140
120
100
80
60
40
20
0
control
C1P (0,05 µM)
C1P (0,5 µM)
Estradiol (150 nM)
C1P impact on NF (D18)
Neurite network in% control
140
120
100
80
60
40
20
0
control
C1P (0,05 µM)
Estradiol (150 nM)
C1P impact on MAG (D18)
MAG signal in% control
140
120
100
80
60
40
20
0
control
C1P (0,05 µM)
Estradiol (150 nM)

Fig.26

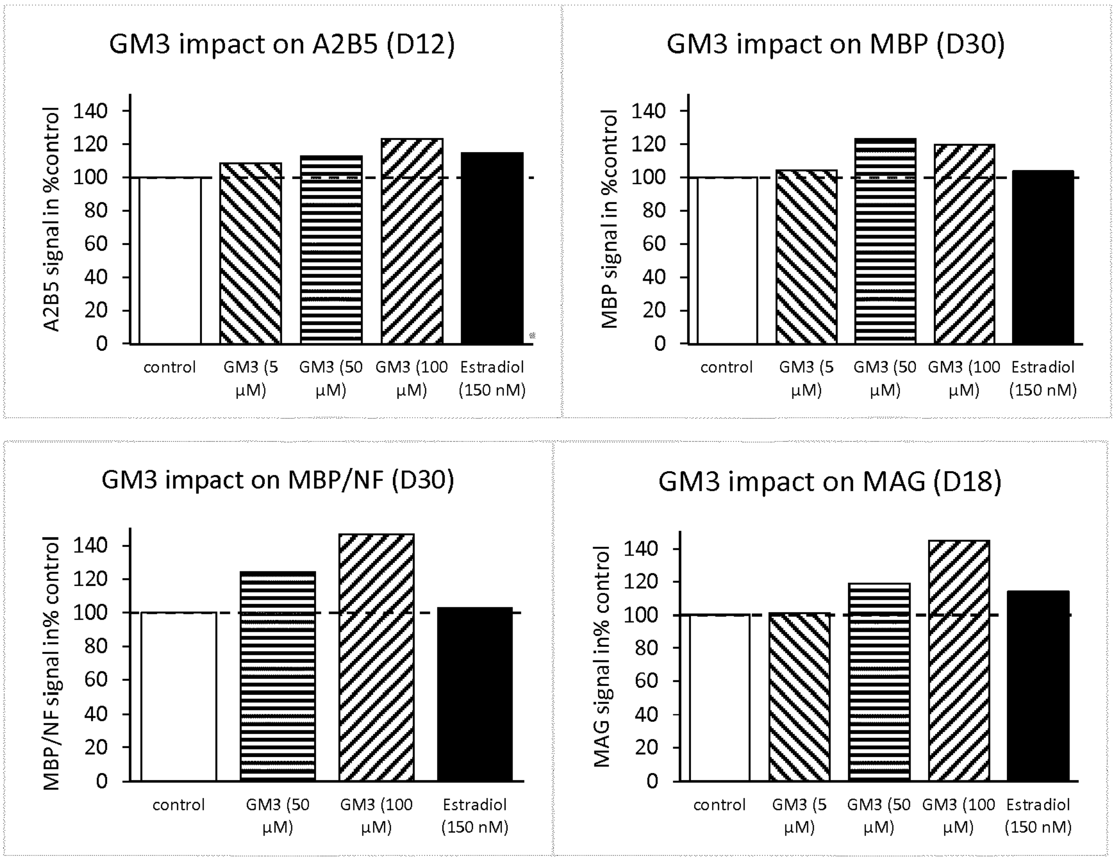
GM3 impact on A2B5 (D12)
A2B5 signal in %control
0
20
40
60
80
100
120
140
control
GM3 (5 µM)
GM3 (50 µM)
GM3 (100 µM)
Estradiol (150 nM)
GM3 impact on MBP (D30)
MBP signal in %control
0
20
40
60
80
100
120
140
control
GM3 (5 µM)
GM3 (50 µM)
GM3 (100 µM)
Estradiol (150 nM)
GM3 impact on MBP/NF (D30)
MBP/NF signal in% control
0
20
40
60
80
100
120
140
control
GM3 (50 µM)
GM3 (100 µM)
Estradiol (150 nM)
GM3 impact on MAG (D18)
MAG signal in% control
0
20
40
60
80
100
120
140
control
GM3 (5 µM)
GM3 (50 µM)
GM3 (100 µM)
Estradiol (150 nM)

Fig.27

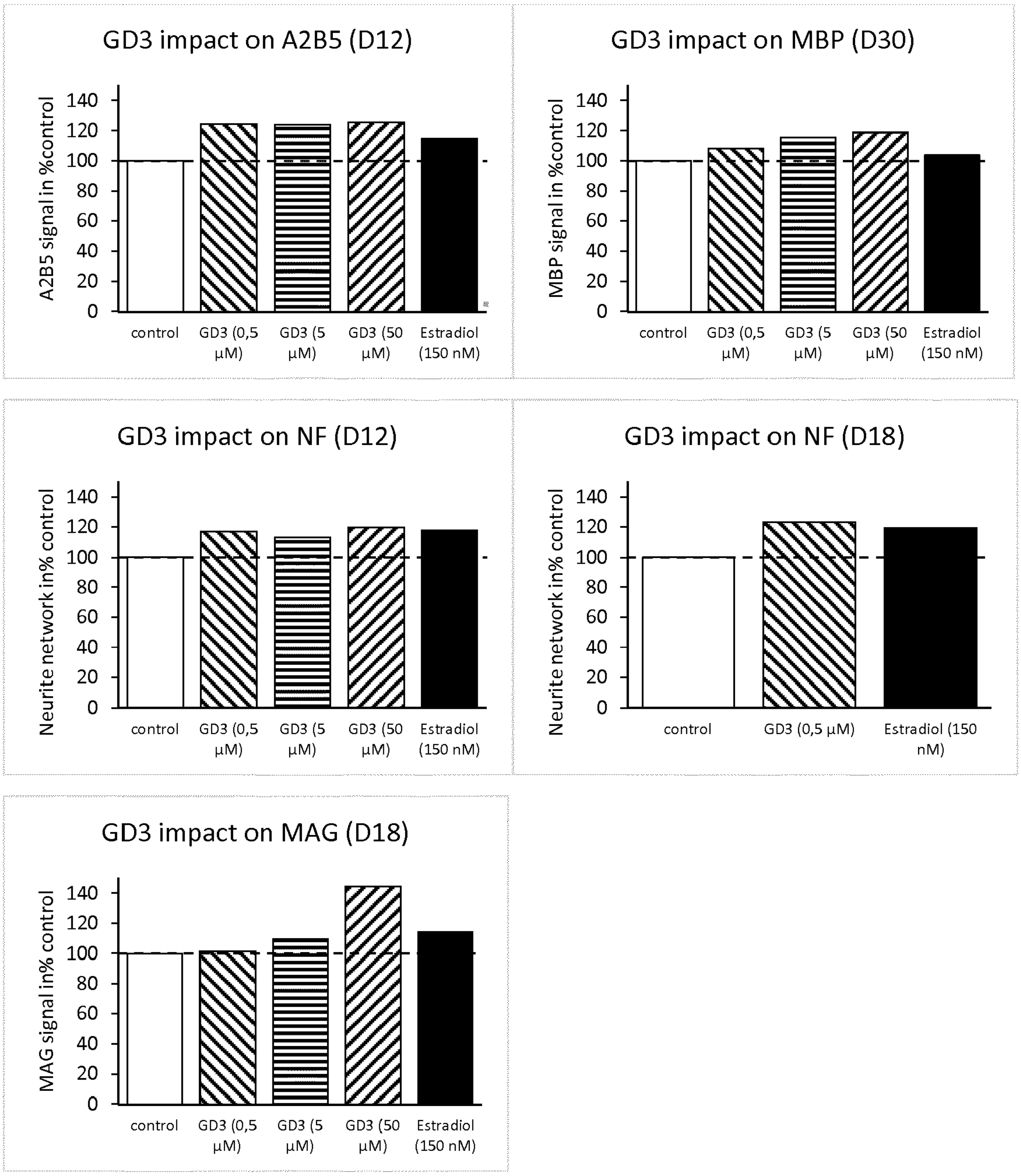
GD3 impact on A2B5 (D12)
A2B5 signal in %control
140
120
100
80
60
40
20
0
control
GD3 (0,5 µM)
GD3 (5 µM)
GD3 (50 µM)
Estradiol (150 nM)
GD3 impact on MBP (D30)
MBP signal in %control
140
120
100
80
60
40
20
0
control
GD3 (0,5 µM)
GD3 (5 µM)
GD3 (50 µM)
Estradiol (150 nM)
GD3 impact on NF (D12)
Neurite network in% control
140
120
100
80
60
40
20
0
control
GD3 (0,5 µM)
GD3 (5 µM)
GD3 (50 µM)
Estradiol (150 nM)
GD3 impact on NF (D18)
Neurite network in% control
140
120
100
80
60
40
20
0
control
GD3 (0,5 µM)
Estradiol (150 nM)
GD3 impact on MAG (D18)
MAG signal in% control
140
120
100
80
60
40
20
0
control
GD3 (0,5 µM)
GD3 (5 µM)
GD3 (50 µM)
Estradiol (150 nM)

Fig.28

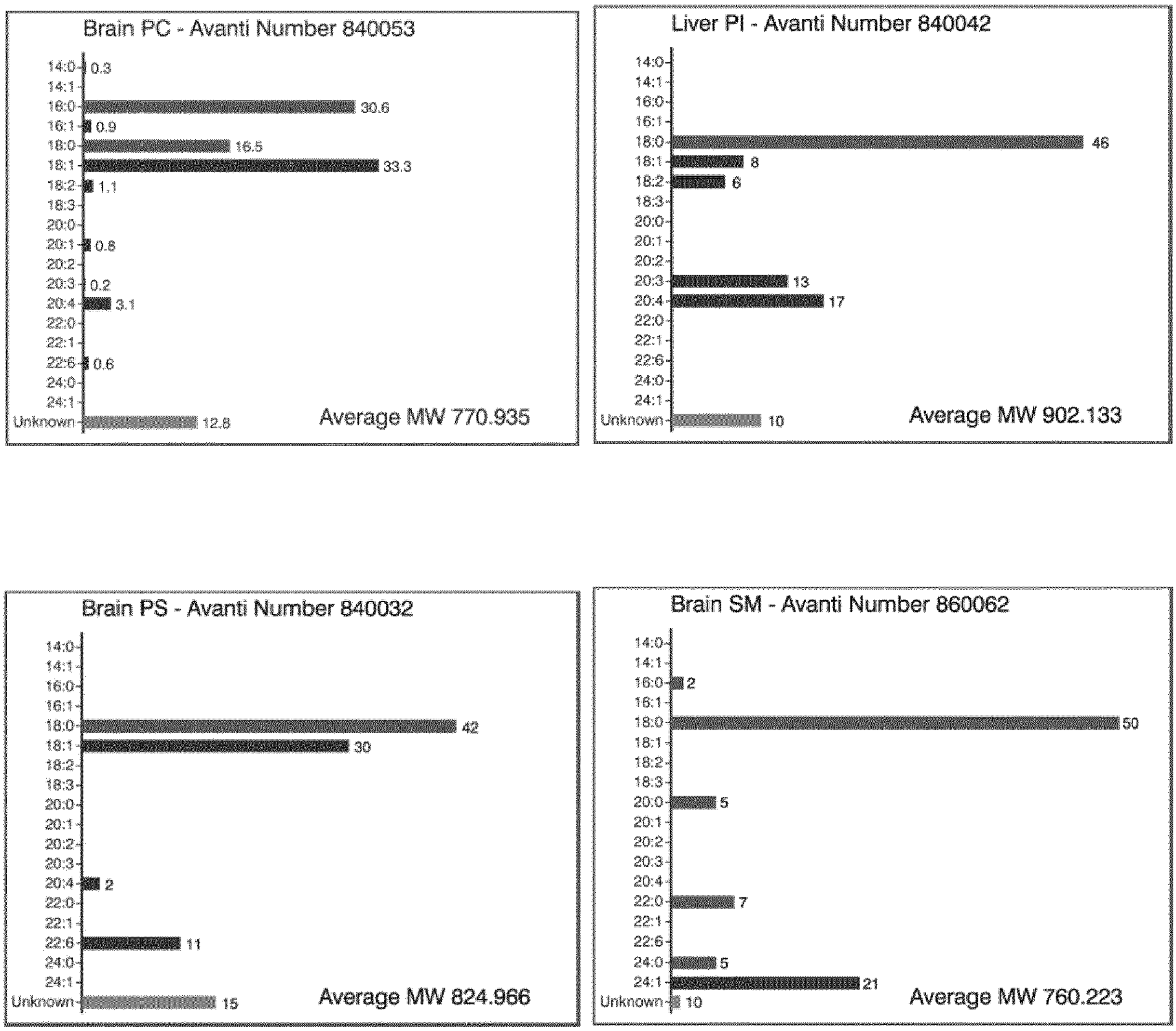
Brain PC - Avanti Number 840053
14:0 0.3
14:1
16:0 30.6
16:1 0.9
18:0 16.5
18:1 33.3
18:2 1.1
18:3
20:0
20:1 0.8
20:2
20:3 0.2
20:4 3.1
22:0
22:1
22:6 0.6
24:0
24:1
Unknown 12.8
Average MW 770.935
Liver PI - Avanti Number 840042
14:0
14:1
16:0
16:1
18:0 46
18:1 8
18:2 6
18:3
20:0
20:1
20:2
20:3 13
20:4 17
22:0
22:1
22:6
24:0
24:1
Unknown 10
Average MW 902.133
Brain PS - Avanti Number 840032
14:0
14:1
16:0
16:1
18:0 42
18:1 30
18:2
18:3
20:0
20:1
20:2
20:3
20:4 2
22:0
22:1
22:6 11
24:0
24:1
Unknown 15
Average MW 824.966
Brain SM - Avanti Number 860062
14:0
14:1
16:0 2
16:1
18:0 50
18:1
18:2
18:3
20:0 5
20:1
20:2
20:3
20:4
22:0 7
22:1
22:6
24:0 5
24:1 21
Unknown 10
Average MW 760.223

Fig.29

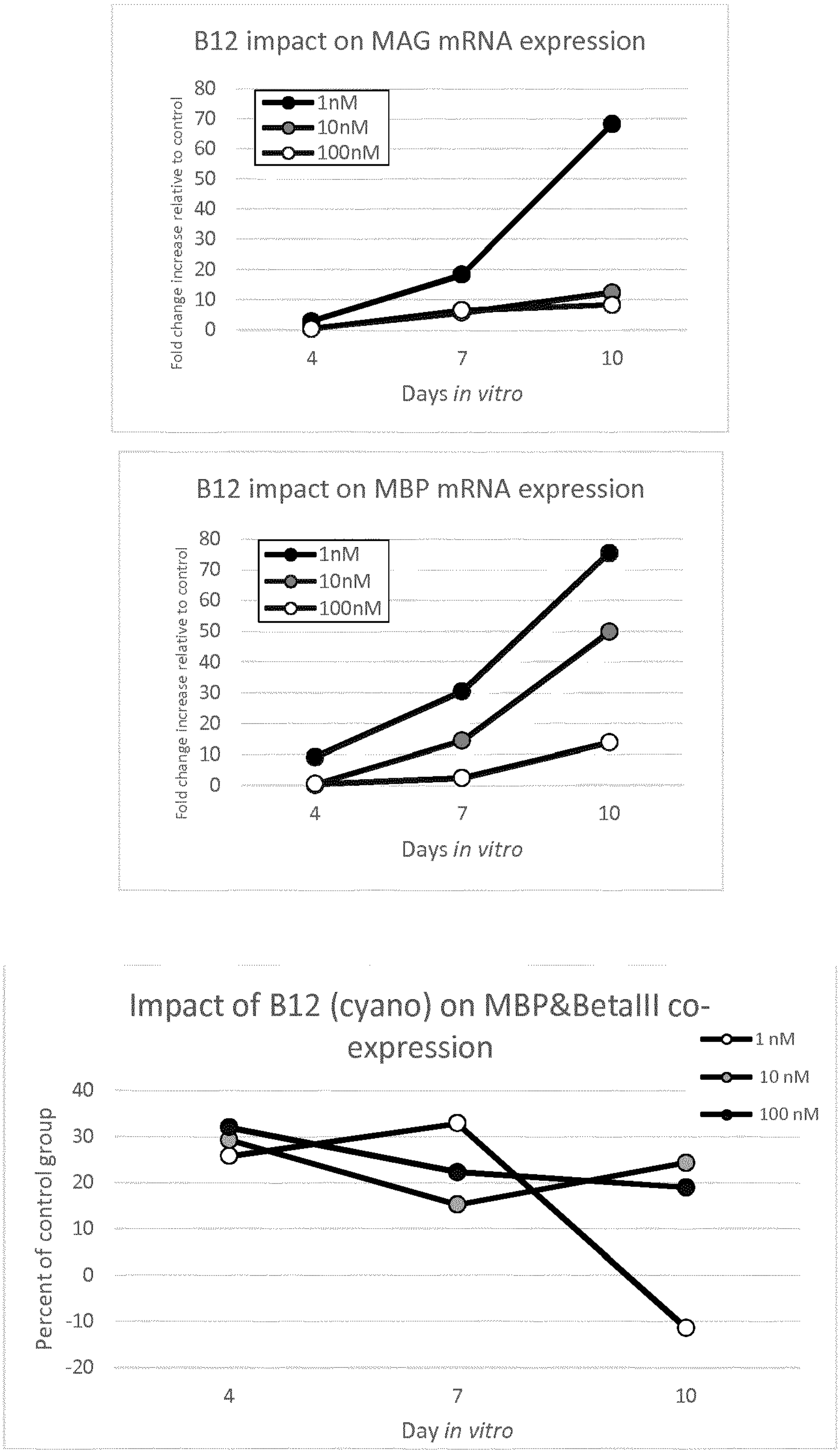
B12 impact on MAG mRNA expression
Fold change increase relative to control
80
70
60
50
40
30
20
10
0
1nM
10nM
100nM
4
7
10
Days in vitro
B12 impact on MBP mRNA expression
Fold change increase relative to control
80
70
60
50
40
30
20
10
0
1nM
10nM
100nM
4
7
10
Days in vitro
Impact of B12 (cyano) on MBP&BetaIII co-expression
1 nM
10 nM
100 nM
Percent of control group
40
30
20
10
0
-10
-20
4
7
10
Day in vitro

Fig. 30

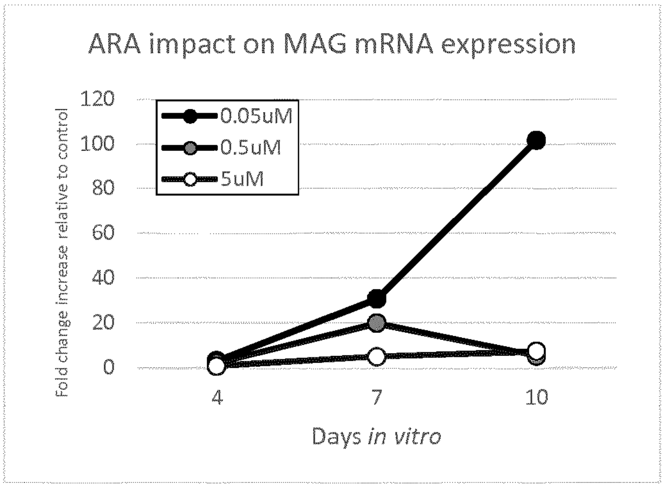
ARA impact on MAG mRNA expression
Fold change increase relative to control
120
100
80
60
40
20
0
0.05uM
0.5uM
5uM
4
7
10
Days in vitro

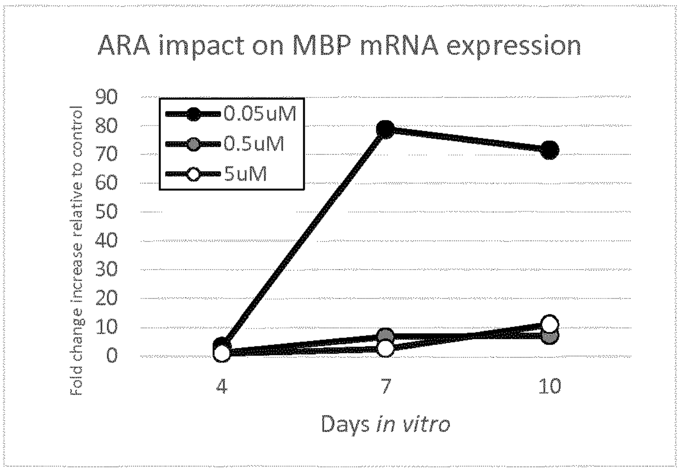
ARA impact on MBP mRNA expression
Fold change increase relative to control
90
80
70
60
50
40
30
20
10
0
0.05uM
0.5uM
5uM
4
7
10
Days in vitro

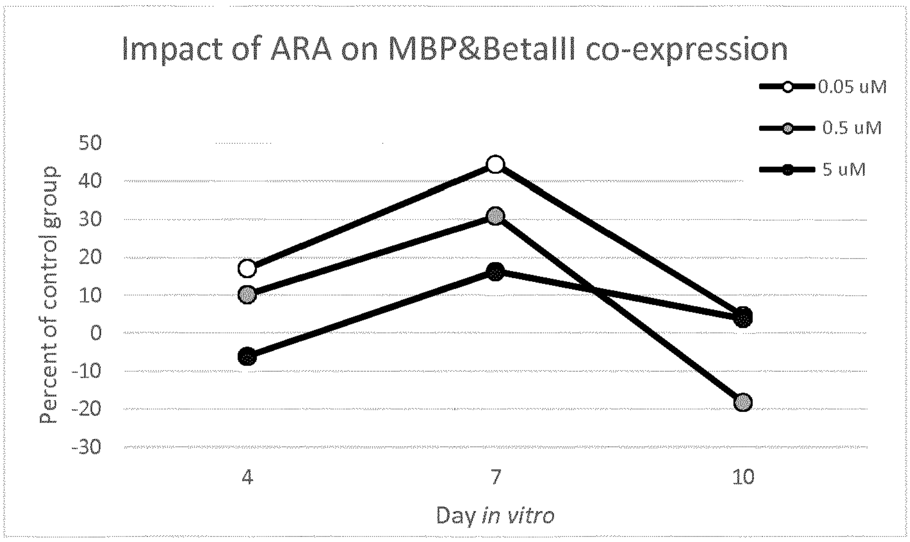
Impact of ARA on MBP&BetaIII co-expression
Percent of control group
50
40
30
20
10
0
-10
-20
-30
0.05 uM
0.5 uM
5 uM
4
7
10
Day in vitro

Fig. 31

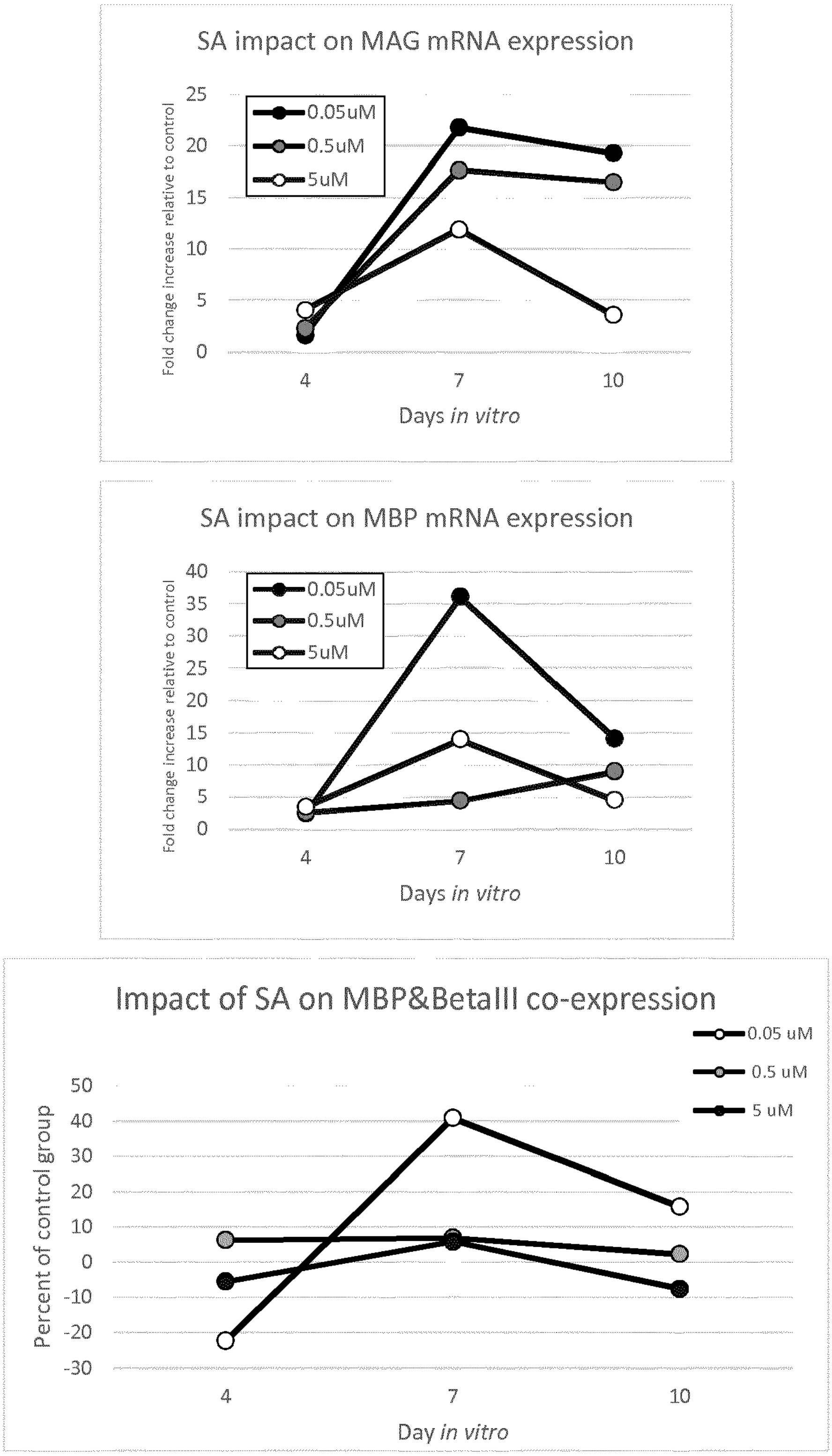
SA impact on MAG mRNA expression
0.05uM
0.5uM
5uM
Fold change increase relative to control
25
20
15
10
5
0
4
7
10
Days in vitro
SA impact on MBP mRNA expression
0.05uM
0.5uM
5uM
Fold change increase relative to control
40
35
30
25
20
15
10
5
0
4
7
10
Days in vitro
Impact of SA on MBP&BetaIII co-expression
0.05 uM
0.5 uM
5 uM
Percent of control group
50
40
30
20
10
0
-10
-20
-30
4
7
10
Day in vitro

Fig. 32

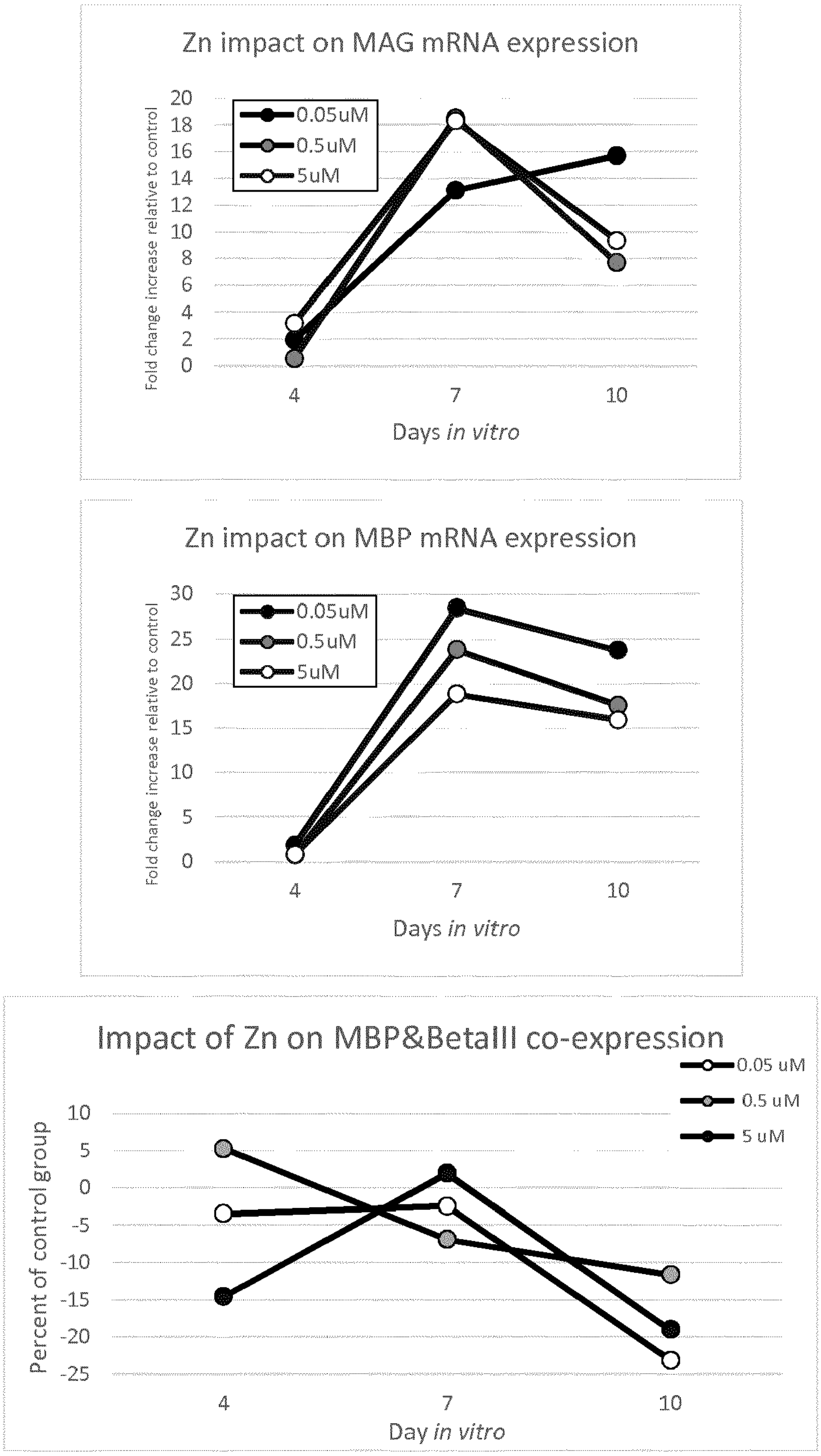
Zn impact on MAG mRNA expression
0.05uM
0.5uM
5uM
Fold change increase relative to control
20
18
16
14
12
10
8
6
4
2
0
4
7
10
Days in vitro
Zn impact on MBP mRNA expression
0.05uM
0.5uM
5uM
Fold change increase relative to control
30
25
20
15
10
5
0
4
7
10
Days in vitro
Impact of Zn on MBP&BetaIII co-expression
0.05 uM
0.5 uM
5 uM
Percent of control group
10
5
0
-5
-10
-15
-20
-25
4
7
10
Day in vitro

Fig.33

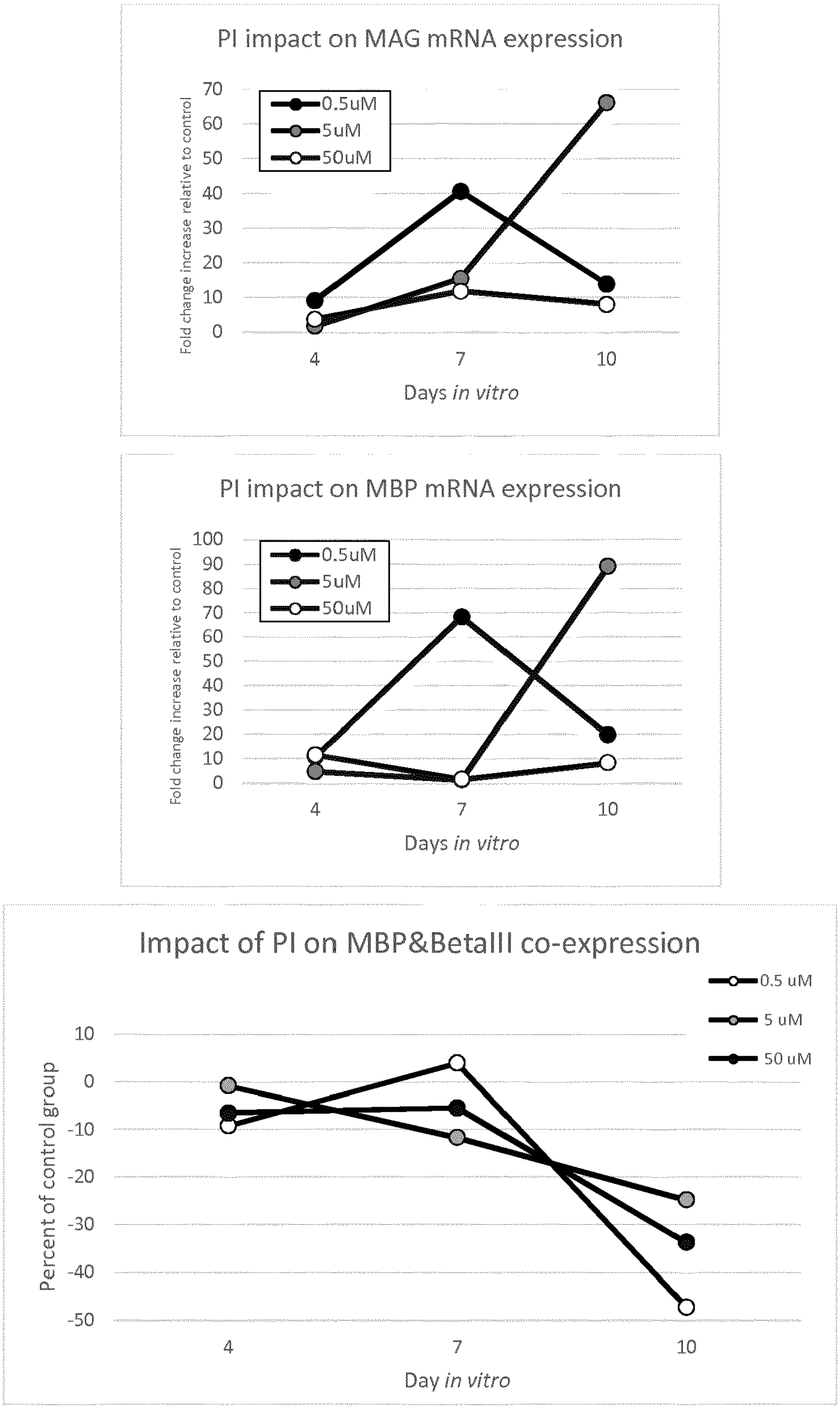
PI impact on MAG mRNA expression
Fold change increase relative to control
70
60
50
40
30
20
10
0
0.5uM
5uM
50uM
4
7
10
Days in vitro
PI impact on MBP mRNA expression
Fold change increase relative to control
100
90
80
70
60
50
40
30
20
10
0
0.5uM
5uM
50uM
4
7
10
Days in vitro
Impact of PI on MBP&BetaIII co-expression
Percent of control group
10
0
-10
-20
-30
-40
-50
0.5 uM
5 uM
50 uM
4
7
10
Day in vitro

Fig. 34

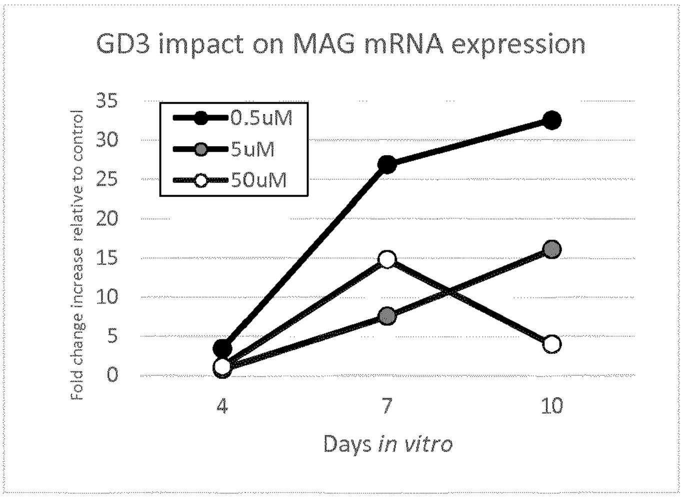
GD3 impact on MAG mRNA expression
Fold change increase relative to control
35
30
25
20
15
10
5
0
0.5uM
5uM
50uM
4
7
10
Days in vitro

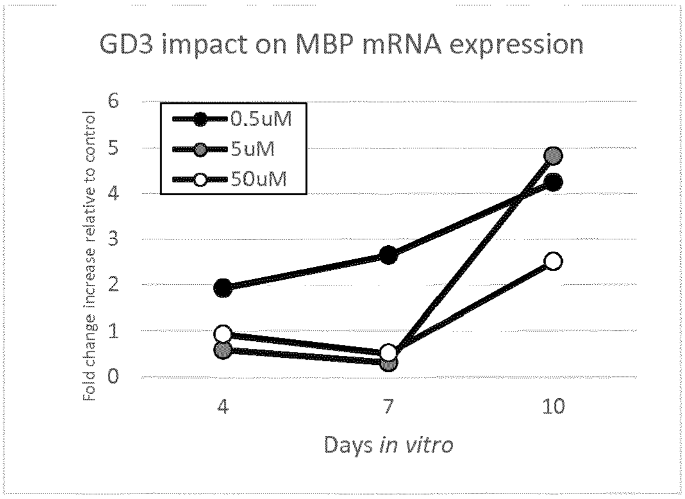
GD3 impact on MBP mRNA expression
Fold change increase relative to control
6
5
4
3
2
1
0
0.5uM
5uM
50uM
4
7
10
Days in vitro

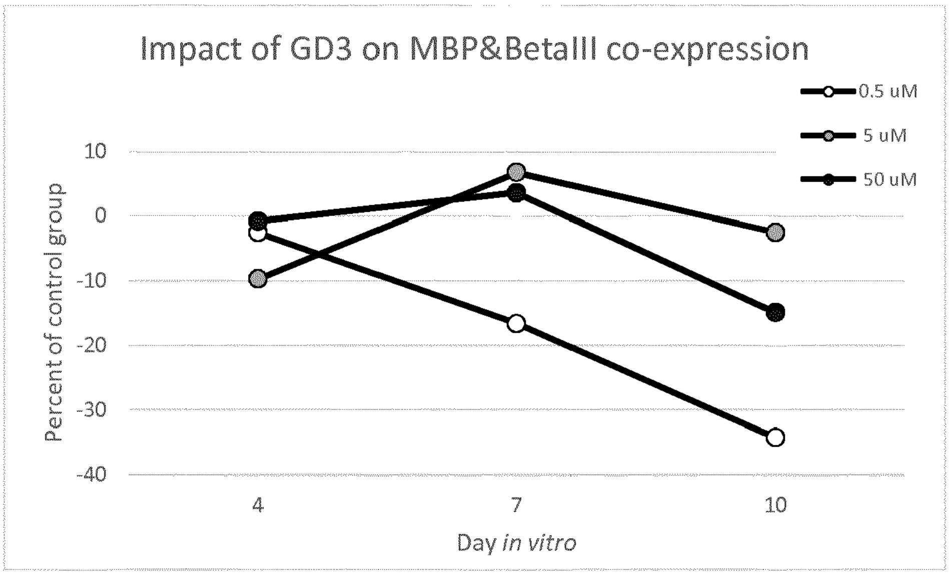
Impact of GD3 on MBP&BetaIII co-expression
Percent of control group
10
0
-10
-20
-30
-40
0.5 uM
5 uM
50 uM
4
7
10
Day in vitro

Fig. 35

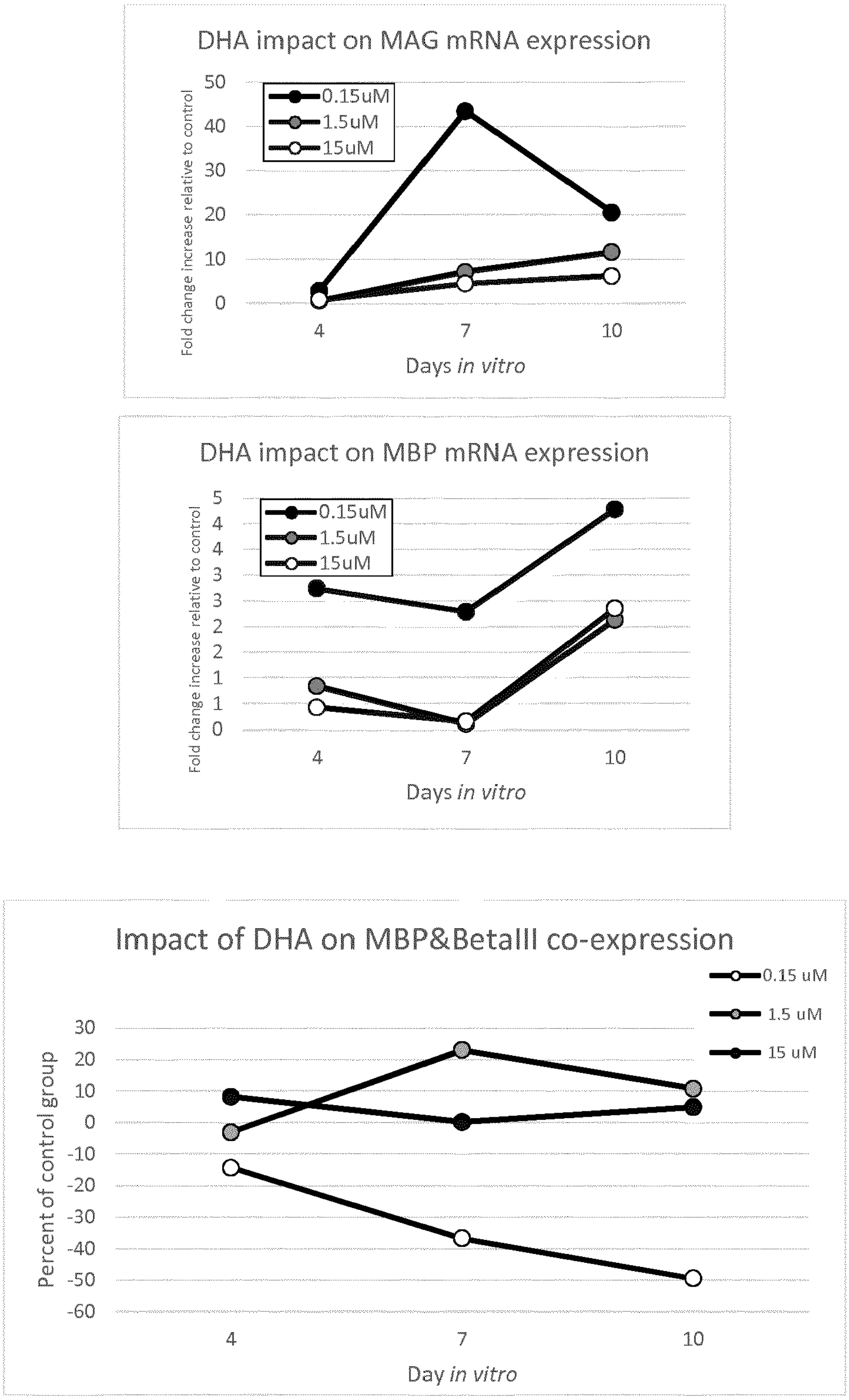
DHA impact on MAG mRNA expression
Fold change increase relative to control
50
40
30
20
10
0
0.15uM
1.5uM
15uM
4
7
10
Days in vitro
DHA impact on MBP mRNA expression
Fold change increase relative to control
5
4
4
3
3
2
2
1
1
0
0.15uM
1.5uM
15uM
4
7
10
Days in vitro
Impact of DHA on MBP&BetaIII co-expression
Percent of control group
30
20
10
0
-10
-20
-30
-40
-50
-60
0.15 uM
1.5 uM
15 uM
4
7
10
Day in vitro

Fig. 36

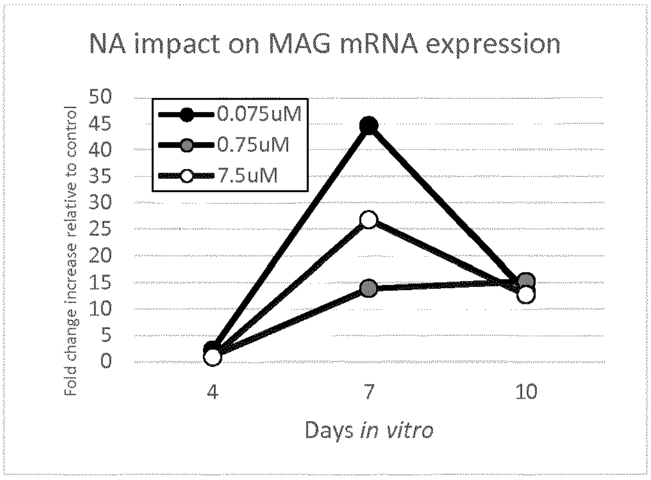
NA impact on MAG mRNA expression
Fold change increase relative to control
0.075uM
0.75uM
7.5uM
50
45
40
35
30
25
20
15
10
5
0
4
7
10
Days *in vitro*

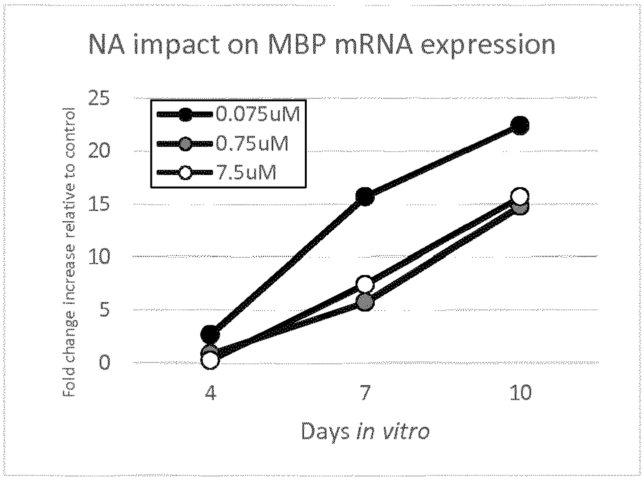
NA impact on MBP mRNA expression
Fold change increase relative to control
0.075uM
0.75uM
7.5uM
25
20
15
10
5
0
4
7
10
Days *in vitro*

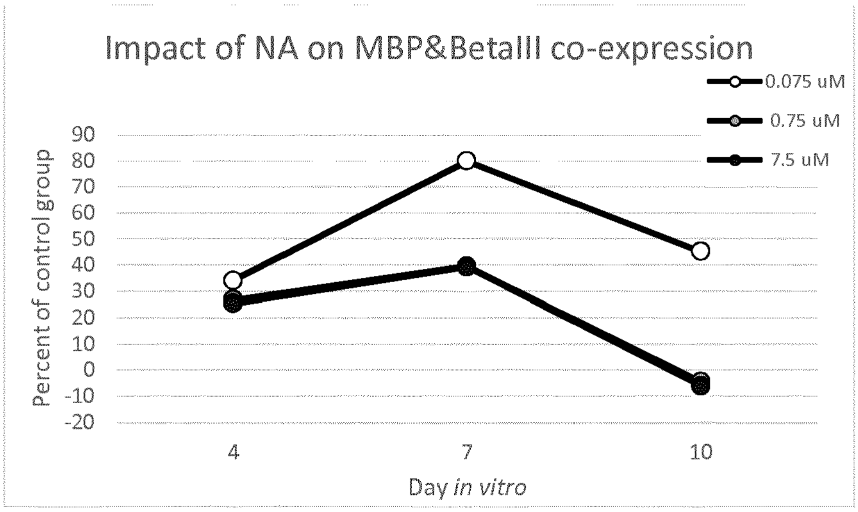
Impact of NA on MBP&BetaIII co-expression
0.075 uM
0.75 uM
7.5 uM
Percent of control group
90
80
70
60
50
40
30
20
10
0
-10
-20
4
7
10
Day *in vitro*

Fig. 37

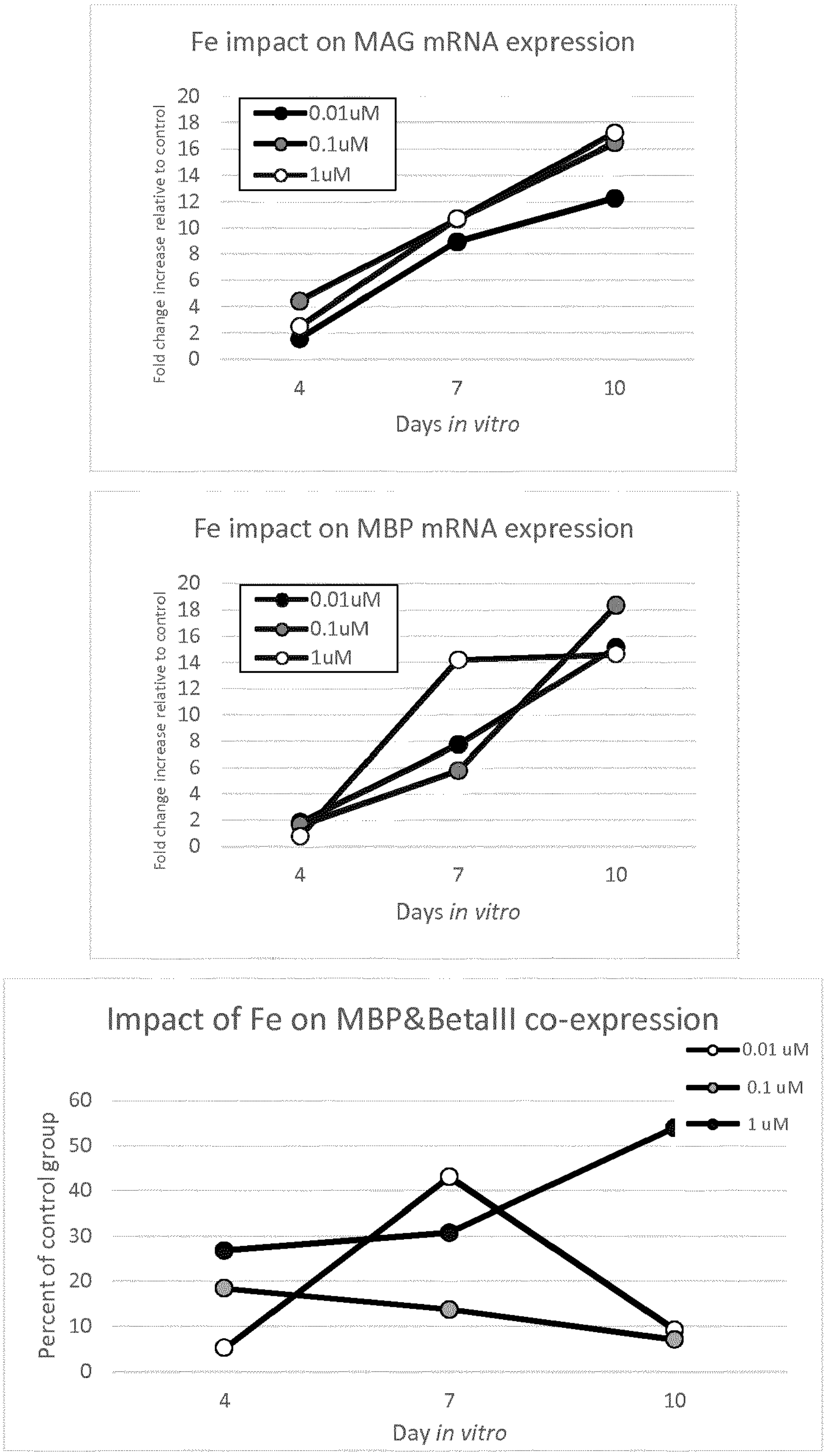
Fe impact on MAG mRNA expression
Fold change increase relative to control
20
18
16
14
12
10
8
6
4
2
0
0.01uM
0.1uM
1uM
4
7
10
Days *in vitro*
Fe impact on MBP mRNA expression
Fold change increase relative to control
20
18
16
14
12
10
8
6
4
2
0
0.01uM
0.1uM
1uM
4
7
10
Days *in vitro*
Impact of Fe on MBP&BetaIII co-expression
0.01 uM
0.1 uM
1 uM
Percent of control group
60
50
40
30
20
10
0
4
7
10
Day *in vitro*

Fig. 38

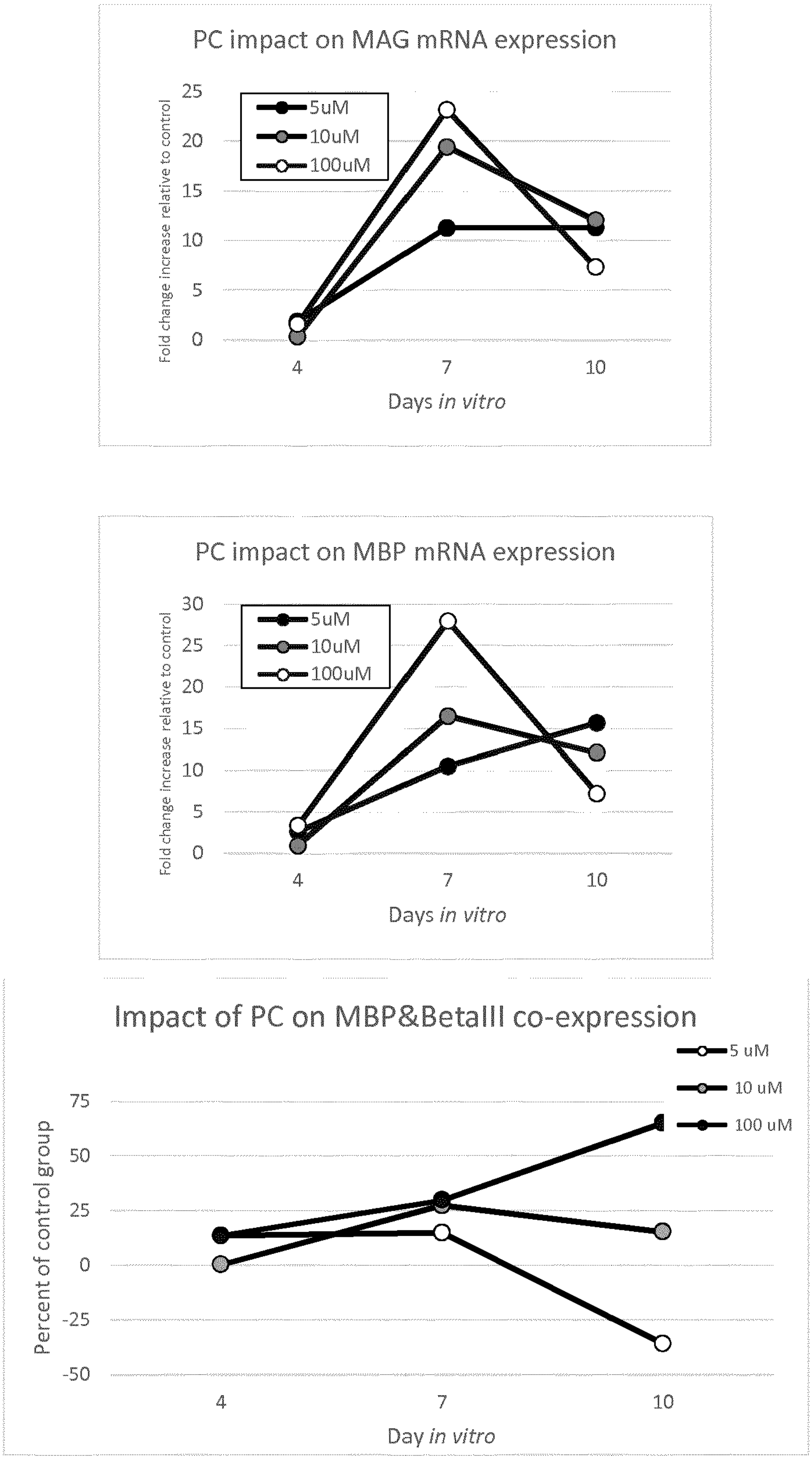

PC impact on MAG mRNA expression
Fold change increase relative to control
25
20
15
10
5
0
5uM
10uM
100uM
4
7
10
Days in vitro
PC impact on MBP mRNA expression
Fold change increase relative to control
30
25
20
15
10
5
0
5uM
10uM
100uM
4
7
10
Days in vitro
Impact of PC on MBP&BetaIII co-expression
5 uM
10 uM
100 uM
Percent of control group
75
50
25
0
-25
-50
4
7
10
Day in vitro


Fig. 39

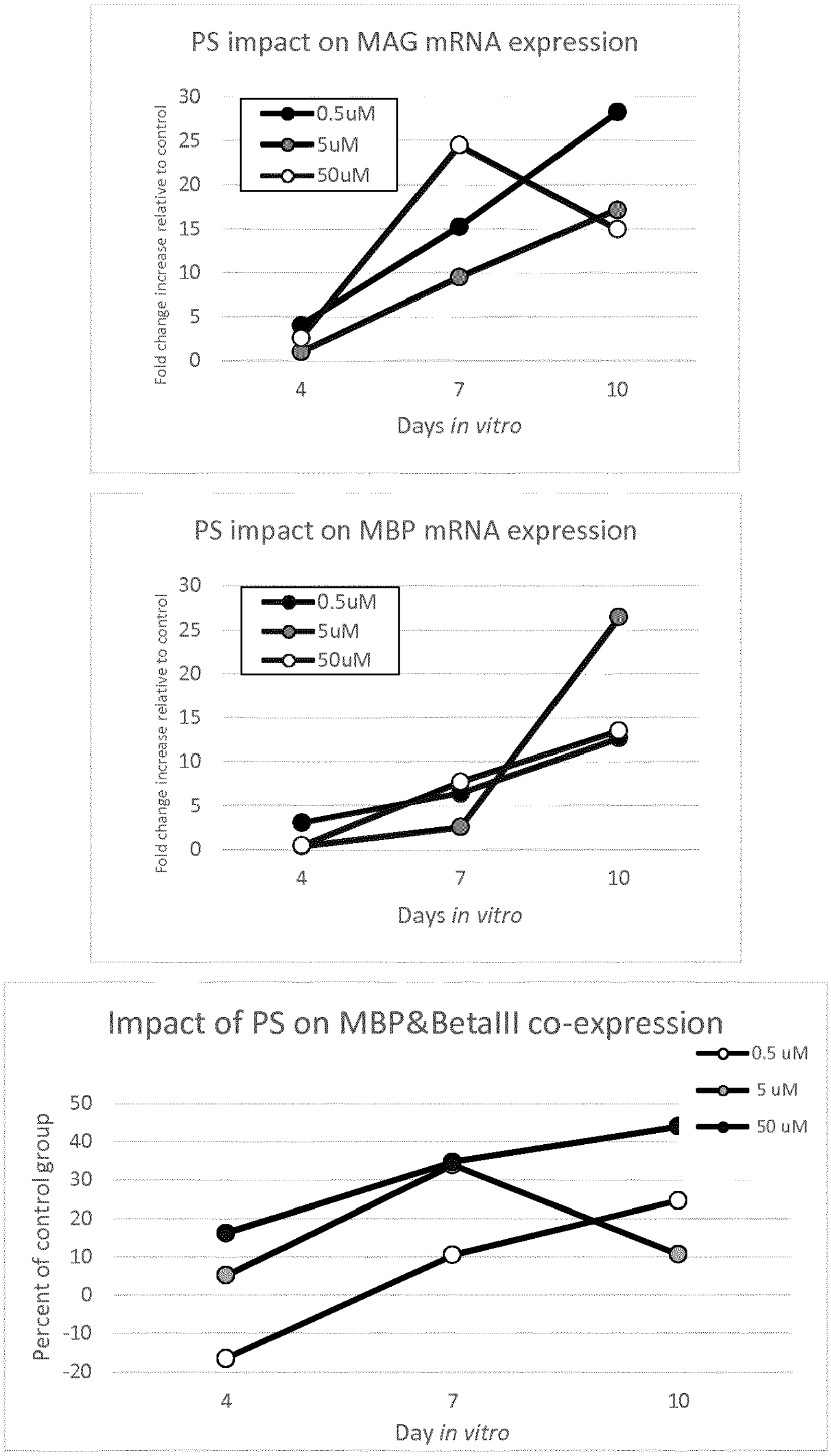
PS impact on MAG mRNA expression
Fold change increase relative to control
30
25
20
15
10
5
0
0.5uM
5uM
50uM
4
7
10
Days *in vitro*
PS impact on MBP mRNA expression
Fold change increase relative to control
30
25
20
15
10
5
0
0.5uM
5uM
50uM
4
7
10
Days *in vitro*
Impact of PS on MBP&BetaIII co-expression
Percent of control group
50
40
30
20
10
0
-10
-20
0.5 uM
5 uM
50 uM
4
7
10
Day *in vitro*

Fig. 40

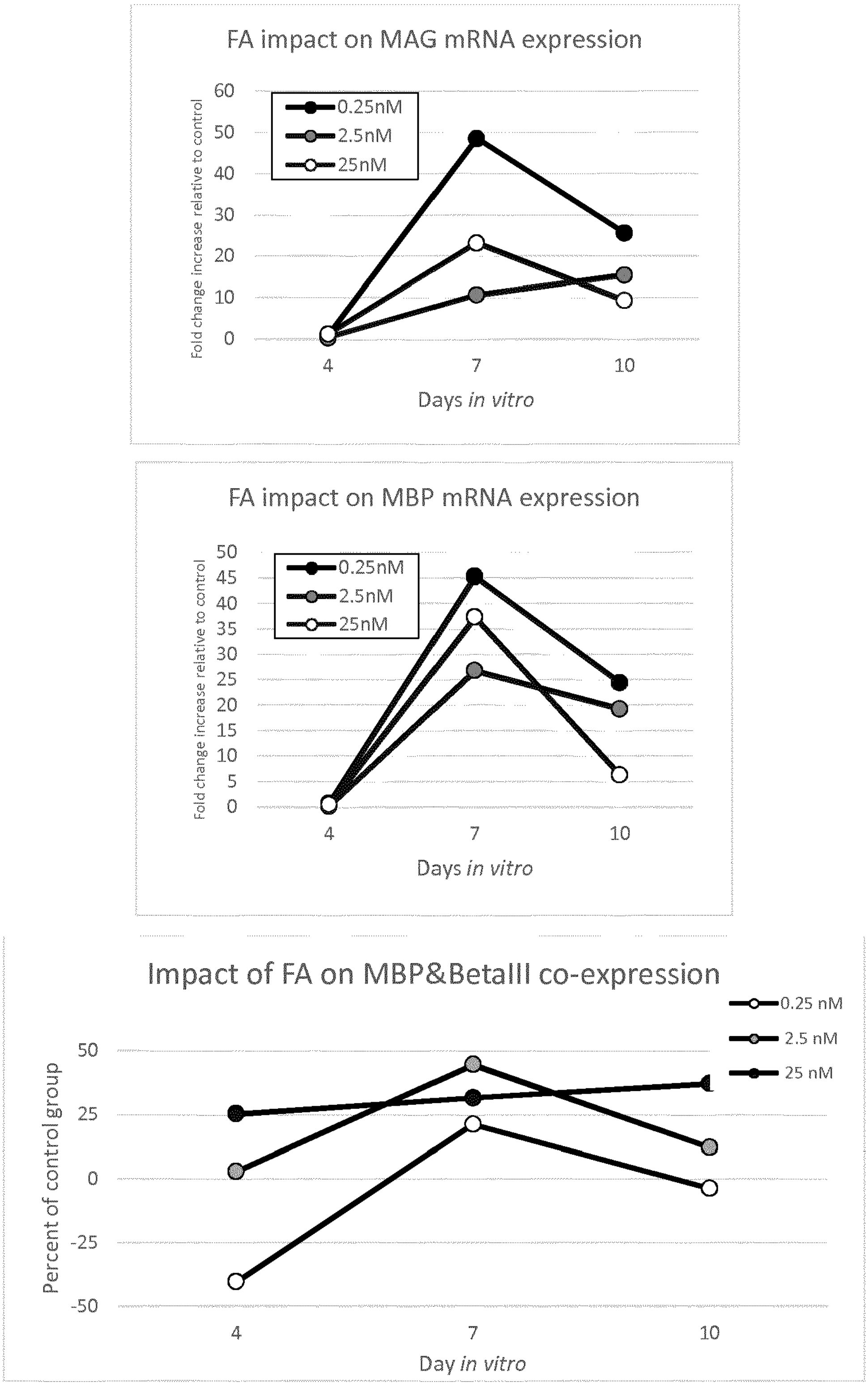
FA impact on MAG mRNA expression
0.25nM
2.5nM
25nM
Fold change increase relative to control
60
50
40
30
20
10
0
4
7
10
Days in vitro
FA impact on MBP mRNA expression
0.25nM
2.5nM
25nM
Fold change increase relative to control
50
45
40
35
30
25
20
15
10
5
0
4
7
10
Days in vitro
Impact of FA on MBP&BetaIII co-expression
0.25 nM
2.5 nM
25 nM
Percent of control group
50
25
0
-25
-50
4
7
10
Day in vitro

Fig. 41

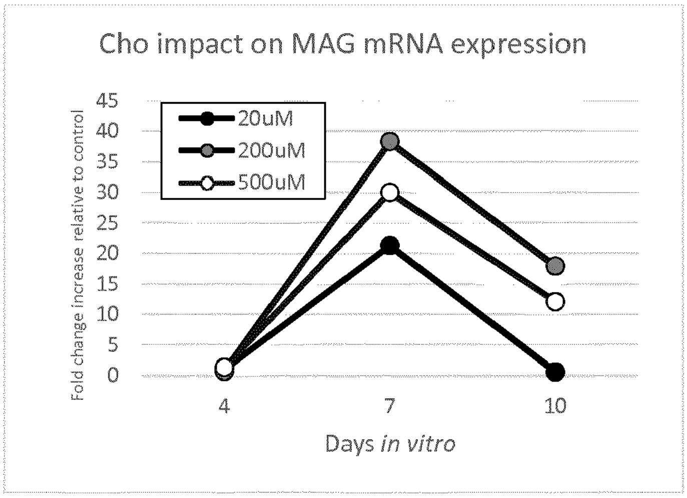

Cho impact on MAG mRNA expression
Fold change increase relative to control
45
40
35
30
25
20
15
10
5
0
20uM
200uM
500uM
4
7
10
Days *in vitro*


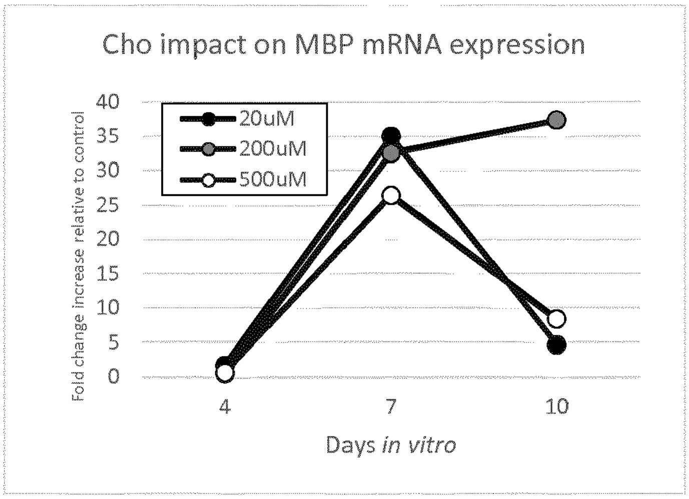

Cho impact on MBP mRNA expression
Fold change increase relative to control
40
35
30
25
20
15
10
5
0
20uM
200uM
500uM
4
7
10
Days *in vitro*


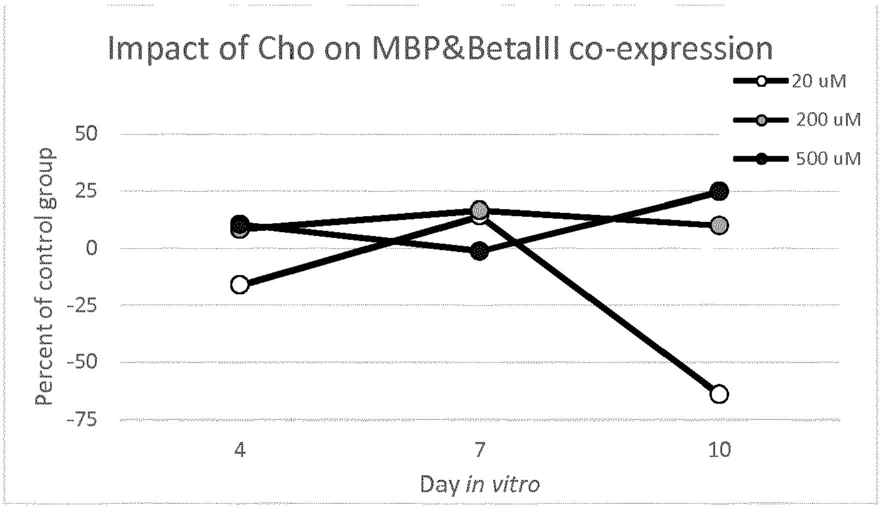

Impact of Cho on MBP&BetaIII co-expression
20 uM
200 uM
500 uM
Percent of control group
50
25
0
-25
-50
-75
4
7
10
Day *in vitro*


Fig. 42

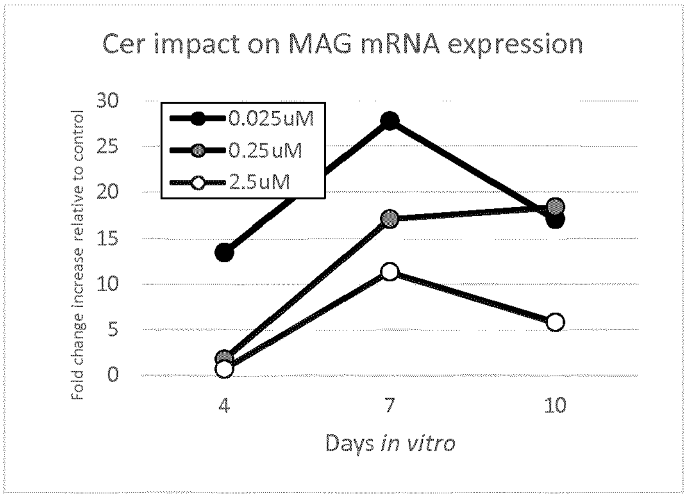
Cer impact on MAG mRNA expression
0.025uM
0.25uM
2.5uM
Fold change increase relative to control
30
25
20
15
10
5
0
4
7
10
Days in vitro

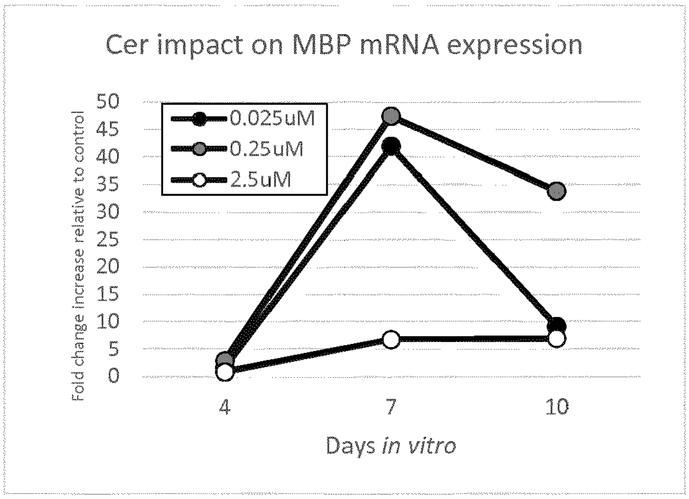
Cer impact on MBP mRNA expression
0.025uM
0.25uM
2.5uM
Fold change increase relative to control
50
45
40
35
30
25
20
15
10
5
0
4
7
10
Days in vitro

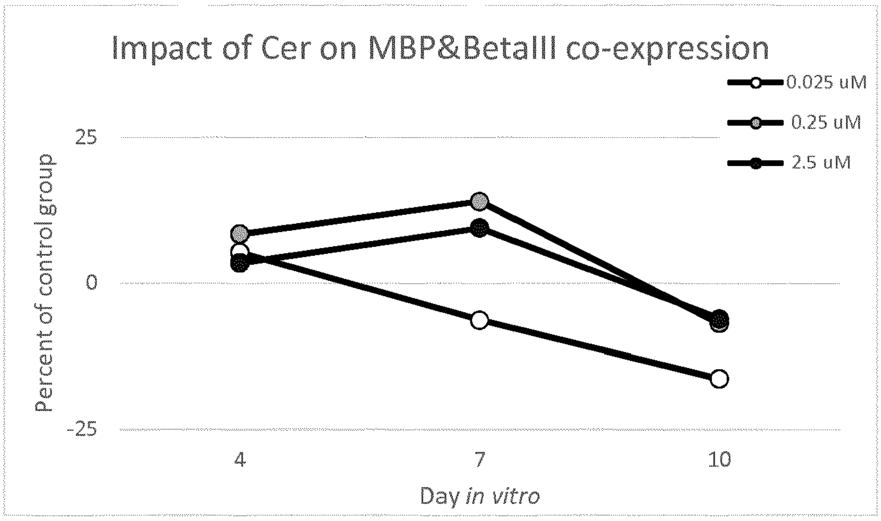
Impact of Cer on MBP&BetaIII co-expression
0.025 uM
0.25 uM
2.5 uM
Percent of control group
25
0
-25
4
7
10
Day in vitro

Fig. 43

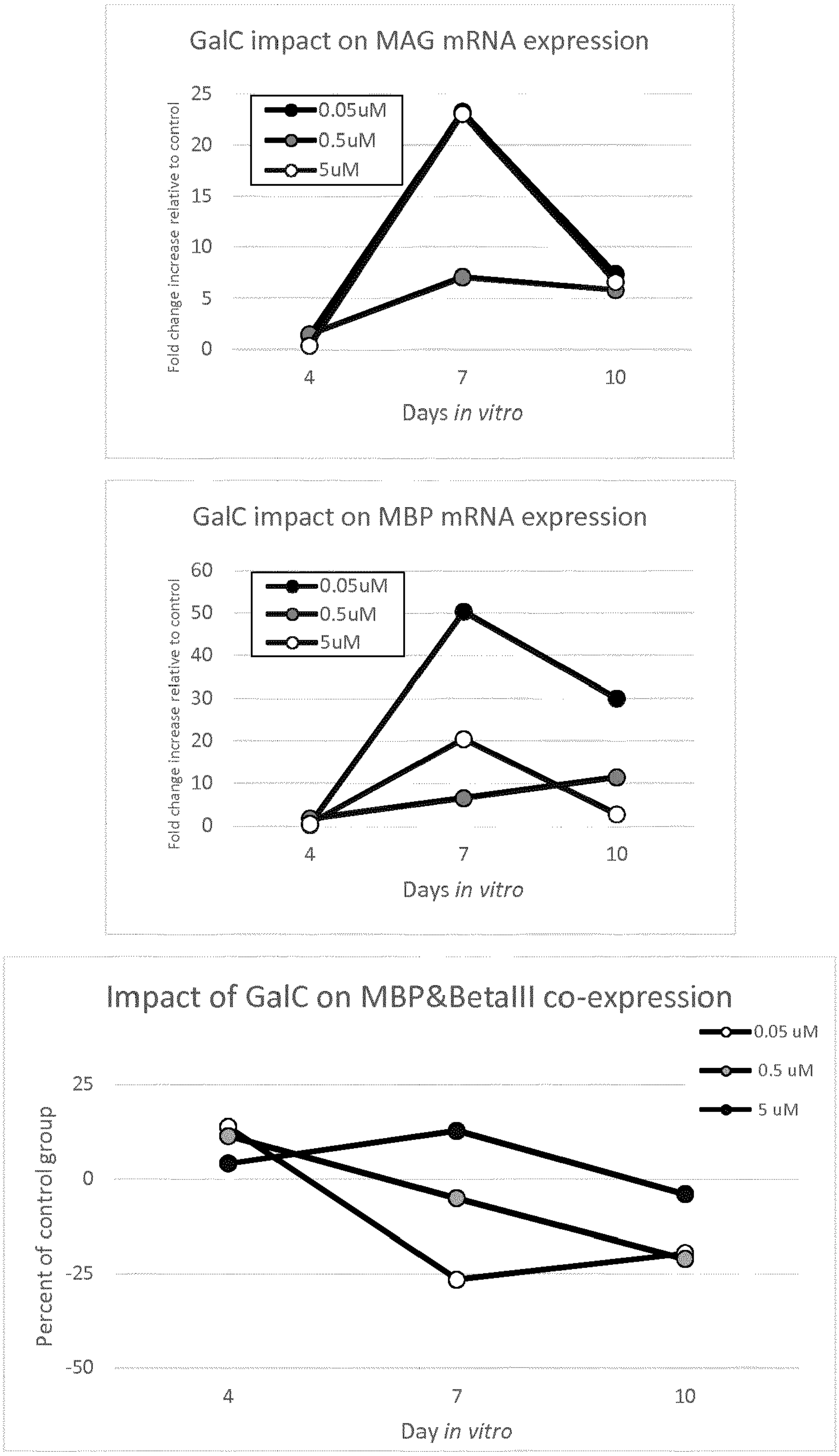
GalC impact on MAG mRNA expression
Fold change increase relative to control
25
20
15
10
5
0
0.05uM
0.5uM
5uM
4
7
10
Days in vitro
GalC impact on MBP mRNA expression
Fold change increase relative to control
60
50
40
30
20
10
0
0.05uM
0.5uM
5uM
4
7
10
Days in vitro
Impact of GalC on MBP&BetaIII co-expression
Percent of control group
25
0
-25
-50
0.05 uM
0.5 uM
5 uM
4
7
10
Day in vitro

Fig. 44

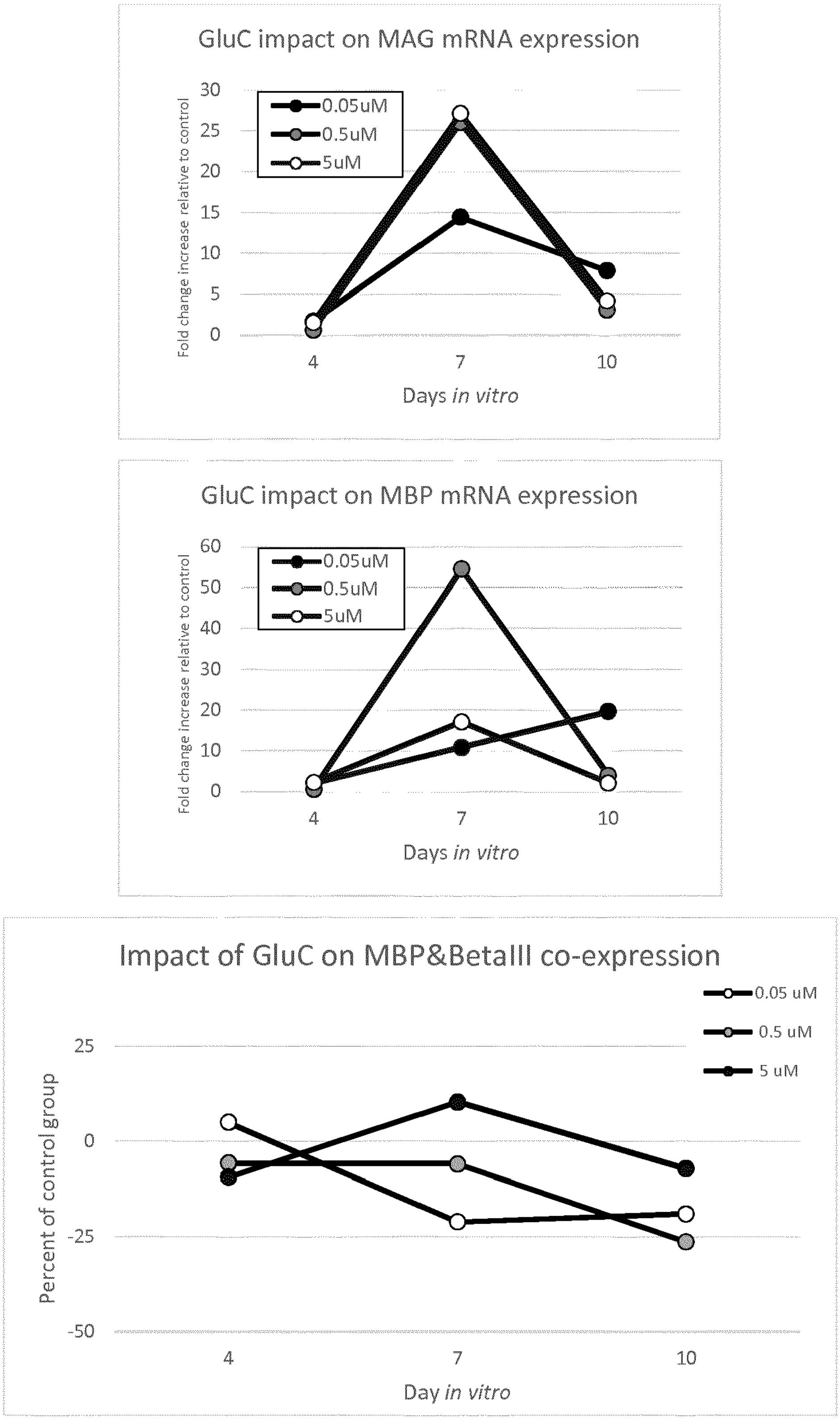
GluC impact on MAG mRNA expression
Fold change increase relative to control
30
25
20
15
10
5
0
0.05uM
0.5uM
5uM
4
7
10
Days *in vitro*
GluC impact on MBP mRNA expression
Fold change increase relative to control
60
50
40
30
20
10
0
0.05uM
0.5uM
5uM
4
7
10
Days *in vitro*
Impact of GluC on MBP&BetaIII co-expression
Percent of control group
25
0
-25
-50
0.05 uM
0.5 uM
5 uM
4
7
10
Day *in vitro*

Fig. 45

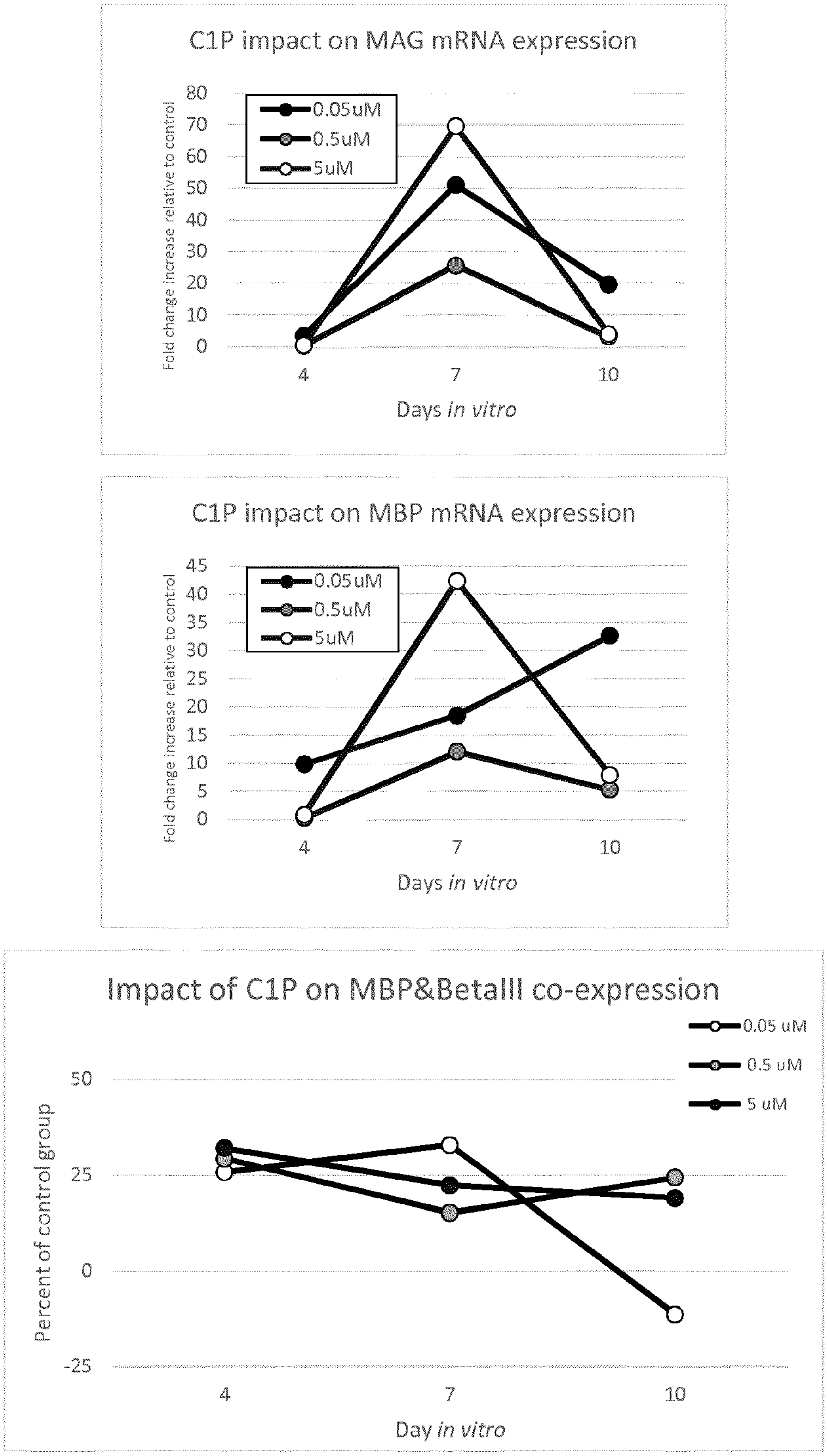
C1P impact on MAG mRNA expression
0.05uM
0.5uM
5uM
Fold change increase relative to control
80
70
60
50
40
30
20
10
0
4
7
10
Days in vitro
C1P impact on MBP mRNA expression
0.05uM
0.5uM
5uM
Fold change increase relative to control
45
40
35
30
25
20
15
10
5
0
4
7
10
Days in vitro
Impact of C1P on MBP&BetaIII co-expression
0.05 uM
0.5 uM
5 uM
Percent of control group
50
25
0
-25
4
7
10
Day in vitro

Fig. 46

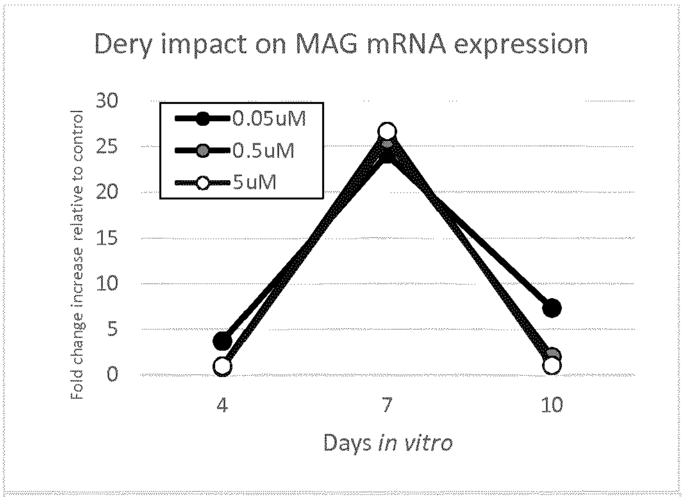
Dery impact on MAG mRNA expression
0.05uM
0.5uM
5uM
Fold change increase relative to control
30
25
20
15
10
5
0
4
7
10
Days *in vitro*

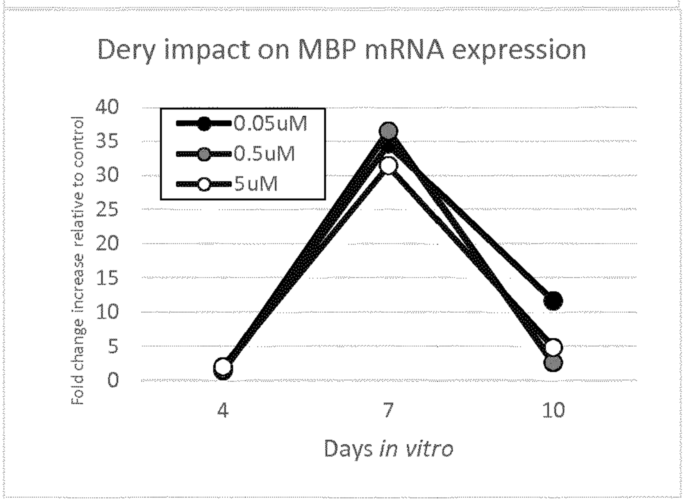
Dery impact on MBP mRNA expression
0.05uM
0.5uM
5uM
Fold change increase relative to control
40
35
30
25
20
15
10
5
0
4
7
10
Days *in vitro*

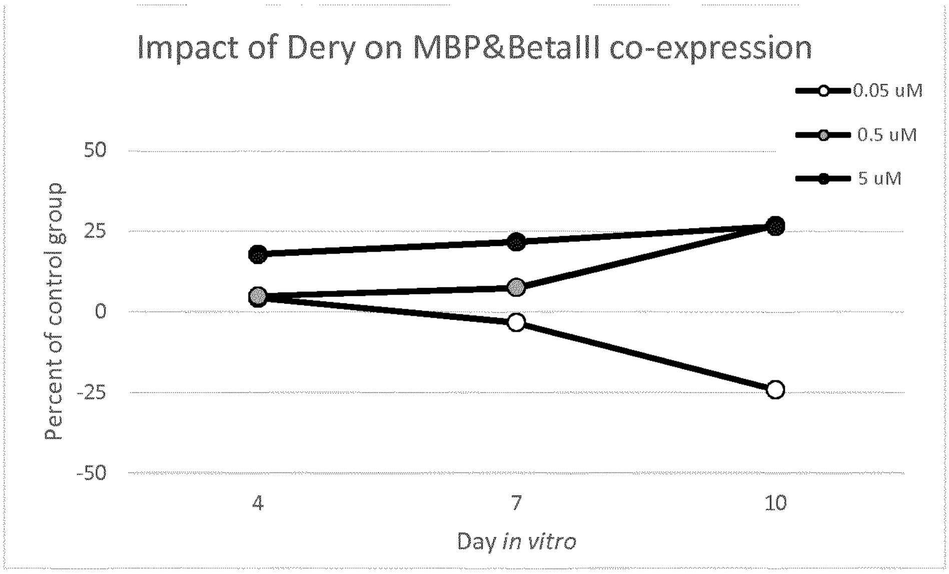
Impact of Dery on MBP&BetaIII co-expression
0.05 uM
0.5 uM
5 uM
Percent of control group
50
25
0
-25
-50
4
7
10
Day *in vitro*

Fig. 47

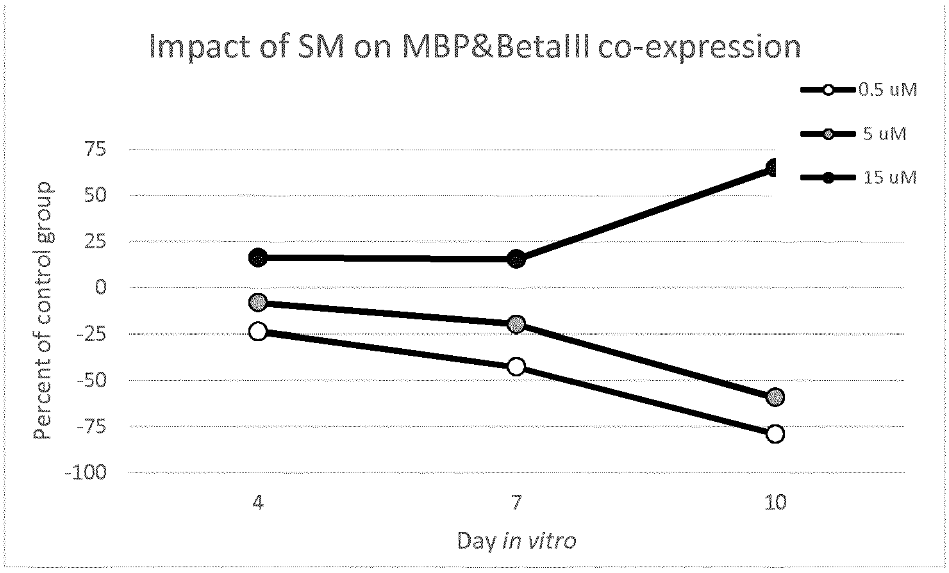
Impact of SM on MBP&BetaIII co-expression
0.5 uM
5 uM
15 uM
75
50
25
0
-25
-50
-75
-100
Percent of control group
4
7
10
Day in vitro

Fig. 48

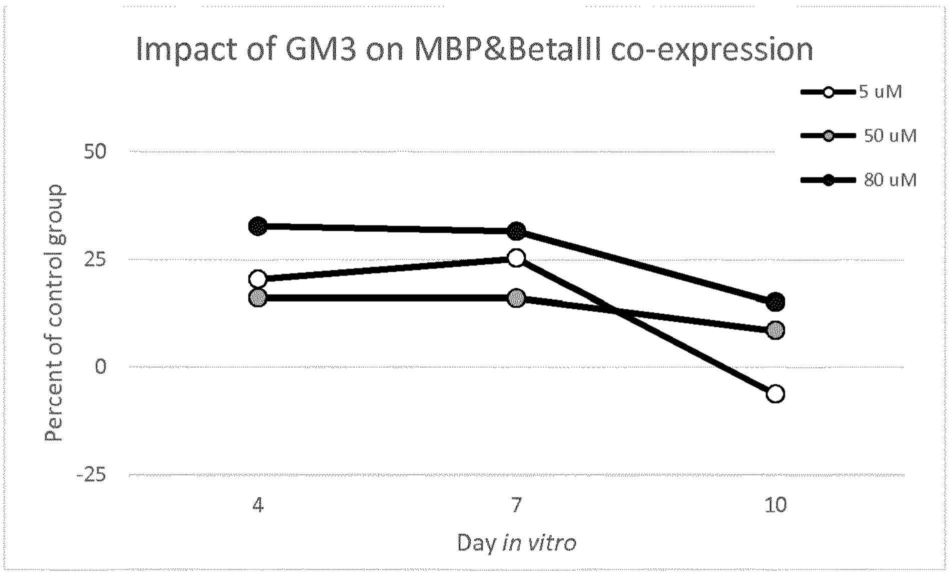
Impact of GM3 on MBP&BetaIII co-expression
5 uM
50 uM
80 uM
50
25
0
-25
Percent of control group
4
7
10
Day in vitro

Fig. 49

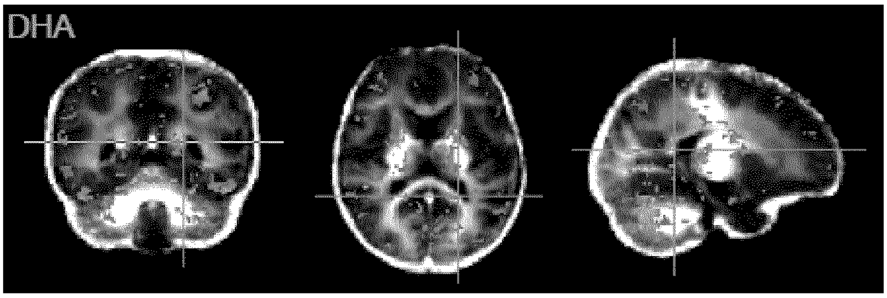


Fig. 50a

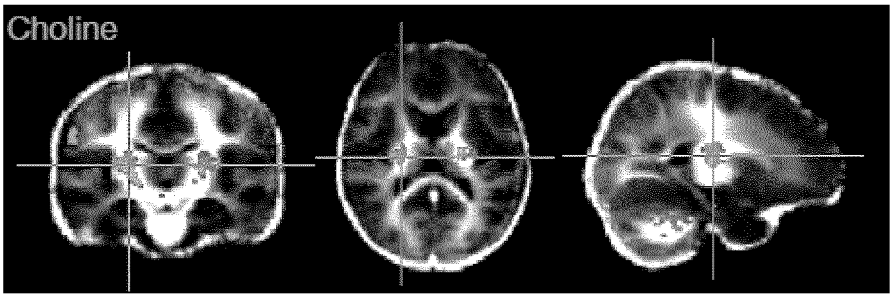


Fig. 50b

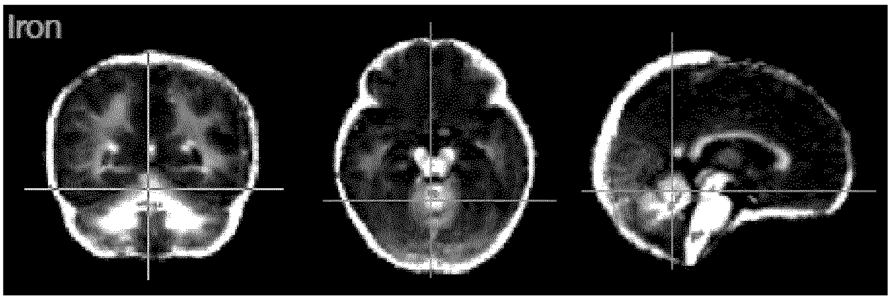


Fig.50c

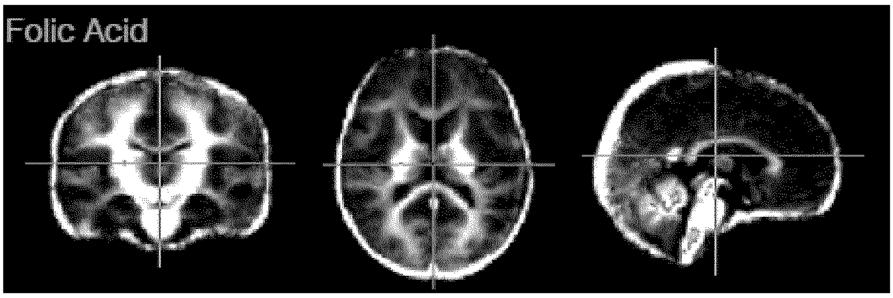


Fig. 50d

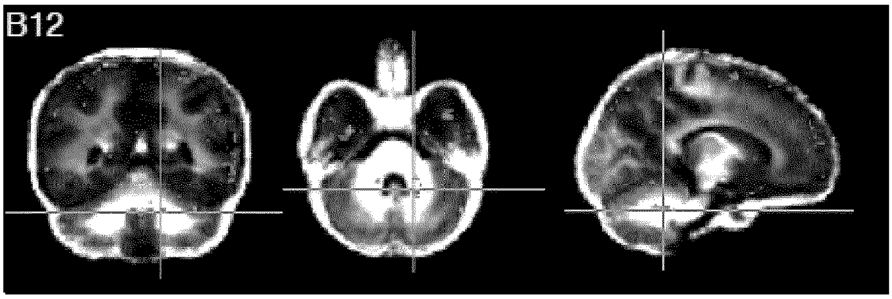


Fig. 50e

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 880902 A
- WO 0111990 A
- EP 322589 A
- WO 2012069416 A


### Non-patent literature cited in the description


- *Magn.Reson.Med.,* 2008, vol. 60, 1372-1387
- **WRODNIGG, T. M. ; STUTZ, A.E.** *Angew. Chem. Int. Ed.,* 1999, vol. 38, 827-828
- **DEONI et al.** *Magn. Reson. Med.,* 2008, vol. 60, 1372-1387
- **TRENERRY V.C. ; AKBARIDOUST G. ; PLOZZA T. ; ROCHFORT S. ; WALES W.J. ; AULDIST M. ; AJILOUNI S.** Ultra-highperformance liquid chromatography-ion trap mass spectrometry characterisation of milk polar lipids from dairy cows fed different diets. *Food Chemistry,* 2013, vol. 141, 1451-1460
- **GODZIEN J. ; CIBOROWSKI M. ; MARTINEZ-ALCAZAR M.P. ; SAMCZUK P. ; KRETOWSKI A. ; BARBAS C.** Rapid and reliable identification of phospholipids for untargeted metabolomics with LC-ESI-QTOF-MS/MS. *Journal of Proteome Research,* 2015, vol. 14, 3204-3216